# EUROPEAN PATENT APPLICATION

(11) **EP 1 333 092 A2**
(43) Date of publication of application: **06.08.2003**
(21) Application number: 03006709.4
(22) Date of filing: 12.03.1998
(51) Int. Cl.: C12N 15/12, C07K 14/47, C12Q 1/68, C12N 1/21, C12N 15/62, C07K 19/00, C12N 5/10, C07K 16/18, G01N 33/60, G01N 33/68, C12Q 1/48, G01N 33/53, A61K 38/17, A61K 48/00, A61K 31/70, C12N 15/11

(54) **28 human secreted proteins**

(30) Priority: 14.03.1997 US 40762 P; 14.03.1997 US 40710 P; 30.05.1997 US 50934 P; 30.05.1997 US 48100 P; 30.05.1997 US 48357 P; 30.05.1997 US 48189 P; 06.06.1997 US 48970 P; 05.09.1997 US 57765 P; 19.12.1997 US 68368 P
(62) Divisional of application: 98911597.7
(71) Applicant: HUMAN GENOME SCIENCES, INC., Rockville, MD 20850-3338 (US)
(72) Inventor: Ruben, Steven M., Brookeville, MD 20833 (US); Rosen, Craig A., Laytonsville, MD 20882 (US); Li, Yi, Sunnyvale, CA 94086 (US); Zeng, Zhizhen, Lansdale, PA 19446 (US); Kyaw, Hla, Boonsboro, MD 21713 (US); Fischer, Carrie L., Burke, VA 22015 (US); Li, Haodong, Gaithersburg, MD 20878 (US); Soppet, Daniel R., Centreville, VA 20120 (US); Gentz, Reiner L., 30.140.100 Belo Horizonte-Mg (BR); Wei, Ying-Fei, Berkeley, CA 94705 (US); Moore, Paul A., Germantown, MD 20874 (US); Young, Paul E., Gaithersburg, MD 20878 (US); Greene, John M., Gaithersburg, MD 20878 (US); Ferrie, Ann M., Painted Post, NY 14870 (US)
(74) Representative: VOSSIUS & PARTNER

(57) **Abstract**

The present invention relates to 28 human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

(1) GENERAL INFORMATION:

(i) APPLICANT:   Human Genome Sciences, Inc. et al.
(ii) TITLE OF INVENTION: 28 Human Secreted Proteins
(iii) NUMBER OF SEQUENCES: 113
(iv) CORRESPONDENCE ADDRESS:

(A) ADDRESSEE: Human Genome Sciences, Inc.
(B) STREET: 9410 Key West Avenue
(C) CITY: Rockville
(D) STATE: Maryland
(E) COUNTRY: USA
(F) ZIP: 20850

(v) COMPUTER READABLE FORM:
(A) MEDIUM TYPE: Diskette, 3.50 inch, 1.4Mb storage
(B) COMPUTER: HP Vectra 486/33
(C) OPERATING SYSTEM: MSDOS version 6.2
(D) SOFTWARE: ASCII Text

(vi) CURRENT APPLICATION DATA:
(A) APPLICATION NUMBER:
(B) FILING DATE: March 12, 1998
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: A. Anders Brookes
(B) REGISTRATION NUMBER: 36,373
(C) REFERENCE/DOCKET NUMBER: PS009PCT

(vi) TELECOMMUNICATION INFORMATION:
(A) TELEPHONE: (301) 309-8504
(B) TELEFAX: (301) 309-8439

(2) INFORMATION FOR SEQ ID NO: 1:

(i) SEQUENCE CHARACTERISTICS:
(A) LENGTH: 733 base pairs
(B) TYPE: nucleic acid
(C) STRANDEDNESS: double
(D) TOPOLOGY: linear

(xi) SEQUENCE DESCRIPTION: SEQ ID NO: 1:

GGGATCCGGA GCCCAAATCT TCTGACAAAA CTCACACATG CCCACCGTGC CCAGCACCTG 60

AATTCGAGGG TGCACCGTCA GTCTTCCTCT TCCCCCCAAA ACCCAAGGAC ACCCTCATGA 120

TCTCCCGGAC TCCTGAGGTC ACATGCGTGG TGGTGGACGT AAGCCACGAA GACCCTGAGG 180

TCAAGTTCAA CTGGTACGTG GACGGCGTGG AGGTGCATAA TGCCAAGACA AAGCCGCGGG 240

AGGAGCAGTA CAACAGCACG TACCGTGTGG TCAGCGTCCT CACCGTCCTG CACCAGGACT 300

GGCTGAATGG CAAGGAGTAC AAGTGCAAGG TCTCCAACAA AGCCCTCCCA ACCCCCATCG 360

AGAAAACCAT CTCCAAAGCC AAAGGGCAGC CCCGAGAACC ACAGGTGTAC ACCCTGCCCC 420

CATCCCGGGA TGAGCTGACC AAGAACCAGG TCAGCCTGAC CTGCCTGGTC AAAGGCTTCT 480

ATCCAAGCGA CATCGCCGTG GAGTGGGAGA GCAATGGGCA GCCGGAGAAC AACTACAAGA 540

CCACGCCTCC CGTGCTGGAC TCCGACGGCT CCTTCTTCCT CTACAGCAAG CTCACCGTGG 600

ACAAGAGCAG GTGGCAGCAG GGGAACGTCT TCTCATGCTC CGTGATGCAT GAGGCTCTGC 660

ACAACCACTA CACGCAGAAG AGCCTCTCCC TGTCTCCGGG TAAATGAGTG CGACGGCCGC 720

GACTCTAGAG GAT 733

(2) INFORMATION FOR SEQ ID NO: 2:

(i) SEQUENCE CHARACTERISTICS:
(A) LENGTH: 5 amino acids
(B) TYPE: amino acid
(D) TOPOLOGY: linear

(xi) SEQUENCE DESCRIPTION: SEQ ID NO: 2:

Trp Ser Xaa Trp Ser
1               5

(2) INFORMATION FOR SEQ ID NO: 3:

(i) SEQUENCE CHARACTERISTICS:
(A) LENGTH: 86 base pairs
(B) TYPE: nucleic acid
(C) STRANDEDNESS: double
(D) TOPOLOGY: linear

(xi) SEQUENCE DESCRIPTION: SEQ ID NO: 3:

GCGCCTCGAG ATTTCCCCGA AATCTAGATT TCCCCGAAAT GATTTCCCCG AAATGATTTC 60

CCCGAAATAT CTGCCATCTC AATTAG 86

(2) INFORMATION FOR SEQ ID NO: 4:

(i) SEQUENCE CHARACTERISTICS:
(A) LENGTH: 27 base pairs
(B) TYPE: nucleic acid
(C) STRANDEDNESS: double
(D) TOPOLOGY: linear

(xi) SEQUENCE DESCRIPTION: SEQ ID NO: 4:

GCGGCAAGCT TTTTGCAAAG CCTAGGC 27

(2) INFORMATION FOR SEQ ID NO: 5:

(i) SEQUENCE CHARACTERISTICS:
(A) LENGTH: 271 base pairs
(B) TYPE: nucleic acid
(C) STRANDEDNESS: double
(D) TOPOLOGY: linear

(xi) SEQUENCE DESCRIPTION: SEQ ID NO: 5:

CTCGAGATTT CCCCGAAATC TAGATTTCCC CGAAATGATT TCCCCGAAAT GATTTCCCCG 60

AAATATCTGC CATCTCAATT AGTCAGCAAC CATAGTCCCG CCCCTAACTC CGCCCATCCC 120

GCCCCTAACT CCGCCCAGTT CCGCCCATTC TCCGCCCCAT GGCTGACTAA TTTTTTTTAT 180

TTATGCAGAG GCCGAGGCCG CCTCGGCCTC TGAGCTATTC CAGAAGTAGT GAGGAGGCTT 240

TTTTGGAGGC CTAGGCTTTT GCAAAAAGCT T 271

(2) INFORMATION FOR SEQ ID NO: 6:

(i) SEQUENCE CHARACTERISTICS:
(A) LENGTH: 32 base pairs
(B) TYPE: nucleic acid
(C) STRANDEDNESS: double
(D) TOPOLOGY: linear

(xi) SEQUENCE DESCRIPTION: SEQ ID NO: 6:

GCGCTCGAGG GATGACAGCG ATAGAACCCC GG 32

(2) INFORMATION FOR SEQ ID NO: 7:

(i) SEQUENCE CHARACTERISTICS:
(A) LENGTH: 31 base pairs
(B) TYPE: nucleic acid
(C) STRANDEDNESS: double
(D) TOPOLOGY: linear

(xi) SEQUENCE DESCRIPTION: SEQ ID NO: 7:

GCGAAGCTTC GCGACTCCCC GGATCCGCCT C 31

(2) INFORMATION FOR SEQ ID NO: 8:

(i) SEQUENCE CHARACTERISTICS:
(A) LENGTH: 12 base pairs
(B) TYPE: nucleic acid
(C) STRANDEDNESS: double
(D) TOPOLOGY: linear

(xi) SEQUENCE DESCRIPTION: SEQ ID NO: 8:

GGGGACTTTC CC 12

(2) INFORMATION FOR SEQ ID NO: 9:

(i) SEQUENCE CHARACTERISTICS:
(A) LENGTH: 73 base pairs
(B) TYPE: nucleic acid
(C) STRANDEDNESS: double
(D) TOPOLOGY: linear

(xi) SEQUENCE DESCRIPTION: SEQ ID NO: 9:

GCGGCCTCGA GGGGACTTTC CCGGGGACTT TCCGGGGACT TTCCGGGACT TTCCATCCTG 60

CCATCTCAAT TAG 73

(2) INFORMATION FOR SEQ ID NO: 10:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID NO: 10:

CTCGAGGGGA CTTTCCCGGG GACTTTCCGG GGACTTTCCG GGACTTTCCA TCTGCCATCT 60

CAATTAGTCA GCAACCATAG TCCCGCCCCT AACTCCGCCC ATCCCGCCCC TAACTCCGCC 120

CAGTTCCGCC CATTCTCCGC CCCATGGCTG ACTAATTTTT TTTATTTATG CAGAGGCCGA 180

GGCCGCCTCG GCCTCTGAGC TATTCCAGAA GTAGTGAGGA GGCTTTTTTG GAGGCCTAGG 240

CTTTTGCAAA AAGCTT 256

(2) INFORMATION FOR SEQ ID NO: 11:

(i) SEQUENCE CHARACTERISTICS:
(A) LENGTH: 2084 base pairs

(B) TYPE: nucleic acid
(C) STRANDEDNESS: double
(D) TOPOLOGY: linear

(xi) SEQUENCE DESCRIPTION: SEQ ID NO: 11:

CTATCAGATG CTGGGCCTCC TCAGCCATAG CCCCCTGCTC CTACCCCCTG ACTGGCTCTT 60

GTGTCCTCAC CTCTCACCCT CTCCTTCCTG GGAGGCCCTG GGAGGTGATC ATTGACACCC 120

AGCCAAGCAG ACAGCTGCGG GTGCCCAAGC CCTTGCTGGG CCTGCGCGTG AGGAGTCCCA 180

CTGCTTCTAA AGGAAGTCCT GGGCAGGAGG TGGCTTTGGT GGTTGGTTCC AAAGTTGAAA 240

ATGCTTGCAG TTTGACCTTA GAAGAAGTGG GAAGAAGAAG GAGCTCTACA GGGTCAGCTT 300

TGTTTGATTT GTCCAGTCTA AGAAGTCCCA TTGCCAAAGC TTTCTGCAGG AGGGTGAATG 360

CCGCAGCTTG GCAGCCCCTG GGTTTCTCTT GGAAATGGTC AGTTTCCCCT CAAAGTACCC 420

AAAGTAGCCT TGGCTTGAGT TTTTGTCCTT GCCTCCTTTT TAGAGAAGAG GGCATTTAGA 480

CTGCATTTTC CTGGTTAAAG AAGGTTAAAG CAAATGTTTA TTGCCTTTTC TAGTGAACTA 540

ACTCGTAGAG ATGTTCTCAG CAGGAAGACA GTCTTAGCAC TGTCACTTAG CAGATTGCAC 600

TTAAGTCCCT TGTGCTGGCC AGATGGCGTG GCTGGTTGCC TTAATATGTC CCAGGACCCC 660

TGACAGGGCT GCCTGGCCTC TCCCTCGTGC TCCTCAAGAG CCCAGTCCAT ACACTGTGGA 720

TGTCATTGCT GTCGGGTTAG GAAGTCTTGT CCTAGAACGC CCTGGCTGGT ATGACCACAG 780

TTCATGGCGG CTCTTCTCGC TTGGGTCATG GTCATCTTCC AGCACCTGCT GTGCTGGGNA 840

AGGCCGAGGA TGGGGGCCCA GCACTGTCCA GGCCTGCTGG GGCCTGGCTG GGAGTCCTGT 900

GGGCAGCATG GAACATGCAG CTGGGCTTCC TGTGACCAGG CACCCTCTGG CACTGTTGCT 960

TGCCCTGTGC CCTGGACCTT TTCCTGCCCT TCTCCTTCCT CTGCTCCCTT GGGGCTACCC 1020

CTTGGCCCCT CCTGGTCTGT GCAAACTCCC TCAGGGAGCC CCCCTGCCCT GTAGCTCTCR 1080

CTTAACTTCC TAGGGGCTGC TGAGCCCACC CAGAGGTTGT TGGAGTTCAG CGGGGCAGCT 1140

TGTCTCCCTT GTCAGCAGGG GCGTAAGGGC TGGGTTTGGC CATACAAGGT TGGCTACGCC 1200

CTCAATCCCT GACCGTTCCA GGCACTGAGC TGGGCACCCA CGGAAGGACA TGCTGTCCAG 1260

ACTGTGATGA CTGCCAGCAC AGGGCATCTC GGGCTTGGCT GGTCTGCGAG GCCTTGCCCC 1320

TGTGGAACTC TGGGTTCCTG TTTTCTCAGT CTTTTTTGCG GCTTTGCTGT GGTTGGCAGC 1380

TGCCGTACTC CAGGCTTGTG TCGGCCACTC AGATGAGGGC TGTGGTGCGA GCCAGTGCAG 1440

GAGAGCTGCG CTTGGGATTG TGCCCTCTCC TGTGTCTGTC CTCCGGACCT ACCCAGGTCT 1500

CCACCATCAG GACCCTGTCT TTGGGTTTAG AAGACCAAGT ATGGGGAAAA CCAGGCACCA 1560

GCCTCTGCAG CAATGGGTCC CTCTAGCCTG TGGACACCAG CTGGGGGATC CAGGGTCAGG 1620

CCCCCTCCTC TCCCCAGTTT CCCTCTGCTG TGGGTTCTGG GCTGTCATGT CTCCACCACT 1680

TAAGGATGTC TTTACACTGA CTTCAGGATA GATGCTGGGA TGCCTGGGCA TGGCCACATG 1740

TTACATGTAC AGAACTTTGT CTACAGCACA AATTAAGTTA TATAAACACA GTGACTGGTA 1800

TTTAATGCTG ATCTACTATA AGGTATTCTA TATTTATATG ACTTCAGAGA CGCGTATGTA 1860

ATAAAGGACG CCCTCCCTCC AGTGTCCACA TCCAGTTCAC CCCAGAGGGT CGGGCAGGTT 1920

GACATATTTA TTTTTGTCTA TTCTGTAGGC TTCCATGTCC AGAATCCTGC TTAAGGTTTT 1980

AGGGTACCTT CAGTACTTTT TGCAATAAAA GTATTTCCTA TCCAAAAAAA AAAAAAAAAA 2040

ACTCGAGGGG GGGCCCGGTA CCCAATTCGC CCCTATAAAG AGTC 2084

(2) INFORMATION FOR SEQ ID NO: 12:

(i) SEQUENCE CHARACTERISTICS:
(A) LENGTH: 1586 base pairs
(B) TYPE: nucleic acid
(C) STRANDEDNESS: double
(D) TOPOLOGY: linear

(xi) SEQUENCE DESCRIPTION: SEQ ID NO: 12:

AATTCGGCAC CAGGAGAAGT GGAGTTTGGA AGTTCAGGGG CACAGGGGCA CAGGCCCACG 60

ACTGCAGCGG GATGGACCAG TACTGCATCC TGGGCCGCAT CGGGGAGGGC GCCCAMGGCA 120

TCGTCTTCAA GGCCAAGCAC GTGGAGACTG GCGAGATAGT TGCCCTCAAG AAGGTGGCCC 180

TAAGGCGGTT GGAAGACGGC TTCCCTAACC AGGCCCTGCG GGAGATTAAG GCTCTGCAGG 240

ARATGGAGGA CAATCAGTAT GTGGTACAAC TGAAGGCTGT GTTCCCACAC GGTGGAGGCT 300

TTGTGCTGGC CTTTGAGTTC ATGCTGTCGG ATCTGGCCGA GGTGGTGCGC CATGCCCAGA 360

GGCCACTAGC CCAGGCACAG GTCAAGAGCT ACCTGCAGAT GCTGCTCAAG GGTGTCGCCT 420

TCTGCCATGC CAACAACATT GTACATCGGG ACCTGAAACC TGCCAACCTG CTCATCAGCG 480

CCTCAGGCCA GCTCAAGATA GCGGACTTTG GCCTGGCTCG AGTCTTTTCC CCAGACGGCA 540

GCCGCCTCTA CACACACCAG GTGGCCACCA GGAGCTCACT GAGCTGCCGG ACTACAACAA 600

GATCTCCTTT AAGGAGCAGG TGCCCATGCC CCTGGAGGAK GTGCTGCCTG ACGTCTCTCC 660

CCAGGCATTG GATCTGCTGG GTCAATTCCT TCTCTACCCT CCTCACCAGC GCATCGCAGC 720

TTCCAAGGCT CTCCTCCATC AGTACTTCTT CACAGCTCCC CTGCCTGCCC ATCCATCTGA 780

GCTGCCGATT CCTCAGCGTC TAGGGGGACC TGCCCCCAAG GCCCATCCAG GGCCCCCCCA 840

CATCCATGAC TTCCACGTGG ACCGGCCTCT TGAGGARTCG CTGTTGAACC CARARCTGAT 900

TCGGCCCTTC ATCCTGGARG GGTGAGAAGT TGGCCCTGGT CCCGTCTGCC TGCTCCTCAG 960

GACCACTCAG TCCACCTGTT CCTCTGCCAC CTGCCTGGCT TCACCCTCCA AGGCCTCCCC 1020

ATGGCCACAG TGGGCCCACA CCACACCCTG CCCCTTAGCC CTTGCGAAGG TTGGTCTCGA 1080

RGCAGARGTC ATGTTCCCAG CCAAGAGTAT GAGAACATCC AGTCGAGCAG AGGAGATTCA 1140

TGGCCTGTSC TCGGTGAGCC TTACCTTCTG TGTGCTTCAC ATCACTGAGC ACTCATTTAG 1200

AAGTGAGGGA GACAGAAGTC TAGSCCCAGG GATGGCTCCA GTTGGGGATC CAGCAGGAGA 1260

CCCTCTGCAC ATGAGGCTGG TTTMCCAACA TCTACTCCCT CAGGATGAGC GTGAGCCAGA 1320

AGCAGCTGTG TATTTAAGGA AACAAGCGTT CCTGGAATTA ATTTATAAAT TTAATAAATC 1380

CCAATATAAT CCCAGCTAGT GCTTTTTCCT TATTATAATT TGATAAGGTG ATTATAAAAG 1440

ATACATGGAA GGAAGTGGAA CCAGATGCAG AAGAGGAAAT GATGGAAGGA CTTATGGTAT 1500

CAGATACCAA TATTTAAAAG TTTGTATAAT AATAAAGAGT ATGATTGTGG TTCAAGGATA 1560

AAAAAAAAAA AAAAAAAAAA ACTCGA 1586

(2) INFORMATION FOR SEQ ID NO: 13:

(i) SEQUENCE CHARACTERISTICS:
(A) LENGTH: 2350 base pairs
(B) TYPE: nucleic acid
(C) STRANDEDNESS: double
(D) TOPOLOGY: linear

(xi) SEQUENCE DESCRIPTION: SEQ ID NO: 13:

GAAGAAGAGC GACCTGCCCT AATGGATGAC AGAAAGCACA AAATTTGTAG CATGTATGAC 60

AACTTAAGGG GGAAATTGCC TGGACAAGAG AGGCCTAGTG ATGACCACTT TGTACAGATC 120

ATGTGTATCC GAAAAGGGAA GAGAATGGTT GCCCGTATTC TTCCTTTCCT CTCCACAGAG 180

CAAGCAGCTG ACATTCTCAT GACAACAGCC AGGAACCTCC CTTTCCTTAT CAAGAAGGAT 240

GCACAAGATG AGGTGCTGCC ATGCTTACTG AGTCCCTTCT CTCTCCTTCT CTATCATCTT 300

CCATCAGTGA GTATCACCAG CCTTTTGCGA CATAATGAAC CTACCTCAAA GTGCAGCTAC 360

ACCAGCACTC TCCAATCCTC ACCTCACTGC TGTGCTCCAG AACAAGTTTG GCCTGTCACT 420

GSTCCTCATC CTCCTGAGCC GTGGTGAAGA CCTACAGAGT TCAGACCCTG CTACAGAATC 480

AACACAAAAT AATCAGTGGA CGGAGGTGAT GTTCATGGCA ACACGAGAAC TTCTGCGGAT 540

TCCCCAAGCA GCCCTGGCCA AGCCAATCTC TATACCTACA AACCTAGTGT CCCTCTTTTC 600

TCGCTATGTT GACCGGCAGA AACTGAACTT GCTGGAGASA AAACTGCAGC TAGTTCAGGG 660

GATACGATAA AAGATCTCCA AATGTGTCCT GTACCTCCTT TTGGCTGCCA CCTGCACTGC 720

TGCCATCACC AATGGRGTGT TTTTAATGAG GGAAGGAAGG TAGCTTTTTC CCCAAAGCAA 780

AGKMTTGTGG GATCGATTCC TGTTTACAGG GGTTGTCTCT CTAAATGTCA GATATTTCCC 840

CACTGCTCTA TGAAATTTGG CTGGGTGATA CTTCTGCTGG TTTCTTTACC TTCTGTGTTA 900

CAGTTCTGCA TGTCCTACTT TTACTCAGTT CTGTTTTGCA TTTWCTTTGC CCTAGAGACA 960

CAAGTGTAAT CTCTCCCTTT ATCCCTCCAC TACTCCACCT CAGAGTAGAT TGTAGCCTGC 1020

CAAAGGATTC CTTCCCTCAT CCTATTGAAG TTGTTTTTTC ATTGCCCCAT ATTAATATGA 1080

CTATAGAAGA GCCAATTAAG TAGAAATCAA GATATACACA CACACATAGA TACACACACA 1140

CACACCCCAT ACATGTATTT ATGTGGTCTT CAGAGGGTCC TTAAAGAATG AATTTTAGAT 1200

TGAAAAATAT TTAGTTGTCT CATTACCTCT TCTAAACACA AACCAGCTGA TGTATTTTAA 1260

TCTGTTTCTG TTCTATCTTG TAATTAATTT GGTGGGTTCT ACTTGTTTTA ACATAAATAA 1320

AGAGTATGCA GCACGTTTAA TAAAATCAGA ACTCTTAATT GGCTTATGCC CAGGTCTAGG 1380

CTGAGAAGTC CTTTTTCTTC TTCCCACCTT TATTTCCTTA GTTTCTGTCC ACCTTAATCG 1440

AAACAACACA TGGTTATGTC TTTTTCCTGC TACAACTACA GGGTACTTGA GCCTTTCCCC 1500

TCAAGTGCAT TCGAAGTCAC CCAGGATGAT CCTCACTAGT AGCCTGCTTT GGCAGTGTGG 1560

CTTTTTGCAC ACTTGCCCTG TCTTCCTGAG ACTACTTCAG TAAGCCATGC TTCCTTCTTC 1620

CCCACTTTTA TTTGGTGTCA TGAATAGAAA CTTCCAAATG TAACCATGGA AGCTAAGTTT 1680

GGCCTGCTTT GCTTTTTAGT CTCCACACCA TGGGCAGAAC TGCTGTCTTT ACTACTTCAT 1740

CTCACCCAAG TCCCGTTCCC AGGCAGCCAR GGGCCTGGGT TTTGAATAAT TGCAAGGGCC 1800

AGCCTGCCAT GATCTTTCTC ACTTACTCCT CTCCCATTCA GCAATCAACC AGACTAAGGA 1860

GTTTTGATCC CTAGTGATTA CAGCCCTGAA GAAAATTAAA TCTGAATTAA TTTTACATGG 1920

CCTTCGTGAT CTTTCTGCTG TTCTTACTTT TTCGAATGTA GTTGGGGGGT GGGAGGGACA 1980

GGTTATGGTA TTTAAAGAGA ATAAACATTT TGCACATACA TGTATTGTAC AACAGTAAGA 2040

TCCTCTGTTA AAACCAGCTG TCCTGTTCTC CATCTCCATT TCTTCCCATG CTGTAACCCC 2100

AGGCTCCACC AGCTGTTCCC CAGTGATGTT ACCTAGCTTC CCTCTACCGT TGTCTACTGA 2160

CCATTTCCAC TACATGCCTT TCCTACCTTC CCTTCACAAC CAATCAAGTG AATACTTGAT 2220

TATTATCTCT TCCTTACTGT GCTTTATCTT TTTTGTTTGG ATTGGTTCTA ATTAATGAAA 2280

ATAAAAGTTT CTAAATTTAC ATTTTTATAG GGTATTGTAA ATAAAAACAA ATTGTATACT 2340

TAAAAAAAAA 2350

(2) INFORMATION FOR SEQ ID NO: 14:

(i) SEQUENCE CHARACTERISTICS:
(A) LENGTH: 1348 base pairs

(B) TYPE: nucleic acid
(C) STRANDEDNESS: double
(D) TOPOLOGY: linear

(xi) SEQUENCE DESCRIPTION: SEQ ID NO: 14:

ACGAAGACAC CAGACCCTGT GGAGCCTGTG GTGACCACCG AAGGNCAGTT CGGGTGCAGC 60

AGGGCTCGAG CCCAGAAAAC TATCCTCTAA GACCAGACGT GACAAGGAGA AGCAGAGCTG 120

TAAGAGCTGT GGTGAGACCT TCAACTCCAT CACCAAGAGG AGGCATCACT GCAAGCTGTG 180

TGGGGCGGTC ATCTGTGGGA AGTGCTCCGA GTTCAAGGCC GAGAACAGCC GGCAGAGCCT 240

GTCTGCAGAG ATTGTTTCCT GACACAGCCA GTGGCCCCTG AGAGCACAGA GAAGACACCC 300

ACTGCAGACC CCCAGCCCAG CCTGCTCTGC GGCCCCCTGC GGCTGTYAGA GAGCGGTGAG 360

ACCTGGAGCG AGGTGTGGGC CGCCATCCCC ATGTCAGATC CCCAGGTGCT GCACCTGCAG 420

GKAGGCAGCC AGGACGGCCG GCTGCCCCGC ACCATCCCTC TCCCCAGCTG CAAACTGAGT 480

GTGCCGGACC CTGAGGAGAG GCTGGACTCG GGGCATGTGT GGAAGCTGCA GTGGGCCAAG 540

CAGTCCTGGT ACCTGAGCGC CTCCTCCGCA GAGCTGCAGC AGCAGTGGCT GGAAACCCTA 600

AGCACTGCTG CCCATGGGGA CACGGCCCAG GACAGCCCGG GGGCCCTGCA GCTTCAGGTC 660

CCTATGGGCG CAGTGCTCCG TGAGCTGAGT CTCCCACTGC CCTGCACACC ACCACATTGG 720

ACCTGTGCTG TCCTGGGAGG TGGTGTTGGA GGCCCCATGA AGAGCGCCCT GGACTTGCTT 780

GAGGGTGGGC CAACAGCCCA GAGYTCAGGA CATTTGGCTT TGGGGGGAAG GAAAYTGAGG 840

CCCAGAGAGG GGCAACCAYT GGCCAAGGGT CACCCAGCAA GTTTTGGYTA AGAGCCTGGC 900

CTCCAGCCCC AGCAGTKTGG CCCAGAGCAG GGGCCGAYTG CCAAAGTAAC CATCATCCAT 960

ATGGGCCGTG TGGTGATGCT GGCCCGGAAG GCAGAAAGAG GCAGCATGGG CACTGCCAGG 1020

GACAGCCACA TCCTGCTGGT CTGCAGCGTG GTCCACCCCG CCTCTGCCCA GCCTGTCTAC 1080

ACCGTGTGAG CTGAATCGTG ACTTGCTTCC CACCTCCTTT CTCTGTCCTC TCCTGAGGTT 1140

CTGCCTGCAG CCCCCAGGAG GTGGGCCTGC CCCATCCTAG CTGGACTCAT GGTTCCTAAA 1200

TAACCACGCT CAGAAGCTCT GCTAGGACTT ACCCCAGCCA CTGAGTGGCA GGCGCATGAG 1260

ATTTGTGGCT GTTCCTGATG CTAGTGGCAC ACAGTGCTTA TCTGCATAAA TAAACACTGG 1320

SCACCAAAAA AAAAAAAAAA AAAAAAAC 1348

(2) INFORMATION FOR SEQ ID NO: 15:

(i) SEQUENCE CHARACTERISTICS:
(A) LENGTH: 1123 base pairs
(B) TYPE: nucleic acid
(C) STRANDEDNESS: double

(D) TOPOLOGY: linear

(xi) SEQUENCE DESCRIPTION: SEQ ID NO: 15:

CGCGCCCAGC CCCTGCTGCT CTGGGCAGAC GATGCTGAAG ATGCTCTCCT TTAAGCTGCT 60

GCTGCTGGCC GTGGCTCTGG GCTTCTTTGA AGGAGATGCT AAGTTTGGGG AAAGAAACGA 120

AGGGAGCGGA GCAAGGAGGA GAAGGTGCCT GAATGGGAAC CCCCCGAAGC GCCTGAAAAG 180

GAGAGACAGG AGGATGATGT CCCAGCTGGA GCTGCTGAGT GGGGGAGAGA TGCTGTGCGG 240

TGGCTTCTAC CCTCGGCTGT CCTGCTGCCT GCGGAGTGAC AGCCCGGGGC TAGGGCGCCT 300

GGAGAATAAG ATATTTTCTG TTACCAACAA CACAGAATGT GGGAAGTTAC TGGAGGAAAT 360

CAAATGTGCA CTTTGCTCTC CACATTCTCA AAGCCTGTTC CACTCACCTG AGAGAGAAGT 420

CTTGGAAAGA GACCTAGTAC TTCCTCTGCT CTGCAAAGAC TATTGCAAAG AATTCTTTTA 480

CACTTGCCGA GGCCATATTC CAGGTTTCCT TCAAACAACT GCGGATGAGT TTTGCTTTTA 540

CTATGCAAGA AAAGATGGTG GGTTGTGCTT TCCAGATTTT CCAAGAAAAC AAGTCAGAGG 600

ACCAGCATCT AACTACTTGG ACCAGATGGA AGAATATGAC AAAGTGGAAG AGATCAGCAG 660

AAAGCACAAA CACAACTGCT TCTGTATTCA GGAGGTTGTG AGTGGGCTGC GGCAGCCCGT 720

TGGTGCCCTG CATAGTGGGG ATGGCTCGCA ACGTCTCTTC ATTCTGGAAA AAGAAGGTTA 780

TGTGAAGATA CTTACCCCTG AAGGAGAAAT TTTCAAGGAG CCTTATTTGG ACATTCACAA 840

ACTTGTTCAA AGTGGAATAA AGGTTGGCTT TTTAAATTTT ATTTATTTTT GTGCTGGCTA 900

CGTTAATTTT ATTTTAGTGT TACCTTCCTC ACTGAAGGTA TTTCTTTGTA ATAAAAGAAA 960

GAATCTTGCA GGAGAAAATA AGGGGGCAAC ATAAGAAACA ATAATTATGG CACCTGAATT 1020

AGGACAGTGA CATTAAATTT CTGTTATTTG TTAAAAAAAA AAAAAAAAAA AAAAAAAAAA 1080

AAAAAAAAAA AAAAAAAAAA AAAAAAAAAA AAAAAAAAAA AAA 1123

(2) INFORMATION FOR SEQ ID NO: 16:

(i) SEQUENCE CHARACTERISTICS:
(A) LENGTH: 890 base pairs
(B) TYPE: nucleic acid
(C) STRANDEDNESS: double
(D) TOPOLOGY: linear

(xi) SEQUENCE DESCRIPTION: SEQ ID NO: 16:

TTTTAATTGA TCTGTGARAA AACTTAAGAA AATCACAATT TCAGCTAACA GCAATTGTGT 60

CCCAAAGATG AAGATACTAT AACCTCAAAT GGTGCAGATC CAGAACTGGG CTGGATGACA 120

TCCCTACTGT GCCATGTCCT GGGGCATTTG GAAGGGACTG GACCTCTTTC CCCTCATCAA 180

AGGAAACAGC AGTCTTTGCC TCTTTCTGTT GGTTGTGCCC AAGGGCTACA GTAGCTCTGA 240

AATAACAAGA GCTCTGTAAT AACAGTAATA AATAGCTCTG AAATAACAGT CCTAAGAACT 300

CCTAAAGTCC TGAGAACTTT TCTTGTAATG CAGCTTTTTC TCTTCCTGAG AAACAGTGTG 360

TTCTAATGGG ATTCCCAGGC AGTTCCTACA CCTACGGTGT GTGTTCCAGC AGGGAGGAGT 420

TATGGGCTGG GCTGCCTTTT CCCATGGGTC TTCATTCCCA ATGGAAAGTT CACTCTGCTT 480

AGTTTGGAAT TATTTTTCTT TCAGTTGTTC TGGAACCTTT GCTTTTTATT GATTTATACA 540

ATACAATTGG TGGGAGGGTG GACTTGGGAT GGGAGTGGGA AAAGCATGTA AGAGCTCCTT 600

TTGTGATGGT CCATCTACCC AAAAGAGATC TGCTTTAGTG AACGATACTC TTTCATTTTT 660

CTAAATTAGA TCAAGTTGTT ATTGATTTTA GATGACTTGT ATGCAAATTT GAAAAACTTT 720

TTTTTTTAAA GCTGATTGGG AACTACAAAC AATGAATGGA ATCTACTGAC ACAGCTAATT 780

GGAAAACAGA TGTCTTCTTC TGTCCTATTG ATGCTGGTGT TTAAAAAACA TCACTTAAAA 840

AAAAAGAATA AATAGTTCTA AAAGCAAAAA AAAAAAAAAA AAAAAAATTC 890

(2) INFORMATION FOR SEQ ID NO: 17:

(i) SEQUENCE CHARACTERISTICS:
(A) LENGTH: 619 base pairs
(B) TYPE: nucleic acid
(C) STRANDEDNESS: double
(D) TOPOLOGY: linear

(xi) SEQUENCE DESCRIPTION: SEQ ID NO: 17:

TCAGGCCCCG CTGACTCCGC CCCGCAACAC TCTCACTCGC CCTTCGTGTC CCATCAGGTC 60

CCGCTGACTC CGCCCCGCAA TACTCTCACT CGCCCTTYGT GTCCCATCAG GTCCCGCTGA 120

CTCCGCCCCG CAACACTCTC ACTTGCCCTT CGTGTCCCAT CAGGTCCTGC TGACTCCATC 180

TCCTCAGCGT CTCCAACATG TCCCTTCCTT GCCACCTCTT GCCTGGATTA CTACAGCAGC 240

TTCTAACGAG TCTCCCTGCC TTTCAGTTCT CCGCACCGCT TCAAGTGTTC AGTCTGGATG 300

GTCTGTCACT CCCAGCGCCA AAACTGCTGA CGGCTTCCCT TTGCCTTCAG GACGAAGTCC 360

GTGCTGTCTG ACATAACTTA TAGGACCTTT TAGCCAGCCT GGGCAACATA GCAAGACCCT 420

GTCTCTACCA GAAAATACAA AAATGAGCCA GGCATAGTGG TGTGCACCTG TAGTCCCAGC 480

TACTTGGGAG GCTGAGGTGG GAGGATCACC TGAGCCCAGG AAGTCAAGGC TGCCAGTGAG 540

CCATGATCAC ACCACTGCAC TCCAGCCTGG GCCACAGAGT GAGACCCTGT CTCAAAAAAA 600

AAAAAAAAAA AAAACTCGA 619

(2) INFORMATION FOR SEQ ID NO: 18:

(i) SEQUENCE CHARACTERISTICS:
(A) LENGTH: 1768 base pairs
(B) TYPE: nucleic acid
(C) STRANDEDNESS: double
(D) TOPOLOGY: linear

(xi) SEQUENCE DESCRIPTION: SEQ ID NO: 18:

AATTTAATAT TTTTTAGTAT TACAATATAT TCTTATAAAA AAGGTGCAAG TGAAAAAGGA 60

CACTGTAGAT TATGTCCATT AGCCTCATTT GTCATCTGAG GCAGCTGGTG AGAACAGCCT 120

TGGCTGAAGG CATCCCTGGT AGAAGTCGGG GGAGATAGAT AGTCACAGTT CCCCAGTTGG 180

TGGAAATGGG ATGGGAGTAG GGAGAGGCTG GAACAGACCC TTCCCCATTC ACCTGGRRGA 240

ATTTTCTCCT CCCACTGCCC TAAACACTTT ATTTCCATCA CAGGGGAGAA ATGCTGCTGA 300

GAAGGTTGTG TTTGTTAGGT TGATGACGAA TTTTACATTG GCCACAAAAT TAGCTAGAGA 360

AACTTATCTA AAGGTGGCAG GAGCAGTGGG GAGGGCATGA AGAAAGCAAG ACCAAGAAAC 420

AACCTATTAA GGACCAGCTC AGCCACCCCG ACTGGCACCA GCCCCTTCTT ACTCAGTTGA 480

GTATGAGTCC ATGGTCCAAG GCACTGTTGG AGATCTGGCT ACAGTGGCAT CTAGCACCAG 540

AGCCACTGGC CAGATGTAGA AAATAAATAG AAAAATATCT TTCTTTTAGA GTGAGAAGGC 600

TGAGCTCTGG AACAACGTAT TTGTGTCCTC TGTCAACAGT TGAACCAAAT TCTGCTTTTC 660

TGAAGATCAA ATGTATCTTG AACAGCTTCC ATAGTCCTTT TGTTTCCAGG TGCGTATCCA 720

GTCTTCCATG GTGGGTGGGA ATGCCAGACA CGCTTGTGGA GCCCTCCCCT GTTCCCTGCC 780

CCTGAGGGGG TTAGGTTGAC ATCAGCCTGG TCAGTTTGGG AGAGGACCTT TAGAGGCCTC 840

ACCCACAACC TCCCATCTTC CCCAACACTT GTCTTGCAGT GGGAGCTCTT GGGGCTGCAG 900

ATGCATATAG CCAAACTCTC TGCAGCTGTT CTGCCTGGAA GCCTTCATCT TGCCCTCAMC 960

TGGGTTCCAG GATGGCCTCT TCACACCTGT GTCAGCCAGG CTTGCACTTG CTCAGATCCC 1020

TCCCACCAGA ACACACACAC ACCGCCCGCC CCCTCAAACC AACGCACATG CTGGGCTCAC 1080

CGACCCTGTG TTTCTTCCCC CCCGCAGCTA CTACGGTCCC AGCCCCAGGA GTTGGATGCA 1140

AGTGAAAGGC AGAAGATAGG CAGCTGAGAG TAGGCCCAGC TCACCAGTCT CCACTGGCAA 1200

TAACCCTGAG CCAGGGATTA GGTTGGAAAG TGAGAAACAC AGGGAAGGGC AGAAGGGCCA 1260

AGAGCTCATT GATGGTAGAG GTTAGNCAGG GCCAGTCTCA AAGAAGATGA AAGGCCAACT 1320

CGGAACGTGG TATTGAATAA GAGCCTTGAT GGAGTTTTAG AAAAATTTTG TCTAGATACA 1380

GCCATCCCAT CCACCAAGGC CAGCATGAGA TGGACAAAAT GGAAGGTGGC AGTGGATGGG 1440

AGGACCAGAA GGAACCCCTT GCAAGTTGGG CTGAAGAACC AAATTGGGTA CCARAAATGG 1500

GTGKCCCCCC TCTCAGCCTT CCTCCTTGGC ACCTTCAGGT GATGTGCTCC CCAGAGGATA 1560

TCAGCCTTCC TCCTCCATCC CCATCTCCCC AGTTTCCCTT GCCTGCTCTG CTGTTCGCAC 1620

CATCTGAACG CCTGAGAGGA GGGGCCACCC TTAGAGACAG CATGTTAATG TAGAGAACTA 1680

TGGGATGGAG CTAAGCATTC AAGTGCTGCC CTCTGCTGAG GGGCTGTAGG GGACTCCAAG 1740

GCAACATTTG AGGTCACTGT CTGGCTTC 1768

(2) INFORMATION FOR SEQ ID NO: 19:

(i) SEQUENCE CHARACTERISTICS:
(A) LENGTH: 1699 base pairs
(B) TYPE: nucleic acid
(C) STRANDEDNESS: double
(D) TOPOLOGY: linear

(xi) SEQUENCE DESCRIPTION: SEQ ID NO: 19:

CTCGTGCCGA ATTCGGCACG AGCGAAAAGA TGGCGGTCTT GGCACCTCTA ATTGCTCTCG 60

TGTATTCGGT GCCGCGACTT TCACGATGGC TCGCCCAACC TTACTACCTT CTGTCGGCCC 120

TGCTCTCTGC TGCCTTCCTA CTCGTGAGGA AACTGCCGCC GCTCTGCCAC GGTCTGCCCA 180

CCCAACGCGA AGACGGTAAC CCGTGTGACT TTGACTGGAG AGAAGTGGAG ATCCTGATGT 240

TTCTCAGTGC CATTGTGATG ATGAAGAACC GCAGATCCAT CACTGTGGAG CAACATATAG 300

GCAACATTTT CATGTTTAGT AAAGTGGCCA ACACAATTCT TTTCTTCCGC TTGGATATTC 360

GCATGGGCCT ACTTTACATC ACACTCTGCA TAGTGTTCCT GATGACGTGC AAACCCCCCC 420

TATATATGGG CCCTGAGTAT ATCAAGTACT TCAATGATAA AACCATTGAT GAGGAACTAG 480

AACGGGACAA GAGGGTCACT TGGATTGTGG AGTTCTTTGC CAATTGGTCT AATGACTGCC 540

AATCATTTGC CCCTATCTAT GCTGACCTCT CCCTTAAATA CAACTGTACA GGGCTAAATT 600

TTGGGAAGGT GGATGTTGGA CGCTATACTG ATGTTAGTAC GCGGTACAAA GTGAGCACAT 660

CACCCCTCAC CAAGCAACTC CCTACCCTGA TCCTGTTCCA AGGTGGCAAG GAGGCAATGC 720

GGCGGCCACA GATTGACAAG AAAGGACGGG CTGTCTCATG GACCTTCTCT GAGGAGAATG 780

TGATCCGAGA ATTTAACTTA AATGAGCTAT ACCAGCGGGC CAAGAAACTA TCAAAGGCTG 840

GAGACAATAT CCCTGAGGAG CAGCCTGTGG NTTCAACCCC CACCACAGTG TCAGATGGGG 900

AAAACAAGAA GGATAAATAA GATCCTCACT TTGGCAGTGC TTCCTCTCCT GTCAATTCCA 960

GGCTCTTTCC ATAACCACAA GCCTGAGGCT GCAGCCTTTT ATTTATGTTT TCCCTTTGGC 1020

TGTGACTGGG TGGGGCAGCA TGCAGCTTCT GATTTTAAAG AGGCATCTAG GGAATTGTCA 1080

GGCACCCTAC AGGAAGGCCT GCCATGCTGT GGCCAACTGT TTCACTGGAG CAAGAAAGAG 1140

ATCTCATAGG ACGGAGGGGG AAATGGTTTC CCTCCAAGCT TGGGTYAGTG TGTTAACTGC 1200

TTATCAGCTA TTCAGACATC TCCATGGTTT CTCCATGAAA CTCTGTGGTT TCATCATTCC 1260

TTCTTAGTTG ACCTGCACAG CTTGGTTAGA CCTAGATTTA ACCCTAAGGT AAGATGCTGG 1320

GGTATAGAAC GCTAAGAATT TTCCCCCAAG GACTCTTGCT TCCTTAAGCC CTTCTGGCTT 1380

CGTTTATGGT CTTCATTAAA AGTATAAGCC TAACTTTGTC GCTAGTCCTA AGGAGAAACC 1440

TTTAACCACA AAGTTTTTAT CATTGAAGAC AATATTGAAC AACCCCCTAT TTTGTGGGGA 1500

TTGAGAAGGG GTGAATAGAG GCTTGAGACT TTCCTTTGTG TGGTAGGACT TGGAGGAGAA 1560

ATCCCCTGGA CTTTCACTAA CCCTCTGACA TACTCCCCAC ACCCAGTTGA TGGCTTTCCG 1620

TAATAAAAAG ATTGGGATTT CCTTTTGAAA AAAAAAAAAA AAAAAAAAAA AAAAAAAAAA 1680

AAAAAAAAAA AAAAAAAAG 1699

(2) INFORMATION FOR SEQ ID NO: 20:

(i) SEQUENCE CHARACTERISTICS:
(A) LENGTH: 736 base pairs
(B) TYPE: nucleic acid
(C) STRANDEDNESS: double
(D) TOPOLOGY: linear

(xi) SEQUENCE DESCRIPTION: SEQ ID NO: 20:

AAGTGAGTTA AGGACGTACT CGTCTTGGTG AGAGCGTGAC TGCTGAGATT TGGGAGTCTG 60

CGCTAGGCCC GCTTGGAGTT CTGAGCCGAT GGAAGAGTTC ACTCATGTTT GCACCCGCGG 120

TGATGCGTGC TTTTCGCAAG AACAAGACTC TCGGCTATGG AGTCCCCATG TTGTTGCTGA 180

TTGTTGGAGG TTCTTTTGGT CTTCGTGAGT TTTCTCAAAT CCGATATGAT GCTGTGAAGA 240

GTAAAATGGA TCCTGAGCTT GAAAAAAAAC TGAAAGAGAA TAAAATATCT TTAGAGTCGG 300

AATATGAGAA AATCAAAGAC TCCAAGTTTG ATGACTGGAA GAATATTCGA GGACCCAGGC 360

CTTGGGAAGA TCCTGACCTC CTCCAAGGAA GAAATCCAGA AAGCCTTAAG ACTAAGACAA 420

CTTGACTCTG CTGATTCTTT TTTCCTTTTT TTTTTTTTTA AATAAAAATA CTATTAACTG 480

GACTTCCTAA TATATACTTC TATCAAGTGG AAAGGAAATT CCAGGCCCAT GGAAACTTGG 540

ATATGGGTAA TTTGATGACA AATAATCTTC ACTAAAGGTC ATGTACAGGT TTTTATACTT 600

CCCAGCTATT CCATCTGTGG ATGAAAGTAA CAATGTTGGC CACGTATATT TTACACCTCG 660

AAATAAAAAA TGTGAATACT GCTCCAAAAA AAAAAAAAGT NGGCGAGCTT TCCCTAGGGG 720

GGTAATTNGC TGNTGC 736

(2) INFORMATION FOR SEQ ID NO: 21:

(i) SEQUENCE CHARACTERISTICS:
(A) LENGTH: 1688 base pairs
(B) TYPE: nucleic acid
(C) STRANDEDNESS: double
(D) TOPOLOGY: linear

(xi) SEQUENCE DESCRIPTION: SEQ ID NO: 21:

CAAAGAAGGG ATTCATCTTG CATTGGTGGA GCTGCTGAAA AATTTAACCA AGTACCCTAC 60

TGATAGGGAC TCCATATGGA AGTGCTTGAA GTTTCTGGGA AGTCGGCATC CAACCCTGGT 120

GCTTCCCTTG GTGCCAGAGC TTCTGAGCAC CCACCCATTT TTTGACACAG CTGAACCAGA 180

CATGGATGAT CCAGCTTATA TTGCAGTTTT GGTACTTATT TTCAATGCTG CTAAAACCTG 240

TCCAACAATG CCAGCATTGT TCTCAGATCA CACCTTCAGG CACTATGCCT ACCTCCGAGA 300

CAGTCTTTCT CATCTTGTTC CTGCCTTGAG GTTACCAGGT AGAAAACTGG TGTCATCAGC 360

TGTTTCTCCC AGCATCATAC CTCAAGAGGA TCCTTCCCAG CAGTTCCTGC AGCAGAGCCT 420

TGAAAGAGTG TATAGTCTTC AGCACTTGGA CCCTCAGGGA GCCCAGGAGC TGCTGGAATT 480

CACCATCAGG GATCTGCAAA GACTTGGAGA ACTTCAATCT GAATTGGCAG GAGTAGCTGA 540

TTTCTCTGCC ACCTATCTTC GCTGTCAACT ACTTCTCATC AAGGCCTTGC AGGAAAAGTT 600

GTGGAATGTA GCTGCCCCTT TGTATTTGAA GCAGAGTGAT TTGGCCTCAG CAGCAGCGAA 660

ACAGATTATG GAAGAGACCT ACAAAATGGA ATTCATGTAC AGTGGTGTGG AGAATAAGCA 720

GGTGGTGATT ATACATCACA TGAGGCTGCA GGCCAAAGCT TTGCAACTTA TAGTAACAGC 780

ACGAACTACA CGAGGACTTG ACCCCTTATT TGGGATGTGT GAAAAATTTT TACAGGAAGT 840

AGACTTTTTT CAGAGGTATT TCATCGCTGA TTTGCCCCAC TTGCAGGACA GCTTTGTGGA 900

CAAACTCCTT GACCTTATGC CCCGACTCAT GACATCCAAA CCTGCAGAAG TGGTCAAAAT 960

TCTACAGACC ATGCTGCGAC AGAGTGCCTT TCTGCATCTC CCGCTTCCAG AGCAGATCCA 1020

CAAAGCCTCA GCCACCATCA TCGAGCCAGC GGGCGAGTTC AGACAACCCT TTGCGGTTTA 1080

CCTCTGGGTT GGTGGTTGCC CTGGGATGTT GATGCAACCC TGGAGCATGT GCAGGATCCT 1140

CAGAACACTG TTAAGGTCCA GGGTCTTATA TCCAGATGGC CAGGSTTCAG ATGATTCACC 1200

CCAAGCCTGC AGACTTCCGG AATCCTGGCC CAGGGCGGCA CCGGCTCATC ACTCAGGTTT 1260

ATCTCTCCCA CACCGCTTGG ACAGAGGCAT GCCAGGTGGA AGTGAGGCTG CTGCTGGCCT 1320

ACAACTCCAG TGCTCGCATT CCAAAATGCC CCTGGATGGA GGGTGGTGAG ATGTCACCAC 1380

AGGTGGAAAC CAGCATCGAG GGCACCATTC CCTTCAGCAA GCCTGTAAAA GTTTATATAA 1440

TGCCCAAACC TGCACGGCGC TAAGGCAAAA ACAGTCTTCC CAACCGTGCC TAGAGGGCCC 1500

TTCTTAGGTG TCAGAATGAG CCAAGCCTGA AGCACTTCAC CTGGAATTGA TGTGTAGGCT 1560

TAAGGAGTAT GTGACCCTTA CAGTCTCATC TGGTATCAAA CACAGGATAA ATTGTTTCTT 1620

CATTAAAAAA TAAAAAACCT TCAAGTCTAC TTACCCTTCT CCTGTCCACA ATAAAGTTGA 1680

GAAAACAC 1688

(2) INFORMATION FOR SEQ ID NO: 22:

(i) SEQUENCE CHARACTERISTICS:
(A) LENGTH: 2045 base pairs
(B) TYPE: nucleic acid
(C) STRANDEDNESS: double
(D) TOPOLOGY: linear

(xi) SEQUENCE DESCRIPTION: SEQ ID NO: 22:

GAGCTCTCGG GGTATCGAGG AGGCAGGCCC GCGGGCGCAC GGGCGAGCGG GCCGGGAGCC 60

GGAGCGGCGG AGGAGCCGGC AGCAGCGGCG CGGCGRGCTC CAGGCGAGGC GGTCGACGCT 120

CCTGAAAACT TGCGCGCGCG CTCGCCCACT GCGCCCGGAG CGATGAAGAT GGTCGCGCCC 180

TGGACGCGGT TCTACTCCAA CAGCTGCTGC TTGTGCTGCC ATGTCCGCAC CGGCACCATC 240

CTGCTCGGCG TCTGGTATCT GATCATCAAT GCTGTGGTAC TGTTGATTTT ATTGAGTGCC 300

CTGGCTGATC CGGATCAGTA TAACTTTTCA AGTTCTGAAC TGGGAGGTGA CTTTGAGTTC 360

ATGGATGATG CCAACATGTG CATTGCCATT GCGATTTCTC TTCTCATGAT CCTGATATGT 420

GCTATGGCTA CTTACGGAGC GTACAAGCAA CGCGCAGCTG GGATCATCCC ATTCTTCTGT 480

TACCAGATCT TTGACTTTGC CCTGAACATG TTGGTTGCAA TCACTGTGCT TATTTATCCA 540

AACTCCATTC AGGAATACAT ACGGCAACTG CCTCCTAATT TTCCCTACAG AGATGATGTC 600

ATGTGCAGTG AATCCTACCT GTTTGGTCCT TATTATTCTT CTGTTTATTA GCATTATCTT 660

GACTTTTAAG GGTTACTTGA TTAGCTGTGT TTGGAACTGC TACCGATACA TCAATGGTAG 720

GAACTCCTCT GATGTCCTGG TTTATGTTAC CAGCAATGAC ACTACGGTGC TGCTACCCCC 780

GTATGATGAT GCCACTGTGA ATGGTGCTGC CAAGGAGCCA CCGCCACCTT ACGTGTCTGC 840

CTAAGCCTTC AAGTGGGCGG ACTGAGGGCA GCAGCTTGAC TTTGCAGACA TCTGAGCAAT 900

AGTTCTGTTA TTTCACTTTT GCCATGAGCC TCTCTGAGCT TGTTTGTTGC TGAAATGCTA 960

CTTTTTAAAA TTTAGATGTT AGATTGAAAA CTGTAGTTTT CAACATATGC TTTGCTRGAA 1020

CACTGTGATA GATTAACTGT AGAATTCTTC CTGTACGATT GGGGATATAA YGGGCTTCAC 1080

TAACCTTCCC TAGGCATTGA AACTTCCCCC AAATCTGATG GACCTAGAAG TCTGCTTTTG 1140

TACCTGCTGG GCCCCAAAGT TGGGCATTTT TCTCTCTGTT CCCTCTCTTT TGAAAATGTA 1200

AAATAAAACC AAAAATAGAC AACTTTTTCT TCAGCCATTC CAGCATAGAG AACAAAACCT 1260

TATGGAAACA GGAATGTCAA TTGTGTAATC ATTGTTCTAA TTAGGTAAAT AGAAGTCCTT 1320

ATGTATGTGT TACAAGAATT TCCCCCACAA CATCCTTTAT GACTGAAGTT CAATGACAGT 1380

TTGTGTTTGG TGGTAAAGGA TTTTCTCCAT GGCCTGAATT AAGACCATTA GAAAGCACCA 1440

GGCCGTGGGA GCAGTGACCA TCTGCTGACT GTTCTTGTGG ATCTTGTGTC CAGGGACATG 1500

GGGTGACATG CCTCGTATGT GTTAGAGGGT GGAATGGATG TGTTTGGCGC TGCATGGGAT 1560

CTGGTGCCCC TCTTCTCCTG GATTCACATC CCCACCCAGG GCCCGCTTTT ACTAAGTGTT 1620

CTGCCCTAGA TTGGTTCAAG GAGGTCATCC AACTGACTTT ATCAAGTGGA ATTGGGATAT 1680

ATTTGATATA CTTCTGCCTA ACAACATGGA AAAGGGTTTT CTTTTCCCTG CAAGCTACAT 1740

CCTACTGCTT TGAACTTCCA AGTATGTCTA GTCACCTTTT AAAATGTAAA CATTTTCAGA 1800

AAAATGAGGA TTGCCTTCCT TGTATGCGCT TTTTACCTTG ACTACCTGAA TTGCAAGGGA 1860

TTTTTATATA TTCATATGTT ACAAAGTCAG CAACTCTCCT GTTGGTTCAT TATTGAATGT 1920

GCTGTAAATT AAGTYGTTTG CAATTAAAAC AAGGTTTGCC CACATCCAAA AAAAAAAAAA 1980

AAAAAAAAAA AAAAAAAAAA AAAAAAAAAA AAAAAAAAAA AAAAAAAAAA AAAAAAAAAN 2040

NAAAA 2045

(2) INFORMATION FOR SEQ ID NO: 23:

(i) SEQUENCE CHARACTERISTICS:
(A) LENGTH: 1101 base pairs
(B) TYPE: nucleic acid
(C) STRANDEDNESS: double
(D) TOPOLOGY: linear

(xi) SEQUENCE DESCRIPTION: SEQ ID NO: 23:

TTGTTTGCCG ACCGTCAATA TTCCCGCGCC TGGACGGTTA AATAGCTAAA GCTGGCGCGG 60

GGCTGTCACC TCCGCCTCTG CTCCCCGACC CGGCCATGCG CGGCCTCGGG CTCTGGCTGC 120

TGGGCGCGAT GATGCTGCCT GCGATTGCCC CCAGCCGGCC CTGGGCCCTC ATGGAGCAGT 180

ATGAGGTCGT GTTGCCGYGG CGTCTGCCAG GCCCCCGAGT CCGCCGAGCT CTGCCCTCCC 240

ACTTGGGCCT GCACCCAGAG AGGGTGAGCT ACGTCCTTGG GGCCACAGGG CACAACTTCA 300

CCCTCCACCT GCGGAAGAAC AGGGACCTGC TGGGYTCCGG CTACACAGAG ACCTATACGG 360

CTGCCAATGG CTCCGAGGTG ACGGAGCAGC CTCGCGGGCA GGACCACTGC TTYTACCAGG 420

GCCACGTAGA GGGGTACCCG GACTCAGCCG CCAGCCTCAG CACCTGTGCC GGCCTCAGGG 480

GTTTCTTCCA GGTGGGGTCA GACCTGCACC TGATCGAGCC CCTGGATGAA GGTGGCGAGG 540

GCGGACGGCA CGCCGTGTAC CAGGCTGAGC ACCTGCTGCA GACGGCCGGG ACCTGCGGGG 600

TCAGCGACGA CAGCCTGGGC AGCCTCCTGG GACCCCGGAC GGCAGCCGTC TTCAGGCCTC 660

GGCCCGGGGA CTCTCTGCCA TCCCGAGAGA CCCGCTACGT GGAGCTGTAT GTGGTCGTGG 720

ACAATGCAGA GTTCCAGATG CTGGGGAGCG AAGCAGCCGT GCGTCATCGG GTGCTGGAGG 780

TGGTGAATCA CGTGGACAAG CTATATCAGA AACTCAACTT CCGTGTGGTC CTGGTGGGCC 840

TGGAGATTTG GAATAGTCAG GACAGGTTCC ACGTCAGCCC CGACCCCAGT GTCACACTGG 900

AGAACCTCCT GACCTGGCAR GCACGGCAAC GGACACGGCG GCACCTGCAT GACAACGTAC 960

AGCTCATCAC GGGTGTCGAC TTCAMCGGGA CTACTGTGGG GTTTGCCAGG GTGTCCACCA 1020

TGTGCTCCCA CAGCTCAGGG GCTGTGAACC AGGACCACAG CAAGAACCCC GTGGGCGTGG 1080

CCTGCACCAT GGCCCATGAG A 1101

(2) INFORMATION FOR SEQ ID NO: 24:

(i) SEQUENCE CHARACTERISTICS:
(A) LENGTH: 1659 base pairs
(B) TYPE: nucleic acid
(C) STRANDEDNESS: double
(D) TOPOLOGY: linear

(xi) SEQUENCE DESCRIPTION: SEQ ID NO: 24:

CCGGGCTGCA GGATTCGGCA CGAGGTGGGA GCCAAGAAGA AAGGTTTGCT CCCGGGTGGA 60

ACAGGGATTA TCCTCCTCCT CCCCTTAAGA GTCATGCTCA AGAGAGACAC TCTGGCAACT 120

TTCCTGGCAG AGATTCACTT CCCTTTGATT TCCAGGGGCA TTCGGGGCCT CCTTTTGCAA 180

ATGTAGAGGA GCATTCTTTC AGCTATGGAG CTAGAGACGG ACCGCATGGT GACTATCGAG 240

GAGGGGAGGG ACCTGGACAT GATTTCAGGG GGGGAGATTT TTCGTCTTCT GATTTCCAGA 300

GCAGAGATTC ATCACAGTTG GACTTCAGGG GTAGGGACAT ACATTCTGGG GATTTTCGGG 360

ATAGAGAAGG ACCACCTATG GACTATAGGG GTGGAGATGG TACTTCTATG GATTATAGAG 420

GTAGGGAGGC ACCTCATATG AACTACAGAG ACAGGGATGC TCACGCTGTT GACTTCAGAG 480

GTAGGGATGC TCCTCCATCT GACTTCAGGG GCCGGGGCAC TTATGATTTA GATTTTAGAG 540

GCCGGGATGG ATCCCATGCA GATTTTAGGG GAAGGGATTT ATCAGATTTG GATTTTAGGG 600

CCAGAGAACA GTCCCGTTCT GATTTTAGGA ATAGAGATGT ATCTGATTTG GACTTTAGAG 660

ACAAAGACGG AACACAAGTA GACTTTAGAG GCCGAGGTTC AGGTACTACT GATCTAGACT 720

TTAGGGACAG GGATACGCCA CATTCAGATT TCAGAGGTAG ACACCGATCT AGGACTGATC 780

AGGATTTTAG GGGCAGAGAG ATGGGATCTT GTATGGAATT TAAAGATAGG GAGATGCCCC 840

CTGTGGATCC AAATATTTTG GATTACATTC AGCCCTCTAC ACAAGATAGA GAACATTCTG 900

GTATGAATGT GAACAGGAGA GAAGAATCCA CACATGACCA TACGATAGAA AGGCCTGCTT 960

TTGGCATTCA GAAGGGAGAA TTTGAGCATT CAGAAACAAG AGAAGGAGAA ACACAAGGTG 1020

TAGCCTTTGA ACATGAGTCT CCAGCAGACT TTCAGAACAG CCAAAGTCCA GTTCAAGACC 1080

AAGATAAGTC ACAGCTTTCT GGACGTGAAG AGCAGAGTTC AGATGCTGGT CTGTTTAAAG 1140

AAGAAGGCGG TCTGGACTTT CTTGGGCGGC AAGACACCGA TTACAGAAGC ATGGAGTACC 1200

GTGATGTGGA TCATAGGCTG CCAGGAAGCC AGATGTTTGG CTATGGCCAG AGCAAGTCTT 1260

TTCCAGAGGG CAAAACTGCC CGAGATGCCC AACGGGACCT TCAGGATCAA GATTATAGGA 1320

CCGGCCCAAG TGAGGAGAAA CCCAGCAGGC TTATTCGATT AAGTGGGGTA CCTGAAGATG 1380

CCACAAAAGA AGAGATTCTT AATGCTTTTC GGACTCCTGA TGGCATGCCT GTAAAGAATT 1440

GCAGTTGAAG GAGTATAACA CAGGTTACGA CTATGGCTAT GTCTGCGTGG AGTTTTCACT 1500

CTTGGAAGAT GCCATCGGAT GCATGGAGGC CAACCAGGCT GGTGATTAGT AACTAAAGCA 1560

TATGCTGTGG AACATCCAGC ACTGATGCCA GATTACCTGT CCCTAATACT GAGCAGAAGC 1620

TGGTGAATGA AACAGGAGAT CCCTCAGTCA AAACAAAAA 1659

(2) INFORMATION FOR SEQ ID NO: 25:

(i) SEQUENCE CHARACTERISTICS:
(A) LENGTH: 1329 base pairs
(B) TYPE: nucleic acid
(C) STRANDEDNESS: double
(D) TOPOLOGY: linear

(xi) SEQUENCE DESCRIPTION: SEQ ID NO: 25:

TCTGTTCCTC TCTCCTGGAA GCTTGCAGAC CTCCCTTCAG AACCAATCCC AAGAAGCCAC 60

CTATCCGGAA CAACACAAGG ATGCTGCCGG ACTGGAAGAG STCCTTGATC CTCATGGCTT 120

ACATCATCAT CTTCCTCACT GGCCTCCCTG CCAACCTCCT GGCCCTGCGG GCCTTTGTGG 180

GGCGGATCCG CCAGCCCCAG CCTGCACCTG TGCACATCCT CCTGCTGAGC CTGACGCTGG 240

CCGACCTCCT CCTGCTGCTG CTGCTGCCCT TCAAGATCAT CGAGGCTGCG TCGAACTTCC 300

GCTGGTACCT GCCCAAGGTC GTCTGCGCCC TCACGAGTTT TGGSTTCTAC AGCAGCATCT 360

ACTGCAGCAC GTGGCTCCTG GCGGGCATCA GCATCGAGCG CTACCTGGGA GTGGCTTTCC 420

CCGTGCAGTA CAAGCTCTCC CGCCGGCCTC TGTATGGAGT GATTGCAGCT CTGGTGGCCT 480

GGGTTATGTC CTTTGGTCAC TGCACCATCG TGATCATCGN TCAATACTTG AACACGACTG 540

AGCAGGTCAG AAGTGGCAAT GAAATTACCT GCTACGAGAA CTTCACCGAT AACCAGTTGG 600

ACGTGGTGCT GCCCGTGMGG STGGAGCTGT GCCTGGTGCT CTTCTTCATS CCCATGGCAG 660

TCACCATCTT CTGCTACTGG CGTTTTGTGT GGATCATGCT CTCCCAGCCC CTTGTGGGGG 720

CCCAGAGGCG GCGCCGAGCC GTGGGGCTGG CTGTGGTGAC GCTGCTCAAT TTCCTGGTGT 780

GCTTCGGACC TTACAACGTG TCCCACCTGG TGGGGTATCA CCAGAGAAAA AGCCCCTGGT 840

GGCGGTCAAT AGCCGTGKTG TTCAGTTCAC TCAACGCCAG TCTGGACCCC CTGCTCTTCT 900

ATTTCTCTTC TTCAGTGGTG CGCAGGGCAT TTGGGAGAGG GCTGCAGGTG CTGCGGAATC 960

AGGGCTCCTC CCTGTTGGGA CGCAGAGGCA AAGACACAGC AGAGGGGACA AATGAGGACA 1020

GGGGTGTGGG TCAAGGAGAA GGGATGCCAA GTTCGGACTT CACTACAGAG TAGCAGTTTC 1080

CCTGGACCTT CAGAGGTCGC CTGGGTTACA CAGGAGCTGG GAAGCCTGGG AGAGGCGGAN 1140

CAGGAAGGCT CCCATCCAGA TTCAGAAATC CTTAGACCCA GCCCAGGACT GCGACTTTGA 1200

AAAAAATGCC TTTCACCAGC TTGGTATCCC TTCCTGACTG AATTGTCCTA CTCAAAGGAG 1260

CATAAGTCAG AGATGCACGA AGAAGTAGTT AGGTATAGAA GCACCTGCCG GGTGTGGTGG 1320

CTCATGCCT 1329

(2) INFORMATION FOR SEQ ID NO: 26:

(i) SEQUENCE CHARACTERISTICS:
(A) LENGTH: 700 base pairs
(B) TYPE: nucleic acid
(C) STRANDEDNESS: double
(D) TOPOLOGY: linear

(xi) SEQUENCE DESCRIPTION: SEQ ID NO: 26:

GGCAGAGAGC ACCATCTGTC ATGGCGGCTG GGCTGTTTGG TTTGAGCGCT CGCCGTCTTT 60

TGGCGGCAGC GGCGACGCGA NGGGCTCCCG GCCGCCCGCG TCCGCTGGGA ATCTAGCTTC 120

TCCAGGACTG TGGTCGCCCC GTCCGCTGTG GCGGRAAAGC GGCCCCCAGA ACCGACCACA 180

CCGTGGCAAG AGGACCCAGA ACCCGAGGAC GAAAACTTGT ATGAGAAGAA CCCAGACTCC 240

CATGGTTATG ACAAGGACCC CGTTTTGGAC GTCTGGAACA TGCGACTTGT CTTCTTCTTT 300

GGCGTCTCCA TCATCCTGGT CCTTGGCAGC ACCTTTGTGG CCTATCTGCC TGACTACAGG 360

TGCACAGGGT GTCCAAGAGC GTGGGATGGG ATGAAAGAGT GGTCCCGCCG CGAAGCTGAG 420

AGGCTTGTGA AATACCGAGA GGCCAATGGC CTTCCCATCA TGGAATCCAA CTGCTTCGAC 480

CCCAGCAAGA TCCAGCTGCC AGAGGATGAG TGACCAGTTG CTAAGTGGGG CTCAAGAAGC 540

ACCGCCTTCC CCACCCCCTG CCTGCCATTC TGACCTCTTC TCAGAGCACC TAATTAAAGG 600

GGCTGAAAGT CTGAAAAAAA AAAAAAAAAA AAAAAAAAAA AAAAAAAAAA AAAAAAAANA 660

AAAAAAAAAA AAAAAAAAAA AAAAAAAAAA ANGGGGGGGN 700

(2) INFORMATION FOR SEQ ID NO: 27:

(i) SEQUENCE CHARACTERISTICS:
(A) LENGTH: 832 base pairs
(B) TYPE: nucleic acid
(C) STRANDEDNESS: double
(D) TOPOLOGY: linear

(xi) SEQUENCE DESCRIPTION: SEQ ID NO: 27:

GGCACGAGCT CCACTCGGTT TCTCTCTTTG CAGGAGCACC GGCAGCACCA GTGTGTGAGG 60

GGAGCAGGCA GCGGTCCTAG CCAGTTCCTT GATCCTGCCA GACCACCCAG CCCCTGGCAC 120

AGAGCTGCTC CACAGGCACC ATGAGGATCA TGCTGCTATT CACAGCCATC CTGGCCTTCA 180

GCCTAGCTCA GAGCTTTGGG GCTGTCTGTA AGGAGCCACA GGAGGAGGTG GTTCCTGGCG 240

GGGGCCGCAG CAAGAGGGAT CCAGATCTCT ACCAGCTGCT CCAGAGACTC TTCAAAAGCC 300

ACTCATCTCT GGAGGGATTG CTCAAAGCCC TGAGCCAGGC TAGCACAGAT CCTAAGGAAT 360

CAACATCTCC CGAGAAACGT GACATGCATG ACTTCTTTGT GGGACTTATG GGCAAGAGGA 420

GCGTCCAGCC AGACTCTCCT ACGGATGTGA ATCAAGAGAA CGTCCCCAGC TTTGGCATCC 480

TCAAGTATCC CCCGAGAGCA GAATAGGTAC TCCACTTCCG GACTCCTGGA CTGCATTAGG 540

AAGACCTCTT TCCCTGTCCC AATCCCCAGG TGCGCACGCT CCTGTTACCC TTTCTCTTCC 600

CTGTTCTTGT AACATTCTTG TGCTTTGACT CCTTCTCCAT CTTTTCTACC TGACCCTGGT 660

GTGGAAACTG CATAGTGAAT ATCCCCAACC CCAATGGGCA TTGACTGTAG AATACCCTAG 720

AGTTCCTGTA GTGTCCTACA TTAAAAATAT AATGTCTCTC TCTATTCCTC AACAATAAAG 780

GATTTTTGCA TATGAAAAAA AAAAAAAAAA AAAAAAAAAA NAAANAAAAA AA 832

(2) INFORMATION FOR SEQ ID NO: 28:

(i) SEQUENCE CHARACTERISTICS:
(A) LENGTH: 2361 base pairs
(B) TYPE: nucleic acid
(C) STRANDEDNESS: double
(D) TOPOLOGY: linear

(xi) SEQUENCE DESCRIPTION: SEQ ID NO: 28:

GGCACGAGGC TCCCTAAGCG GTTGTCACCG CTGGAGACGG TTGGGAGAAC CGTTGTGGCG 60

AGCGCTACAC GAGGCAAACG ACTTCTCCCT TCTTTGAACT GGACCCCGCG AGCACCAGAG 120

TCGGCGTAAC TATCGCCTGA CAGGCATTTA AATCAAACGG TATTGAGATG GATTGGGTTA 180

TGAAACATAA TGGTCCAAAT GACGCTATGA TGGGACAGTA CGACTTCGTG GACTACCATT 240

TGGTTGCAGC AAAGAGGAAA TAGTTCAGTT CTTTCAAGGG TTGGAAATCG TGCCAATGGG 300

ATAACATTGA CGATGGACTA CCAGGGGAGA AGCACAGGGG AGGCCTTCGT GCAGTTTGCT 360

TCAAAGGAGA TAGCAGAAAA TGCTCTGGGG AAACACAAGG AAAGAATAGG GCACAGGTAT 420

ATTGAGATCT TCAGAAGTAG CAGGAGTGAA ATCAAAGGAT TTTATGATCC ACCAAGAAGA 480

TTGCTGGGAC AGCGACCGGG ACCATATGAT AGACCAATAG GAGGAAGAGG GGGTTATTAT 540

GGAGCTGGGC GTGGAAGTAT GTATGACAGA ATGCGACGAG GAGGTGATGG ATATGATGGT 600

GGTTATGGAG GTTTTGATGA CTATGGTGGC TATAATAATT ACGGCTATGG GAATGATGGC 660

TTTGATGACA GAATGAGAGA TGGAAGAGGT ATGGGAGGAC ATGGCTATGG TGGAGCTGGT 720

GATGCAAGTT CAGGTTTTCA TGGTGGTCAT TTCGTACATA TGAGAGGGTT GCCTTTTCGT 780

GCAACTGAAA ATGACATTGC TAATTTCTTC TCACCACTAA ATCCAATACG AGTTCATATT 840

GATATTGGAG CTGATGGCAG AGCACAGGAG AAGCAGATGT AGAGTTTGTG ACACATGAAG 900

ATGCAGTAGC TGCCATGTCT AAAGATAAAA ATAACATGCA ACATCGATAT ATTGAACTCT 960

TCTTGAATTC TACTCCTGGA GGCGGCTCTG GCATGGGAGG TTCTGGAATG GGAGGCTACG 1020

GAAGAGATGG AATGGATAAT CAGGGAGGCT ATGGATCAGT TGGAAGAATG GGAATGGGGA 1080

ACAATTACAG TGGAGGATAT GGTACTCCTG ATGGTTTGGG TGGTTATGGC CGTGGTGGTG 1140

GAGGCAGTGG AGGTTACTAT GGGCAAGGCG GCATGAGTGG AGGTGGATGG CGTGGGATGT 1200

ACTGAAAGCA AAAACACCAA CATACAAGTC TTGACAACAG CATCTGGTCT ACTAGACTTT 1260

CTTACAGATT TAATTTCTTT TGTATTTTAA GAACTTTATA ATGACTGAAG GAATGTGTTT 1320

TCAAAATATT ATTTGGTAAA GCAACAGATT GTGATGGGAA AATGTTTTCT GTAGGTTTAT 1380

TTGTTGCATA CTTTGACTTA AAAATAAATT TTTATATTCA AACCACTGAT GTTGATACTT 1440

TTTATATACT AGTTACTCCT AAAGATGTGC TGCCTTCATA AGATTTGGGT TGATGTATTT 1500

TACTATTAGT TCTACAAGAA GTAGTGTGGT GTAATTTTAG AGGATAATGG TTCACCTCTG 1560

CGTAAACTGC AAGTCTTAAG CAGACATCTG GAATAGAGCT TGACAAATAA TTAGTGTAAC 1620

TTTTTTCTTT AGTTCCTCCT GGACAACACT GTAAATATAA AGCCTAAAGA TGAAGTGGCT 1680

TCAGGAGTAT AAATTCAGCT AATTATTTCT ATATTATTAT TTTTCAAATG TCATTTATCA 1740

GGCATAGCTC TGAAACATTG ATGATCTAAG AGGTATTGAT TTCTGAATAT TCATAATTGT 1800

GTTACCTGGG TATGAGAGTG TTGGAAGCTG AATTCTAGCC CTAGATTTTG GAGTAAAACC 1860

CCTTCAGCAC TTGACCGAAA TACCAAAAAT GTCTCCAAAA AATTGATAGT TGCAGGTTAT 1920

CGCAAGATGT CTTAGAGTAG GGTTAAGGTT CTCAGTGACA CAAGAATTCA GTATTAAGTA 1980

CATAGGTATT TACTATGGAG TATAATTCTC ACAATTGTAT TTTCAGTTTT CTGCCCAATA 2040

GAGTTTAAAT AACTGTATAA ATGATGACTT TAAAAAAATG TAAGCAACAA GTCCATGTCA 2100

TAGTCAATAA AAACAATCCT GCAGTTGGGT TTTGTATCTG ATCCCTGCTT GGAGTTTTAG 2160

TTTAAAGAAT CTATATGTAG CAAGGAAAAG GTGCTTTTTA ATTTTAATCC CTTTGATCAA 2220

TATGGCTTTT TTCCAAATTG GCTAATGGAT CAAAATGAAA CCTGTTGATG TGAATTCAGT 2280

TATTGAACTT GTTACTTGTT TTTGCCAGAA ATGTTATTAA TAAATGTCAA TGTGGGAGAT 2340

AATAAAAAAA AAAAAAAAAA N 2361

(2) INFORMATION FOR SEQ ID NO: 29:

(i) SEQUENCE CHARACTERISTICS:
(A) LENGTH: 879 base pairs
(B) TYPE: nucleic acid
(C) STRANDEDNESS: double
(D) TOPOLOGY: linear

(xi) SEQUENCE DESCRIPTION: SEQ ID NO: 29:

GGAATCTGCA CCATGCCCTG GGTTCTGCTC CTCCTGACCC TCCTCACTCA CTCTGCAGTG 60

TCAGTGGTCC AGGCAGGGCT GACTCAGCCC CCCTCGGTGT CCAAGGACTT GAGACAGACC 120

GCCACACTCA CCTGCACCGG GAACAACAAC AATGTTGGCG ACCAAGGAGC AGCTTGGCTG 180

CAGCAGCACC AGGGCCACCC TCCCAAACTC CTGTCCTACA GGAATAATAA CCGGCCCTCA 240

GGGATCTCAG AGAGATTATC TGCATCCAGG TCAGGAGCCA CATCCTCCCT GACCATTACT 300

GGACTCCAGC CTGAGGACGA GGCTGACTAT TACTGCGCAG CATATGACAG CAGCCTCGCA 360

GTTTGGATGT TCGGCGGAGG GACCAAGCTG ACCGTCCTAG GTCAGCCCAA GGCTGCCCCC 420

TCGGTCACTC TGTTCCCACC CTCCTCTGAG GAGCTTCAAG CCAACAAGGC CACACTGGTG 480

TGTCTCATAA GTGACTTCTA CCCGGGAGCC GTGACAGTGG CCTGGAAGGC AGATAGCAGC 540

CCCGTCAAGG CGGGAGTGGA GACCACCACA CCCTCCAAAC AGAGCAACAA CAAGTACGCG 600

GCCAGCAGCT ACCTGAGCCT GACGCCTGAG CAGTGGAAGT CCCACAGAAG CTACAGCTGC 660

CAGGTCACGC ATGAAGGGAG CACCGTGGAG AAGACGGTGG CCCCTACAGA ATGTTCATAG 720

GTTCCCAACT CTAACCCCAC CCACGGGAGC CTGGAGCTGC AGGATCCCAG GGGAGGGGTC 780

TCTCTCCCCA TCCCAAGTCA TCCAGCCCTT CTCCCTGCAC TCATGAAACC CCAATAAATA 840

TTCTCATTGT CAATCAGAAA AAAAAAAAAA AAAAAAAAA 879

(2) INFORMATION FOR SEQ ID NO: 30:

(i) SEQUENCE CHARACTERISTICS:
(A) LENGTH: 1732 base pairs
(B) TYPE: nucleic acid
(C) STRANDEDNESS: double
(D) TOPOLOGY: linear

(xi) SEQUENCE DESCRIPTION: SEQ ID NO: 30:

GTTCGGAGGG AAACGTGTAT TGTGGTCTCA AGMMTTGCCC CAWATTAACC TGTGCCTTCC 60

CAGTCTCTGT TCCAGATTCC TGCTGCCGGG TATGCAGAGG AGATGGAGAA CTGTCATGGG 120

AACATTCTGA TGGTGATATC TTCCGGCAAC CTGCCAACAG AGAAGCAAGA CATTCTTACC 180

ACCGCTCTCA CTATGATCCT CCACCAAGCC GACAGGCTGG AGGTCTGTCC CGCTTTCCTG 240

GGGCCAGAAG TCACCGGGGA GCTCTTATGG ATTCCCAGCA AGCATCAGGA ACCATTGTGC 300

AAATTGTCAT CAATAACAAA CACAAGCATG GACAAGTGTG TGTTTCCAAT GGAAAGACCT 360

ATTCTCATGG CGAGTCCTGG CACCCAAACC TCCGGGCATT TGGCATTGTG GAGTGTGTGC 420

TATGTACTTG TAATGTCACC AAGCAAGAGT GTAAGAAAAT CCACTGCCCC AATCGATACC 480

CCTGCAAGTA TCCTCAAAAA ATAGACGGAA AATGCTGCAA GGTGTGTCCA GAAGAACTTC 540

CAGGCCAAAG CTTTGACAAT AAAGGCTACT TCTGCGGGGA AGAAACGATG CCTGTGTATG 600

AGTCTGTATT CATGGAGGAT GGGGAGACAA CCAGAAAAAT AGCACTGGAG ACTGAGAGAC 660

CACCTCAGGT AGAGGTCCAC GTTTGGACTA TTCGAAAGGG CATTCTCCAG CACTTCCATA 720

TTGAGAAGAT CTCCAAGAGG ATGTTTGAGG AGCTTCCTCA CTTCAAGCTG GTGACCAGAA 780

CAACCCTGAG CCAGTGGAAG ATCTTCACCG AAGGAGAAGC TCAGATCAGC CAGATGTGTT 840

CAAGTCGTGT ATGCAGAACA GAGCTTGAAG ATTTAGTCAA GGTTTTGTAC CTGGAGAGAT 900

CTGAAAAGGG CCACTGTTAG GCAAGACAGA CAGTATTGGA TAGGGTAAAG CAAGAAAACT 960

CAAGCTGCAG CTGGACTGCA GGCTTATTTT GCTTAAGTCA ACAGTGCCCT AAAACTCCAA 1020

ACTCAAATGC AGTCAATTAT TCACGCCATG CACAGCATAA TTTGCTCCTT TGTGTGTGTG 1080

TGTGTGTGTG TGTGTGTGTG TGTGGTAAAG GGGGGAAGGT GTTATGCGGC TGCTCCCTCC 1140

GTCCCAGAGG TGGCAGTGAT TCCATAATGT GGAGACTAGT AACTAGATCC TAAGGCAAAG 1200

AGGTGTTTCT CCTTCTGGAT GATTCATCCC AAAGCCTTCC CACCCAGGTG TTCTCTGAAA 1260

GCTTAGCCTT AAGAGAACAC GCAGAGAGTT TCCCTAGATA TACTCCTGCC TCCAGGTGCT 1320

GGGACACACC TTTGCAAAAT GCTGTGGGAA GCAGGAGCTG GGGAGCTGTG TTAAGTCAAA 1380

GTAGAAACCC TCCAGTGTTT GGTGTTGTGT AGAGAATAGG ACATAGGGTA AAGAGGCCAA 1440

GCTGCCTGTA GTTAGTAGAG AAGAATGGAT GTGGTTCTTC TTGTGTATTT ATTTGTATCA 1500

TAAACACTTG GAACAACAAA GACCATAAGC ATCATTTAGC AGTTGTAGCC ATTTTCTAGT 1560

TAACTCATGT AAACAAGTAA GAGTAACATA ACAGTATTAC CCTTTCACTG TTCTCACAGG 1620

ACATGTACCT AATTATGGTA CTTATTTATG TAGTCACTGT ATTTCTGGAT TTTTAAATTA 1680

ATAAAAAAGT TAATTTTGAA AAATCAAAAA AAAAAAAAAA AAAAAAACTC GA 1732

(2) INFORMATION FOR SEQ ID NO: 31:

(i) SEQUENCE CHARACTERISTICS:
(A) LENGTH: 3259 base pairs
(B) TYPE: nucleic acid
(C) STRANDEDNESS: double
(D) TOPOLOGY: linear

(xi) SEQUENCE DESCRIPTION: SEQ ID NO: 31:

TTTGCAGTAC GGGCCGGATT TCCCGGGTCG ACCCACGCGT CCGCGGAGGC TACGTGAAGA 60

GAGGCGCGGC GTGACTGAGC TACGGTTCTG GCTGCGTCCT AGAGGCATCC GGGGCAGTAA 120

AACCGCTGCG ATCGCGGAGG CGGCGGCCAG GCCGAGAGGC AGGCCGGGCA GGGGTGTCGG 180

ACGCAGGGCG CTGGGCCGGG TTTCGGCTTC GGCCACAGCT TTTTTTCTCA AGGTGCAATG 240

AAAGCCTTCC ACACTTTCTG TGTTGTCCTT CTGGTGTTTG GGAGTGTCTC TGAAGCCAAG 300

TTTGATGATT TTGAGGATGA GGAGGACATA GTAGAGTATG ATGATAATGA CTTCGCTGAA 360

TTTGAGGATG TCATGGAAGA CTCTGTTACT GAATCTCCTC AACGGGTCAT AATCACTGAA 420

GATGATGAAG ATGAGACCAC TGTGGAGTTG GAAGGGCAGG ATGAAAACCA AGAAGGAGAT 480

TTTGAAGATG CAGATACCCA GGAGGGAGAT ACTGAGAGTG AACCATATGA TGATGAAGAA 540

TTTGAAGGTT ATGAAGACAA ACCAGATACT TCTTCTAGCA AAAATAAAGA CCCAATAACG 600

ATTGTTGATG TTCCTGCACA CCTCCAGAAC AGCTGGGAGA GTTATTATCT AGAAATTTTG 660

ATGGTGACTG GTCTGCTTGC TTATATCATG AATTACATCA TTGGGAAGAA TAAAAACAGT 720

CGCCTTGCAC AGGCCTGGTT TAACACTCAT AGGGAGCTTT TGGAGAGCAA CTTTACTTTA 780

GTGGGGGATG ATGGAACTAA CAAAGAAGCC ACAAGCACAG GAAAGTTGAA CCAGGAGAAT 840

GAGCACATCT ATAACCTGTG GTGTTCTGGT CGAGTGTGCT GTGAGGGCAT GCTTATCCAG 900

CTGAGGTTCC TCAAGAGACA AGACTTACTG AATGTCCTGG CCCGGATGAT GAGGCCAGTG 960

AGTGATCAAG TGCAAATAAA AGTAACCATG AATGATGAAG ACATGGATAC CTACGTATTT 1020

GCTGTTGGCA CACGGAAAGC CTTGGTGCGA CTACAGAAAG AGATGCAGGA TTTGAGTGAG 1080

TTTTGTAGTG ATAAACCTAA GTCTGGAGCA AAGTATGGAC TGCCGGACTC TTTGGCCATC 1140

CTGTCAGAGA TGGGAGAAGT CACAGACGGA ATGATGGATA CAAAGATGGT TCACTTTCTT 1200

ACACACTATG CTGACAAGAT TGAATCTGTT CATTTTTCAG ACCAGTTCTC TGGTCCAAAA 1260

ATTATGCAAG AGGAAGGTCA GCCTTTAAAG CTACCTGACA CTAAGAGGAC ACTGTTGTTT 1320

ACATTTAATG TGCCTGGCTC AGGTAACACT TACCCAAAGG ATATGGAGGC ACTGCTACCC 1380

CTGATGAACA TGGTGATTTA TTCTATTGAT AAAGCCAAAA AGTTCCGACT CAACAGAGAA 1440

GGCAAACAAA AAGCAGATAA GAACCGTGCC CGAGTAGAAG AGAACTTCTT GAAACTGACA 1500

CATGTGCAAA GACAGGAAGC AGCACAGTCT CGGCGGGAGG AGAAAAAAAG AGCAGAGAAG 1560

GAGCGAATCA TGAATGAGGA AGATCCTGAG AAACAGCGCA GGCTGGAGGA GGCTGCATTG 1620

AGGCGTGAGC AAAAGAAGTT GGAAAAGAAG CAAATGAAAA TGAAACAAAT CAAAGTGAAA 1680

GCCCATGTAA AGCCATCCCA GAGATTTGAG TTCTGATGCC ACCTGTAAGC TCTGAATTCA 1740

CAGGAAACAT GAAAAACGCC AGTCCATTTC TCAACCTTAA ATTTCAGACA GTCTTGGGCA 1800

ACTGAGAAAT CCTTATTTCA TCATCTACTC TGTTTGGGGT TTGGGTTTTA CAGAGATTGA 1860

AGATACCTGG AAAGGGCTCT GTTTCCAAGA ATTTTTTTTT CCAGATAATC AAATTATTTT 1920

GATTATTTTA TAAAAGGAAT GATCTATGAA ATCTGTGTAG GTTTTAAATA TTTTAAAAAT 1980

TATAATACAA ATCATCAGTG CTTTTAGTAC TTCAGTGTTT AAAGAAATAC CGTGAAATTT 2040

ATAGGTAGAT AACCAGATTG TTGCTTTTTG TTTAAACCAA GCAGTTGAAA TGGCTATAAA 2100

GACTGACTCT AAACCAAGAT TCTGCAAATA ATGATTGGAA TTGCACAATA AACATTGCTT 2160

GATGTTTTCT ATTTCAGGGA CCCAGAACAT AATGTAGTGT ATGTTTTTAG GTGGGAGATG 2220

CTGATAACAA AATTAATAGG AAGTCTGTAG GCATTAGGAT ACTGACATGT ACATGGAAAA 2280

TTCTAGGGAC AGGAGCATCA TTTTTTCCTT ACCTGATACC ACGAACCAGT GACAACGTGA 2340

ATGCTGTATT TTAAGTGGTT GTATGTTTAT TTTCTGGAGT AACAAATGCA TGAAAAATTA 2400

ATGCTTCACC TAGGTAAGAT CATTGGTCTG TGTGAAATCA CAAATGTTTT TTCCTTCTTG 2460

GTTGCTGCAG CCTGGTGGAT GTTCATGGAG AAGCTCTGTT CTCTATATTA TGGCTGTGTG 2520

CCGTTGCTTC TCCCTCTGCT TTTATCTTTT CCACAGTTGA GGCTGGGTAT GTTCTTTCAA 2580

AGAAATGGCC ATGAATATGT GTAAGTATAC TTTTGAAAAT GAGCTTTCCT AAACTATTGA 2640

GAGTTCTTTC CACCTCTTGC GGAACCAACT CTTGGAGGAG AGGCCCATGT ATCTGCACGA 2700

GCACTTAGCT TGTTCAGATC TCTGCATTTT ATAAATGCTT CTTACCAAGA AAGCATTTTT 2760

AGGTCATTGC TTGTACCAGG TAATTTTTGC CGGGGATGGG TAAGGGTTGG GTTTTCTGGT 2820

GGGAGTGGGG TGGTGGGTAT TTTTTGTTGA TGCTTTAGTG CAGGCCTGTT CTGAGGCAAT 2880

AACAAGTTGC TGTGAAAACG CATGTGCTGC TGCCTTTGTA ACTGCCATGG AAACTTTTCA 2940

CATGGGTTTT TCTCCAAGTT AATACAGAAA TATGTAAACT GAGAGATGCA AATGTAATAT 3000

TTTTAACAGT TCATGAAGTT GTTATTAAAA TAACTAACAT AAAACTTAAT TACTTTAATA 3060

TTATATAATT ATAGTAGTGG CCTTGTTTTA CAAACCTTTA AATTACATTT TAGAAATCAA 3120

AGTTGATAGT CTTAGTTATC TTTTGAGTAA GAAAAGCTTT CCTAAAGTCC CATACATTTG 3180

GACCATGGCA GCTAATTTTG TAACTTAAGC ATTCATATGA ACTACCTATG GACATCTATT 3240

AAAGTGATTG ACAAAAAAA 3259

(2) INFORMATION FOR SEQ ID NO: 32:

(i) SEQUENCE CHARACTERISTICS:
(A) LENGTH: 454 base pairs
(B) TYPE: nucleic acid
(C) STRANDEDNESS: double
(D) TOPOLOGY: linear

(xi) SEQUENCE DESCRIPTION: SEQ ID NO: 32:

GGCACGAGGT CTTGTCTGCG AAGAGTTTAC GAGGTTTCAC CCACTCCTTC ATTCTTGAAC 60

ATGCTTTTTC TCTGCTTATT ACCCTCCCTG TTTCCTCCTG GGCTGCCAAC AACACATTAT 120

ATTACCTCCA TCTGCAACCA GAGCTGCTAC CACCACTGTG CCCGAGCCTG AATTTTCATA 180

GTTATATTAA AAAAAATCAA GGTGCTGGGA TTACAGGCGT GAGCCACCGC GCCCGGCTGT 240

AGCCCCTGTC TTTATTCCTC CCCTGTCTAA CCCGTCCTCA GCATGAATGC CAGAGTTACC 300

TCTTAAAWTA TGTCAGGGTG CTAGGCACAG TGGCTCATGC CTGTAATCCC AGCTCTTGGG 360

AAGGCAGAGG CAGGAGGACA AMTTGAGCCC AGGAGTTTGA GACCTGCTTG GGGAATGTAG 420

TGAGACCTTG TTCTCCACAA AAAGGAAAAA AAAA 454

(2) INFORMATION FOR SEQ ID NO: 33:

(i) SEQUENCE CHARACTERISTICS:
(A) LENGTH: 230 base pairs
(B) TYPE: nucleic acid
(C) STRANDEDNESS: double
(D) TOPOLOGY: linear

(xi) SEQUENCE DESCRIPTION: SEQ ID NO: 33:

GCTGCTATGG CTGAACTTTT ATTGANCGTG TTGTCTGTGC AGAGCGCTGT GCACGAGGTG 60

GAAGCAAACG AGGGAGGAAA ACAAAGCCAC ACCCCTGCCC ACAGAGGATG GAACAGAAGG 120

GCCGCTGAGG TCAGGAAGGC AAGGTTGCCA CTAGGTGTTA CTGTGGGGCC CAGATGCCGC 180

CATGCTGTTC ACCCTTCAAA GGGTGGCATC TCAGCCCANG CAGTCCTCCT 230

(2) INFORMATION FOR SEQ ID NO: 34:

(i) SEQUENCE CHARACTERISTICS:
(A) LENGTH: 753 base pairs
(B) TYPE: nucleic acid
(C) STRANDEDNESS: double
(D) TOPOLOGY: linear

(xi) SEQUENCE DESCRIPTION: SEQ ID NO: 34:

GGCACGAGGA AAGGCTGGCC TCTCTTCAAC ATGGGATCTT CTGGACTTTT GAGCCTCCTG 60

GTGCTATTCG TCCTCTTAGC GAATGTCCAG GGACCTGGTC TGACTGATTG GTTATTTCCC 120

AGGAGATGTC CCAAAATCAG AGAAGAATGT GAATTCCAAG AAAGGGATGT GTGTACAAAG 180

GACAGACAAT GCCAGGACAA CAAGAAGTGT TGTGTCTTCA GCTGCGGAAA AAAATGTTTA 240

GATCTCAAAC AAGATGTATG CGAAATGCCA AAAGAAACTG GCCCCTGCCT GGCTTATTTT 300

CTTCATTGGT GGTATGACAA GAAAGATAAT ACTTGCTCCA TGTTTGTCTA TGGTGGCTGC 360

CAGGGAAACA ATAACAACTT CCAATCCAAA GCCAACTGCC TGAACACCTG CAAGAATAAA 420

CGCTTTCCCT GATTGGATAA GGATGCACTG GAAGAACTGC CAGAATGTGG CTCATGCTCT 480

GAGTACTGTT CCTGTACCTG ACTGATGCTC CAGACTGGCT TCCAGTTTCA CTCTCAGCAT 540

TCCAAGATCT TAGCCCTTCC CAGAACAGAA CGCTTGCATC TACCTCCTCT TCCTCCATCT 600

TTGGCTCTTT TGATGCACAA TATCCATCCG TTTTGATTTC ATCTTTATGT CCCCTTTATC 660

TCCAACTTCT AGAACTCCCA GTTTATACCT GTGTCACTCT CAATTTTTTC CAGTAAAGTA 720

CTTGATGTAG TAAAAAAAAA AAAAAAAAAA AAA 753

(2) INFORMATION FOR SEQ ID NO: 35:

(i) SEQUENCE CHARACTERISTICS:
(A) LENGTH: 1022 base pairs
(B) TYPE: nucleic acid
(C) STRANDEDNESS: double
(D) TOPOLOGY: linear

(xi) SEQUENCE DESCRIPTION: SEQ ID NO: 35:

CGCTCCTGCC GCCGGGACCC TCGACCTCCT CAGAGCAGCC GGCTGCCGCC CCGGGAAGAT 60

GGCGAGGAGG AGCCGCCACC GCCTCCTCCT GCTGCTGCTG CGCTACCTGG TGGTCGCCCT 120

GGGCTATCAT AAGGCCTATG GGTTTTCTGC CCCAAAAGAC CAACAAGTAG TCACAGCAGT 180

AGWGTACCAA GAGGCTATTT TAGCCTGCAA AACCCCAAAG AAGACTGTTT SCTCCAGATT 240

AGAGTGGAAG AAACTGGGTC GGAGTGTCTC CTTTGTCTAC TATCAACAGA CTCTTCAAGG 300

TGATTTTAAA AATCGAGCTG AGATGATAGA TTTCAATATC CGGATCAAAA ATGTGACAAG 360

AAGTGATGCG GGGAAATATC GTTGTGAAGT TAGTGCCCCA TCTGAGCAAG GCCAAAACCT 420

GGAAGAGGAT ACAGTCACTC TGGAAGTATT AGTGGCTCCA GCAGTTCCAT CATGTGAAGT 480

ACCCTCTTCT GCTCTGAGTG GAACTGTGGT AGAGCTACGA TGTCAAGACA AAGAAGGGAA 540

TCCAGCTCCT GAATACACAT GGTTTAAGGA TGGCATCCGT TTGCTAGAAA ATCCCAGACT 600

TGGCTCCCAA AGCACCAACA GCTCATACAC AATGAATACA AAAACTGGAA CTCTGCAATT 660

TAATACTGTT TCCAAACTGG ACACTGGAGA ATATTCCTGT GAAGCCCGCA ATTCTGTTGG 720

ATATCGCAGG TGTCCTGGGA AACGAATGCA AGTAGATGAT CTCAACATAA GTGGCATCAT 780

AGCAGCCGTA GTAGTTGTGG CCTTAGTGAT TTCCGTTTGT GGCCTTGGTG TATGCTATGC 840

TCAGAGGAAA GGCTACTTTT CAAAAGAAAC CTCCTTCCAG AAGAGTAATT CTTCATCTAA 900

AGCCACGACA ATGAGTGAAA ATGATTTCAA GCACACAAAA TCCTTTATAA TTTAAAGACT 960

CCACTTTAGA GATACACCAA AGCCACCGTT GTTACACAAG TTATTAAACT ATTATAAAAC 1020

TC 1022

(2) INFORMATION FOR SEQ ID NO: 36:

(i) SEQUENCE CHARACTERISTICS:
(A) LENGTH: 3044 base pairs
(B) TYPE: nucleic acid
(C) STRANDEDNESS: double
(D) TOPOLOGY: linear

(xi) SEQUENCE DESCRIPTION: SEQ ID NO: 36:

CTCTAAGAAC CTAGTGGATC CCCCCGGCCT GCAGGAATTC GGGCACGAGG GGAGACTGCT 60

GTGGCTAAGG AGGGCGGGAA GGGCCCTCTG TGGGGCTGCC ATTTTGGCTG GGACCTAAAT 120

GCAGTAAAGG AGCAGCTACG GGAATATAGA GAGTGGGGCT TCCAGGCAGA GAAGCCTGCA 180

GTGCAAAGGT CTGCAGACAA CGACCTGGGC GTCTTCAAGG GACACAAGGA ATCATATTGC 240

CAGAACACAT TGTACAGGTA GCCAGGTGTC GGTCTCCAGC CTGAGAACTC TGGCTGTTGT 300

TCCTTGTGTC GTCCCATATT CCTGCCTGGC CTGCGATGGA CATCAGCAAG GGCCTCCCAG 360

GCATGCAGGG AGGCCTCCAC ATATGGATCT CTGAGAACCG GAAGATGGTG CCGGTACCCG 420

AGGGGGCTTA CGGGAACTTT TTCGAGGAAC ACTGCTATGT CATCCTCCAC GTCCCCCAGA 480

GCCCGAAGGY CACGCAGGGG GCGTCCAGCG ACCTGCACTA CTGGGTCGGG AAGCAGGCGG 540

GTGCGGAAGC GCAGGGCGCT GCGGAGGCCT TCCAGCAGCG CCTACAGGAC GAGCTGGGGG 600

GCCAGACCGT GCTGCACCGC GAGGCGCAGG GCCACGAGTC CGACTGCTTC TGCAGCTACT 660

TCCGCCCGGG AATCATCTAC AGGAAGGGAG GCCTAGCATC TGACCTCAAG CATGTGGAGA 720

CCAACTTGTT CAACATCCAG CGACTGCTGC ACATCAAAGG GAGGAAGCAC GTGTCTGCCA 780

CTGAGGTGGA GCTCTCCTGG AACAGCTTTA ATAAGGGTGA CATCTTCCTG CTGGACCTAG 840

GCAAGATGAT GATTCAGTGG AATGGGCCCA AGACCAGCAT TTCTGAGAAG GCTCGGGGGC 900

TGGYCTTGAC CTACAGCCTC CGGGACAGGG AACGTGGTGG TGGTCGTGCA CAGATTGGTG 960

TGGTGGATGA TGAGGCCAAA GCCCCGGACC TCATGCAGAT CATGGAGGCT GTGCTGGGCC 1020

GCAGGGTGGG CAGMCTGCGT GCCGCCACGC CCAGCAAGGA TATCAACCAG CTGCAGAAGG 1080

CCAATGTTCG CCTGTACCAT GTCTATGAGA AGGGCAAAGA CCTGGTGGTC CTGGAGTTGG 1140

CGACCCCCCC ACTGACCCAG GACCTGCTGC AGGAGGAGGA CTTCTACATC CTGGACCAGG 1200

GTGGCTTCAA GATCTATGTG TGGCAGGGAC GCATGTCTAG CCTCCAGGAG AGAAAGGCTG 1260

CCTTCAGCCG GGCTGTGGGC TTCATCCAGG CCAAGGGCTA CCCGACCTAC ACCAACGTGG 1320

AGGTGGTGAA CGACGGCGCC GAGTCGGCCG CGTTCAAGCA GCTCTTCCGG ACTTGGTCTG 1380

AGAAGCGGCG CAGGAACCAG AAGCTCGGCG GGAGGGATAA ATCGATTCAT GTAAAGCTGG 1440

ACGTGGGCAA GCTGCACACC CAGCCTAAGT TAGCGGCCCA GCTCAGGATG GTGGACGACG 1500

GCTCTGGGAA GGTGGAGGTG TGGTGCATCC AGGACTTACA CAGGCAGCCC GTGGACCCCA 1560

AGCGTCATGG ACAGCTGTGT GCAGGCAACT GCTACCTTGT GCTCTACACA TACCAGAGGC 1620

TGGGCCGTGT CCAGTACATC CTGTACCTAT GGCAGGGCCA CCAGGCCACT GCGGATGAGA 1680

TTGAGGCCCT GAACAGCAAC GCTGAGGAAC TAGATGTCAT GTATGGTGGC GTCCTAGTAC 1740

AGGAGCATGT GACCATGGGC AGCGAGCCCC CCCACTTCCT CGCCATCTTC CAGGGCCAGC 1800

TGGTGATCTT CCAGGAGAGA GCTGGGCACC ACGGAAAGGG GCAGTCAGCA TCCACCACAA 1860

GGCTTTTCCA AGTGCAAGGC ACTGACAGCC ACAACACCAG GACCATGGAG GTGCCAGCCC 1920

GTGCCTCATC CCTCAACTCC AGTGACATCT TCTTGCTGGT CACAGCCAGC GTCTGCTACC 1980

TCTGGTTTGG GAAGGGCTGT AATGGTGATC AGCGTGAGAT GGCACGGGTG GTGGTCACTG 2040

TCATTTCCAG GAAGAATGAG GAAACGGTGC TGGAGGGTCA GGAGCCTCCC CACTTCTGGG 2100

AGGCCCTGGG AGGCCGGGSC CCCTACCCCA GCAACAAGAG GCTCCCTGAG GAGGTCCCCA 2160

GCTTCCAGCC ACGACTGTTT GAGTGCTCCA GCCACATGGG CTGCCTGGTC CTCGCAGAAG 2220

TGGGGTTCTT CAGCCAGGAG GACCTGGACA AGTATGACAT CATGTTACTG GACACCTGGC 2280

AGGAGATCTT CCTGTGGCTT GGGGAAGCTG CAAGTGAGTG GAAGGAGGCG GTGGCCTGGG 2340

GCCAGGAGTA CCTGAAGACT CACCCAGCAG GGAGGAGCCC GGNCACACCC ATCGTGCTGG 2400

TCAAGCAGGG CCATGAGCCT CCCACCTTCA TTGGATGGTT CTTCACTTGG GACCCCTACA 2460

AGTGGACTAG CCACCCATCC CACAAGGAAG TGGTGGATGG CAGCCCGGCA GCAGCATCAA 2520

CCATCTCTGA GATAACAGCA GAAGTCAACA ACTTCCGGCT ATCCAGATGG CCGGGCAATG 2580

GCAGGGCAGG TGCCGTGGCC CTGCAGGCCC TCAAGGGCTC CCAGGACAGC TCAGAGAATG 2640

ATCTGGTGCG AAGCCCCAAG TCGGCTGGCA GCAGAACCAG CAGCTCCGTC AGCAGCACCA 2700

GCGCCACGAT CAACGGGGGC CTGCGCCGGG AACAACTGAT GCACCAGGCT GTTGAGGACC 2760

TGCCAGAGGG CGTGGACCCT GCCCGCAGGG AGTTCTATCT CTCAGACTCT GACTTCCAAG 2820

ATATCTTTGG GAAATCCAAG GAGGAATTCT ACAGCATGGC CACGTGGAGG CAGCGGCAGG 2880

AGAAAAAGCA GCTGGGCTTC TTCTGAACCC AAGCCCTCTC GACTGCCCCT ATCCCCTGGA 2940

CCCCAACATA CCTACAATGC TGGGGAGGCC CTGCTTCCAC TCCCCTCAGA GGCTTTTGGT 3000

CATCCTCTGC GTGTCAGTAA AAGCAGGCAG CCCATAAAAA AAAA 3044

(2) INFORMATION FOR SEQ ID NO: 37:

(i) SEQUENCE CHARACTERISTICS:
(A) LENGTH: 541 base pairs
(B) TYPE: nucleic acid
(C) STRANDEDNESS: double
(D) TOPOLOGY: linear

(xi) SEQUENCE DESCRIPTION: SEQ ID NO: 37:

TTCAAGGATT ATAATATGCT GAGTAAACTT TTGGCACTAA GGAAGCCAGC TACAGGCCAC 60

GTAATGAAAA CTATTCAGAA AACAGTTCAG CAAATACTAC TATTTGAATA CAGTTCAAAT 120

CGTATTTATA TAAATACTCT GCCTACATTA TTTAACCCAA ACTGGATTAT TCACCATTCT 180

TTGAAGATGC CTTGTGTTTT CTGTTATCTA CTTCTGCTCG TGCAGTTTAC TTACACCTTC 240

ACCCTTTCAA ATCCTAACTC TTCTTCAAGG CCTGATTCAG ATTTTAACTT TTTAAAGGCT 300

ATCTGAATCA TTCAAGGGAG AAGATACCCT TTCTCTCATA AAAACACTTA GAGCAAACTA 360

CCACTATTAA ATCACTTATT GCATACTGAA AAAAAAAAAA AAAAAAACTC GAAGGGGGGN 420

CCGGTACCCA ATTCGCCCTA TAGTGAGTCG TATTACAATT CACTGGCCGG TCGTTTTACA 480

ACGTCNTGAC TGGGAAAACC CTGGCGTTAC CCAACTTAAT CGCCTTGCAN CACATCCCCC 540

T 541

(2) INFORMATION FOR SEQ ID NO: 38:

(i) SEQUENCE CHARACTERISTICS:
(A) LENGTH: 1752 base pairs
(B) TYPE: nucleic acid
(C) STRANDEDNESS: double

(D) TOPOLOGY: linear

(xi) SEQUENCE DESCRIPTION: SEQ ID NO: 38:

GTCGGCGGCG GCGGCGGCGG TTGAACTGAC TCGGAGCGAG GAGACCCGAG CGAGCAGACG 60

CGGCCCTGGC GCCCGCCCTG CGCACTCACC ATGGCGATGC ATTTCATCTT CTCAGATACA 120

GCGGTGCTTC TGTTTGATTT CTGGAGTGTC CACAGTCCTG CTGGCATGGC CCTTTCGGTG 180

TTGGTGCTCC TGCTTCTGGC TGTACTGTAT GAAGGCATCA AGGTTGGCAA AGCAAGCTGC 240

TCAACCAGGT ACTGGTGAAC CTGCCAACCT CCATCAGCCA GCAGACCATC GCAGAGACAG 300

ACGGGGACTC TGCAGGCTCA GATTCATTCC CTGTTGGCAG AACCCACCAC AGGTGNTATT 360

TGTGTCACTT TGGCCAGTCT CTAATCCATG TCATCCAGGT GGTCATCGGC TACTTCATCA 420

TGCTGGCCGT AATGTCCTAC AACACCTGGA TTTTCCTTGG TGTGGTCTTG GGCTCTGCTG 480

TGGGCTACTA CCTAGCTTAC CCACTTCTCA GCACAGCTTA GCTGGTGAGG AACGTGCAGG 540

CACTGAGGCT GGAGGGACAT GGAGCCCCCT CTTCCAGACA CTATACTTCC AACTGCCCTT 600

TCTTCTGATG GCTATTCCTC CACCTTATTC CCAGCCCCTG GAAACTTTGA GCTGAAGCCA 660

GCACTTGCTC CCTGGAGTTC GGAAGCCATT GCAGCAACCT TCCTTCTCAG CCAGCCTACA 720

TAGGGCCCAG GCATGGTCTT GTGTCTTAAG ACAGCTGCTG TGACCAAAGG GAGAATGGAG 780

ATAACAGGGG TGGCAGGGTT ACTGAGCCCA TGACAATGCT TCTCTGTGAC TCAAACCAGG 840

AATTTCCAAA GATTTCAAGC CAGGGAGAAG GGTTCTTGGT GATGCAGGGC ATGGAACCTG 900

GACACCCTCA GCTCTCCTGC TTTGTGCCTT ATCTACAGGA GCATCGCCCA TTGGACTTCC 960

TGACCTCTTC TGTCTTTGAG GGACAGAGAC CAAGCTAGAT CCTTTTTCTC ACCTTTCTGC 1020

CTTTGGAACA CATGAAGATC ATCTCGTCTA TGGATCATGT TGACAAACTA AGTTTTTTTT 1080

ATTTTTCCCA TTGAACTCCT AGTTGGCAAT TTTGCACATT CATACAAAAA AATTTTTAAT 1140

GAAATGATTT CATTGATTCA TGATGGATGG CAGAAACTGC TGAGACCTAT TTCCCTTTCT 1200

TGGGGAGAGA ATAAGTGACA GCTGATTAAA GGCAGAGACA CAGGACTGCT TTCAGGCTCC 1260

TGGTTTATTC TCTGATAGAC TGAGCTCCTT CCACCAGAAG GCACTGCCTG CAGGAAGAAG 1320

AWGATCTGAT GGCCGTGGGT GTCTGGGAAG CTCTTCGTGG CCTCAATGCC CTCCTTTATC 1380

CTCATCTTTC TTCTATGCAG AACAAAAAGC TGCATCTAAT AATGTTCAAT ACTTAATATT 1440

CTCTATTTAT TACTTACTGC TTACTCGTAA TGATCTAGTG GGGAAACATG ATTCATTCAC 1500

TTAAAATACT GATTAAGCCA TGGCAGGTAC TGACTGAAGA TGCAATCCAA CCAAAGCCAT 1560

TACATTTTTT GAGTTAGATG GGACTSTCTG GATAGTTGAA CCTCTTCACT TTATAAAAAA 1620

GGAAAGAGAG AAAATCACTG CTGTATACTA AATACCTCAC AGATTAGATG AAAAGATGGT 1680

TGTAAGCTTT GGGAATTAAA AACAAACAAA TACATTTTAG TAAATATATA TTTTTAAATA 1740

AAAAAAAGAA AA 1752

(2) INFORMATION FOR SEQ ID NO: 39:

(i) SEQUENCE CHARACTERISTICS:
(A) LENGTH: 1907 base pairs
(B) TYPE: nucleic acid
(C) STRANDEDNESS: double
(D) TOPOLOGY: linear

(xi) SEQUENCE DESCRIPTION: SEQ ID NO: 39:

AGTTCAGGGG CACAGGGGCA CAGGCCCACG ACTGCAGCGG GATGGACCAG TACTGCATCC 60

TGGGCCGCAT CGGGGAGGGC GCCCAMGGCA TCGTCTTCAA GGCCAAGCAC GTGGAGACTG 120

GCGAGATAGT TGCCCTCAAG AAGGTGGCCC TAAGGCGGTT GGAAGACGGC TTCCCTAACC 180

AGGCCCTGCG GGAGATTAAG GCTCTGCAGG ARATGGAGGA CAATCAGTAT GTGGTACAAC 240

TGAAGGCTGT GTTCCCACAC GGTGGAGGCT TTGTGCTGGC CTTTGAGTTC ATGCTGTCGG 300

ATCTGGCCGA GGTGGTGCGC CATGCCCAGA GGCCACTAGC CCAGGCACAG GTCAAGAGCT 360

ACCTGCAGAT GCTGCTCAAG GGTGTCGCCT TCTGCCATGC CAACAACATT GTACATCGGG 420

ACCTGAAACC TGCCAACCTG CTCATCAGCG CCTCAGGCCA GCTCAAGATA GCGGACTTTG 480

GCCTGGCTCG AGTCTTTTCC CCAGACGGCA GCCGCCTCTA CACACACCAG GTGGCCACCA 540

GGAGCTCACT GAGCTGCCGG ACTACAACAA GATCTCCTTT AAGGAGCAGG TGCCCATGCC 600

CCTGGAGGAG GTGCTGCCTG ACGTCTCTCC CCAGGCATTG GATCTGCTGG GTCAATTCCT 660

TCTCTACCCT CCTCACCAGC GCATCGCAGC TTCCAAGGCT CTCCTCCATC AGTACTTCTT 720

CACAGCTCCC CTGCCTGCCC ATCCATCTGA GCTGCCGATT CCTCAGCGTC TAGGGGGACC 780

TGCCCCCAAG GCCCATCCAG GGCCCCCCCA CATCCATGAC TTCCACGTGG ACCGGCCTCT 840

TGAGGAGTCG CTGTTGAACC CAGAGCTGAT TCGGCCCTTC ATCCTGGAGG GGTGAGAAGT 900

TGGCCCTGGT CCCGTCTGCC TGCTCCTCAG GACCACTCAG TCCACCTGTT CCTCTGCCAC 960

CTGCCTGGCT TCACCCTCCA AGGCCTCCCC ATGGCCACAG TGGGCCCACA CCACACCCTG 1020

CCCCTTAGCC CTTGCGARGG TTGGTCTCGA GGCAGAGGTC ATGTTCCCAG CCAAGAGTAT 1080

GAGAACATCC AGTCGAGCAG AGGAGATTCA TGGCCTGTGC TCGGTGAGCC TTACCTTCTG 1140

TGTGCTACTG ACGTACCCAT CAGGACAGTG AGYTCTGCTG CCAGTCAAGG CCTGCATATG 1200

CAGAATGACG ATGCCTGCCT TGGTGCTGCT TCCCCGAGTG CTGCCTCCTG GTCAAGGAGA 1260

AGTGCAGAGA GTAAGGTGTC CTTATGTTGG AAACTCAAGT GGAAGGAAGA TTTGGTTTGG 1320

TTTTATTCTC AGAGCCATTA AACACTAGTT CAGTATGTGA GATATAGATT CTAAAAACCT 1380

CAGGTGGCTC TGCCTTATGT CTGTTCCTCC TTCATTTCTC TCAAGGGAAA TGGCTAAGGT 1440

GGCATTGTCT CATGGCTCTC GTTTTTGGGG TCATGGGGAG GGTAGCACCA GGCATAGCCA 1500

CTTTTGCCCT GAGGGACTCC TGTGTGCTTC ACATCACTGA GCACTCATTT AGAAGTGAGG 1560

GAGACAGAAG TCTAGGCCCA GGGATGGCTC CAGTTGGGGA TCCAGCAGGA GACCCTCTGC 1620

ACATGAGGCT GGTTTACCAA CATCTACTCC CTCAGGATGA GCGTGAGCCA GAAGCAGCTG 1680

TGTATTTAAG GAAACAAGCG TTCCTGGAAT TAATTTATAA ATTTAATAAA TCCCAATATA 1740

ATCCCAGCTA GTGCTTTTTC CTTATTATAA TTTGATAAGG TGATTATAAA AGATACATGG 1800

AAGGAAGTGG AACCAGATGC AGAAGAGGAA ATGATGGAAG GACTTATGGT ATCAGATACC 1860

AATATTTAAA AGTTTGTATA ATAATAAAGA GTATGATTGT GGTTCAA 1907

(2) INFORMATION FOR SEQ ID NO: 40:

(i) SEQUENCE CHARACTERISTICS:
(A) LENGTH: 1114 base pairs
(B) TYPE: nucleic acid
(C) STRANDEDNESS: double
(D) TOPOLOGY: linear

(xi) SEQUENCE DESCRIPTION: SEQ ID NO: 40:

GGGCAGACGA TGCTGAAGAT GCTCTCCTTT AAGCTGCTGC TGCTGGCCGT GGCTCTGGGC 60

TTCTTTGAAG GAGATGCTAA GTTTGGGGAA AGAAACGAAG GGAGCGGACA AGGAGGAGAA 120

GGTGCCTGAA TGGGAACCCC CCGAAGCGCC TGAAAAGGAG AGACAGGAGG ATGATGTCCC 180

AGCTGGAGCT GCTGAGTGGG GGAGAGATGC TGTGCGGTGG CTTCTACCCT CGGCTGTCCT 240

GCTGCCTGCG GAGTGACAGC CCGGGGCTAG GGCGCCTGGA GAATAAGATA TTTTCTGTTA 300

CCAACAACAC AGAATGTGGG AAGTTACTGG AGGAAATCAA ATGTGCACTT TGCTCTCCAC 360

ATTCTCAAAG CCTGTTCCAC TCACCTGAGA GAGAAGTCTT GGAAAGAGAC CTAGTACTTC 420

CTCTGCTCTG CAAAGACTAT TGCAAAGAAT TCTTTTACAC TTGCCGAGGC CATATTCCAG 480

GTTTCCTTCA AACAACTGCG GATGAGTTTT GCTTTTACTA TGCAAGAAAA GATGGTGGGT 540

TGTGCTTTCC AGATTTTCCA AGAAAACAAG TCAGAGGACC AGCATCTAAC TACTTGGACC 600

AGATGGAAGA ATATGACAAA GTGGAAGAGA TCAGCAGAAA GCACAAACAC AACTGCTTCT 660

GTATTCAGGA GGTTGTGAGT GGGCTGCGGC AGCCCGTTGG TGCCCTGCAT AGTGGGGATG 720

GCTCGCAACG TCTCTTCATT CTGGAAAAAG AAGGTTATGT GAAGATACTT ACCCCTGAAG 780

GAGAAATTTT CAAGGAGCCT TATTTGGACA TTCACAAACT TGTTCAAAGT GGAATAAAGG 840

TTGGCTTTTT AAATTTTATT TATTTTTGTG CTGGCTACGT TAATTTTATT TTAGTGTTAC 900

CTTCCTCACT GAAGGTATTT CTTTGTAATA AAAGAAAGAA TCTTGCAGGA GAAAATAAGG 960

GGGCAACATA AGAAACAATA ATTATGGCAC CTGAATTAGG ACAGTGACAT TAAAKGTTGG 1020

CTKTTTAWAT TTTAAAAAAA AAAAAAAAAA AAAAAAAAAA AAAAAAAAAA AAAAAAAAAA 1080

AAAAAAAAAA AAAAAAAAAA AAAAAAAAAA AAAA 1114

(2) INFORMATION FOR SEQ ID NO: 41:

(i) SEQUENCE CHARACTERISTICS:
(A) LENGTH: 1652 base pairs
(B) TYPE: nucleic acid
(C) STRANDEDNESS: double
(D) TOPOLOGY: linear

(xi) SEQUENCE DESCRIPTION: SEQ ID NO: 41:

TTGGCACCTC TAATTGCTCT CGTGTATTCG GTGCCGCGAC TTTCACGATG GCTCGCCCAA 60

CCTTACTACC TTCTGTCGGC CCTGCTCTCT GCTGCCTTCC TACTCGTGAG GAAACTGCCG 120

CCGCTCTGCC ACGGTCTGCC CACCCAACGC GAAGACGGTA ACCCGTGTGA CTTTGACTGG 180

AGAGAAGTGG AGATCCTGAT GTTTCTCAGT GCCATTGTGA TGATGAAGAA CCGCAGATCC 240

ATCACTGTGG AGCAACATAT AGGCAACATT TTCATGTTTA GTAAAGTGGC CAACACAATT 300

CTTTTCTTCC GCTTGGATAT TCGCATGGGC CTACTTTACA TCACACTCTG CATAGTGTTC 360

CTGATGACGT GCAAACCCCC CCTATATATG GGSCCTGAGT ATATCAAGTA CTTCAATGAT 420

AAAACCATTG ATGAGGAACT AGAACGGGAC AAGAGGGTCA CTTGGATTGT GGAGTTCTTT 480

GCCAATTGGT CTAATGACTG CCAATCATTT GCCCCTATCT ATGCTGACCT CTCCCTTAAA 540

TACAACTGTA CAGGGCTAAA TTTTGGGAAG GTGGATGTTG GACGCTATAC TGATGTTAGT 600

ACGCGGTACA AAGTGAGCAC ATCACCCCTC ACCAAGCAAC TCCCTACCCT GATCCTGTTC 660

CAAGGTGGCA AGGAGGCAAT GCGGCGGCCA CAGATTGACA AGAAAGGACG GGCTGTCTCA 720

TGGACCTTCT CTGAGGAGAA TGTGATCCGA GAATTTAACT TAAATGAGCT ATACCAGCGG 780

GCCAAGAAAC TATCAAAGGC TGGAGACAAT ATCCCTGAGG AGCAGCCTGT GGCTTCAACC 840

CCCACCACAG TGTCAGATGG GGAAAACAAG AAGGATAAAT AAGATCCTCA CTTTGGCAGT 900

GCTTCCTCTC CTGTCAATTC CAGGCTCTTT CCATAACCAC AAGCCTGAGG CTGCAGCYTT 960

TTATTTATGT TTTCCCTTTG GCTGTGACTG GGTGGGGCAG CATGCAGCTT CTGATTTTAA 1020

AGAGGCATCT AGGGAATTGT CAGGCACCCT ACAGGAAGGC CTGCCATGCT GTGGCCAACT 1080

GTTTCACTGG AGCAAGAAAG AGATCTCATA GGACGGAGGG GGAAATGGTT TCCCTCCAAG 1140

CTTGGGTYAG TGTGTTAACT GCTTATCAGC TATTCAGACA TCTCCATGGT TTCTCCATGA 1200

AACTCTGTGG TTTCATCATT CCTTCTTAGT TGACCTGCAC AGCTTGGTTA GACCTAGATT 1260

TAACCCTAAG GTAAGATGCT GGGGTATAGA ACGCTAAGAA TTTTCCCCCA AGGACTCTTG 1320

CTTCCTTAAG CCCTTCTGGC TTCGTTTATG GTCTTCATTA AAAGTATAAG CCTAACTTTG 1380

TCGCTAGTCC TAAGGAGAAA CCTTTAACCA CAAAGTTTTT ATCATTGAAG ACAATATTGA 1440

ACAACCCCCT ATTTTGTGGG GATTGAGAAG GGGTGAATAG AGGCTTGAGA CTTTCCTTTG 1500

TGTGGTAGGA CTTGGAGGAG AAATCCCCTG GACTTTCACT AACCCTCTGA CATACTCCCC 1560

ACACCCAGTT GATGGCTTTC CGTAATAAAA AGATTGGGAT TTCCTTTTGA AAAAAAAAAA 1620

AAAAAGGGGG CCGCTCTAGN GGTNCCANGC TT 1652

(2) INFORMATION FOR SEQ ID NO: 42:

(i) SEQUENCE CHARACTERISTICS:
(A) LENGTH: 1473 base pairs
(B) TYPE: nucleic acid
(C) STRANDEDNESS: double
(D) TOPOLOGY: linear

(xi) SEQUENCE DESCRIPTION: SEQ ID NO: 42:

GGCACGAGCC GCGGGGCTGT CACCTCCGCC TCTGCTCCCC GACCCGGCCA TGCGCGGCCT 60

CGGGCTCTGG CTGCTGGGCG CGATGATGCT GCCTGCGATT GCCCCCAGCC GGCCCTGGGC 120

CCTCATGGAG CAGTATGAGG TCGTGTTGCC GTGGCGTCTG CCAGGCCCCC GAGTCCGCCG 180

AGCTCTGCCC TCCCACTTGG GCCTGCACCC AGAGAGGGTG AGCTACGTCC TTGGGGCCAC 240

AGGGCACAAC TTCACCCTCC ACCTGCGGAA GAACAGGGAC CTGCTGGGCT CCGGCTACAC 300

AGAGACCTAT ACGGCTGCCA ATGGCTCCGA GGTGACGGAG CAGCCTCGCG GGCAGGACCA 360

CTGCTTCTAC CAGGGCCACT TAGAGGGTAC CGGACTCAGC CGCCAGCCTC AGCACCTGTG 420

CCGGCCTCAG GGGTTTCTTC CAGGTGGGGT CAGACCTGCA CCTGATCGAG CCCCTGGATG 480

AAGGTGGCGA GGGCGGACGG CACGCCGTGT ACCAGGCTGA GCACCTGCTG CAGACGGCCG 540

GGACCTGCGG GGTCAGCGAC GACAGCCTGG GCAGCCTCCT GGGACCCCGG ACGGCAGCCG 600

TCTTCAGGCC TCGGCCCGGG GACTCTCTGC CATCCCGAGA GACCCGCTAC GTGGAGCTGT 660

ATGTGGTCGT GGACAATGCA GAGTTCCAGA TGCTGGGGAG CGAAGCAGCC GTGCGTCATC 720

GGGTGCTGGA GGTGGTGAAT CACGTGGACA AGCTATATCA GAAACTCAAC TTCCGTGTGG 780

TCCTGGTGGG CCTGGAGATT TGGAATAGTC AGGACAGGTT CCACGTCAGC CCCGACCCCA 840

GTGTCACACT GGAGAACCTC CTGACCTGGC AGGCACGGCA ACGGACACGG CGGCACCTGC 900

ATGACAACGT ACAGCTCATC ACGGGTGTCG ACTTCACCGG GACTACTGTG GGGTTTGCCA 960

GGGTGTCCGC CATGTGCTCC CACAGCTCAG GGGCTGTGAA CCAGGACCAC AGCAAGAACC 1020

CCGTGGGCGT GGCCTGCACC ATGGCCCATG AGATGGGCCA CAACCTGGGC ATGGACCATG 1080

ATGAGAACGT CCAGGGCTGC CGCTGCCAGG AAACGCTTCG AGGCCGGCCG CTGCATCATG 1140

GCAAGGCCAG CATTGGCTCC CAGTTTCCCC AGGATGTTCA GTGACTGCAG CCAGGCCTAC 1200

CTGGAGAGCT TTTTGGAGCG GCCGCAGTCG GTGTGCCTCG CCAACGCCCC TGACCTCAGC 1260

CACCTGGTGG GCGGCCCCGT GTGTGGGAAC CTGTTTGTGG AGCGTGGGGA GCAGTGCGAC 1320

TGCGGCCCCC CCGAGGACTG CCGGAACCGC TGCTGCAACT CTACCACCTG CCAGCTGGCT 1380

GAGGGGGCCC AGTGTGCGCA CGGTACCTGC TGCCAGGAGT GCAAGGTGAA GCCGGCTGGT 1440

GAGCTGTGCC GTCCCAAGAA GGACATGTGT GAC 1473

(2) INFORMATION FOR SEQ ID NO: 43:

(i) SEQUENCE CHARACTERISTICS:
(A) LENGTH: 772 base pairs
(B) TYPE: nucleic acid
(C) STRANDEDNESS: double
(D) TOPOLOGY: linear

(xi) SEQUENCE DESCRIPTION: SEQ ID NO: 43:

TCGGTTTCTC TCTTTGCAGG AGCACCGGCA GCACCAGTGT GTGAGGGGAG CAGGCAGCGG 60

TCCTAGCCAG TTCCTTGATC CTGCCAGACC ACCCAGCCCC TGGCACAGAG CTGCTCCACA 120

GGCACCATGA GGATCATGCT GCTATTCACA GCCATCCTGG CCTTCAGCCT AGCTCAGAGC 180

TTTGGGGCTG TCTGTAAGGA GCCACAGGAG GAGGTGGTTC CTGGCGGGGG CCGCAGCAAG 240

AGGGATCCAG ATCTCTACCA GCTGCTCCAG AGACTCTTCA AAAGCCACTC ATCTCTGGAG 300

GGATTGCTCA AAGCCCTGAG CCAGGYTAGC ACAGATCCTA AGGAATCAAC ATCTCCCGAG 360

AAACGTGACA TGCATGACTT CTTTGTGGGA YTTATGGGCA AGAGGAGCGT CCAGCCAGAC 420

TCTCCTACGG ATGTGAATCA AGAGAACGTC CCCAGCTTTG GCATCCTCAA GTATCCCCCG 480

AGAGCAGAAT AGGTACTCCA CTTCCGGACT CCTGGACTGC ATTAGGAAGA CCTCTTTCCC 540

TGTCCCAATC CCCAGGTGCG CACGCTCCTG TTACCCTTTC TCTTCCCTGT TCTTGTAACA 600

TTCTTGTGCT TTGACTCCTT CTCCATCTTT TCTACCTGAC CCTGGTGTGG AAACTGCATA 660

GTGAATATCC CCAACCCCAA TGGGCATTGA CTGTAGAATA CCCTAGAGTT CCTGTAGTGT 720

CCTACATTAA AAATATAATG TCTCTCTCTA TTCCTCAACA AATAAAGGAT TT 772

(2) INFORMATION FOR SEQ ID NO: 44:

(i) SEQUENCE CHARACTERISTICS:
(A) LENGTH: 403 base pairs
(B) TYPE: nucleic acid
(C) STRANDEDNESS: double
(D) TOPOLOGY: linear

(xi) SEQUENCE DESCRIPTION: SEQ ID NO: 44:

AATTCGGCAC GAGCNTGGAA TGGGAGGCTA CGGAAGAGAT GGAATGGATA ATCAGGGAGG 60

CTATGGWTCA KTTGGAAGAW TGGGAATGGG GAACAATTAC AGTGGAGGAT ATGGTACTCC 120

TGATGGTTTG GGTGGTTATG GCCGTGGTGG TGGAGGCAGT GGAGGTTACT ATGGGCAAGG 180

CGGCATGAGT GGAGGTGGAT GGCGTGGGAT GTACTGAAAG CAAAAACACC AACATACAAG 240

TCTTGACAAC AGCATCTGGT CTACTAGACT TTCTTACAGA TTTAATTTCT TTTGTATTTT 300

AAGAACTTTA TAATGACTGA AGGAATGTGT TTTCAAAATA TTATTTGGTA AAGCAACAGA 360

TTGTGATGGG GAAAAAAAAA AAAAAAAGAA TTCAAAAAGC TTC 403

(2) INFORMATION FOR SEQ ID NO: 45:

(i) SEQUENCE CHARACTERISTICS:
(A) LENGTH: 928 base pairs
(B) TYPE: nucleic acid
(C) STRANDEDNESS: double
(D) TOPOLOGY: linear

(xi) SEQUENCE DESCRIPTION: SEQ ID NO: 45:

CCTCTCGCTA ATTAACCCAA TTGGCCAAAA GGGGGATGTT GCCTGCAANG CCAATTAAAT 60

TTGGGTAAAC CCCCAGGNTT TTCCCCAAGT CCACGACGTT GTAAAAAACG ACGGCCCAAT 120

TGAAATTGTW AAAAACSAAC YCACTAANAG GGCCAAWTGG GTNACSGGGC CCCCCCCCGA 180

RTTTTTTTTT TTTTTTTTTT CTGRTTGWCA ATGAGRATAT TTATTGAGGG TTTATTGAGT 240

GCAGGGAGAA GGGCTKGATG MCTTGGGRTG GGAGGAGAGA CCCCTCCCCT GGGATCCTGC 300

AGCTCYAGKC TCCCGTGGGT GGGGGTKAGR GTTGRGAACC TATGAACATT CTGTAGGGGC 360

CACTGTCTTC TCCACGGTGC TCCCTTCATG CGTGACCTGG CAGCTGTAGC TTCTGTGGGA 420

CTTCCACTGC TCRGGCGTCA GGCTCAGGTA GCTGCTGGCC GCGTACTTGT TGTTGCTYTG 480

TTTGGAGGGT KTGGTGGTCT CCACTCCCGC CTTGACGGGG CTGCYATCTG CNTTCCAGGC 540

CACTGTCACR GCTCCCGGGT AGAAGTCACT KATSAGACAC ACYAGTGTGG CCTTGTTGGC 600

TTGRAGCTCC TCAGAGGAGG GCGGGAACAG AGTGACMGWG GGGKYRGCCT TGGGCTGACC 660

TAGGACGGTG ACCTTGGTCC CAGTTCCGAA GACMCCATGA TTACCACTGC TGTCTGTTGA 720

GTAACAGTAG TAGTCAGCCG CATCCTCCAC CTGGGCCCCA CTGATAGTCA AGGTGGCCAC 780

TGTCCCTGAR CTGGAGCCAR AGAATCTCTS AGGGATCCGG AGGGTCGTTT GTTGTCCTCA 840

TAGATGACCA GGCACAGGGG CCTGGCCTGA CTTCTGKTGG TACCAATAWA CATATTTCTT 900

CGGCAATGCA TCTCCAGGAG CAGGTGAT 928

(2) INFORMATION FOR SEQ ID NO: 46:

(i) SEQUENCE CHARACTERISTICS:
(A) LENGTH: 885 base pairs
(B) TYPE: nucleic acid
(C) STRANDEDNESS: double
(D) TOPOLOGY: linear

(xi) SEQUENCE DESCRIPTION: SEQ ID NO: 46:

GGCACGAGGG AATCTGCACC ATGCCCTGGG TTCTGCTCCT CCTGACCCTC CTCACTCACT 60

CTGCAGTGTC AGTGGTCCAG GCAGGGCTGA CTCAGCCCCC CTCGGTGTCC AAGGACTTGA 120

GACAGACCGC CACACTCACC TGCACCGGGA ACAACAACAA TGTTGGCGAC CAAGGAGCAG 180

CTTGGCTGCA GCAGCACCAG GGCCACCCTC CCAAACTCCT GTCCTACAGG AATAATAACC 240

GGCCCTCAGG GATCTCAGAG AGATTATCTG CATCCAGGTC AGGAGCCACA TCCTCCCTGA 300

CCATTACTGG ACTCCAGCCT GAGGACGAGG CTGACTATTA CTGCGCAGCA TATGACAGCA 360

GCCTCGCAGT TTGGATGTTC GGCGGAGGGA CCAAGCTGAC CGTCCTAGGT CAGCCCAAGG 420

CTGCCCCCTC GGTCACTCTG TTCCCACCCT CCTCTGAGGA GCTTCAAGCC AACAAGGCCA 480

CACTGGTGTG TCTCATAAGT GACTTCTACC CGGGAGCCGT GACAGTGGCC TGGAAGGCAG 540

ATAGCAGCCC CGTCAAGGCG GGAGTGGAGA CCACCACACC CTCCAAACAA AGCAACAACA 600

AGTACGCGGC CAGCAGCTAC CTGAGCCTGA CGCCTGAGCA GTGGAAGTCC CACAAAAGCT 660

ACAGCTGCCA GGTCACGCAT GAAGGGAGCA CCGTGGAGAA GACAGTGGCC CCTACAGAAT 720

GTTCATAGGT TCTCATCCCT CACCCCCCAC CACGGGAGAC TAGAGCTGCA GGATCCCAGG 780

GGAGGGGTCT CTCCTCCCAC CCCAAGGCAT CAAGCCCTTC TCCCTGCACT CAATAAACCC 840

TCAATAAATA TTCTCATTGT CAATCAGAAA AAAAAAAAAA AAAAA 885

(2) INFORMATION FOR SEQ ID NO: 47:

(i) SEQUENCE CHARACTERISTICS:
(A) LENGTH: 2315 base pairs
(B) TYPE: nucleic acid
(C) STRANDEDNESS: double
(D) TOPOLOGY: linear

(xi) SEQUENCE DESCRIPTION: SEQ ID NO: 47:

TTTTTTTTTT TTTGATTTTT CAAAATTAAC TTTTTTATTA ATTTAAAAAT CCAGAAATAC 60

AGTGACTACA TAAATAAGTA CCATAATTAG GTACATGTCC TGTGAGAACA GTGAAAGGGT 120

AATACTGTTA TGTTACTCTT ACTTGTTTAC ATGAGTTAAC TAGAAAATGG CTACAACTGC 180

TAAATGATGC TTATGGTCTT TGTTGTTCCA AGTGTTTATG ATACAAATAA ATACACAAGA 240

AGAACCACAT CCATTCTTCT CTACTAACTA CAGGCAGCTT GGCCTCTTTA CCCTATGTCC 300

TATTCTCTAC ACAACACCAA ACACTGGAGG GTTTCTACTT TGACTTAACA CAGCTCCCCA 360

GCTCCTGCTT CCCACAGCAT TTTGCAAAGG TGTGTCCCAG CACCTGGAGG CAGGAGTATA 420

TCTAGGGAAA CTCTCTGCGT GTTCTCTTAA GGCTAAGCTT TCAGAGAACA CCTGGGTGGG 480

AAGGCTTTGG GATGAATCAT CCAGAAGGAG AAACACCTCT TTGCCTTAGG ATCTAGTTAC 540

TAGTCTCCAC ATTATGGAAT CACTGCCACC TCTGGGACGG AGGGAGCAGC CGCATAACAC 600

CTTCCCCCCT TTACCACACA CACACACACA CACACACACA CACACACAAA GGAGCAAATT 660

ATGCTGTGCA TGGCGTGAAT AATTGACTGC ATTTGAGTTT GGAGTTTTAG GGCACTGTTG 720

ACTTAAGCAA AATAAGCCTG CAGTCCAGCT GCAGCTTGAG TTTTCTTGCT TTACCCTATC 780

CAATACTGTC TGTCTTGCCT AACAGTGGCC CTTTTCAGAT CTCTCCAGGT ACAAAACCTT 840

GACTAAATCT TCAAGCTCTG TTCTGCATAC ACGACTTGAA CACATCTGGC TGATCTGAGC 900

TTCTCCTTCG GTGAAGATCT TCCACTGGCT CAGGGTTGTT CTGGTCACCA GCTTGAAGTG 960

AGGAAGCTCC TCAAACATCC TCTTGGAGAT CTTCTCAATA TGGAAGTGCT GGAGAATGCC 1020

CTTTCGAATA GTCCAAACGT GGACCTCTAC CTGAGGTGGT CTCTCAGTCT CCAGTGCTAT 1080

TTTTCTGGTT GTCTCCCCAT CCTCCATGAA TACAGACTCA TACACAGGCA TCGTTTCTTC 1140

CCCGCAGAAG TAGCCTTTAT TGTCAAAGCT TTGGCCTGGA AGTTCTTCTG GACACACCTT 1200

GCAGCATTTT CCGTCTATTT TTTGAGGATA CTTGCAGGGG TATCGATTGG GGCAGTGGAT 1260

TTTCTTACAC TCTTGCTTGG TGACATTACA AGTACATAGC ACACACTCCA CAATGCCAAA 1320

TGCCCGGAGG TTTGGGTGCC AGGACTCGCC ATGAGAATAG GTCTTTCCAT TGGAAACACA 1380

CACTTGTCCA TGCTTGTGTT TGTTATTGAT GACAATTTGC ACAATGGTTC CTGATGCTTG 1440

CTGGGAATCC ATAAGAGCTC CCCGGTGACT TCTGGCCCCA GGAAAGCGGG ACAGACCTCC 1500

AGCCTGTCGG CTTGGTGGAG GATCATAGTG AGAGCGGTGG TAAGAATGTC TTGCTTCTCT 1560

GTTGGCAGGT TGCCGGAAGA TATCACCATC AGAATGTTCC CATGACAGTT CTCCATCTCC 1620

TCTGCATACC CGGCAGCAGG AATCTGGAAC AGAGACTGGG AAGGCACAGG TTAATTTGGG 1680

GCAAGTCTTG AGACCACAAT ACACGTTTCC CTCCGAACAG CTGCACTGGG TGCATTGATT 1740

GGGTTGCCGA TTCTGAAAGA GCCCTTCAGC TACGAACAGC TCTCCATGTT GGTAAGTTGT 1800

CCCATTGTAC TCGCAAGACT TGCTGGTCAC CTTATTGTTC ACTGGGGGTA AGGAGTCTTC 1860

TGGGCAGCGA GGGCAGCACA GATGAGGAAT ATGCACAGGA GAAAGGCAAT GAACATTTGG 1920

ACATCTGACT CGGCTGCAAA GCACATTCCC ATTCTCTGAG CAGATGCAGT TCACGCAGTA 1980

AACCAACCCA TAAGGTTCCA GGTAAGGATG CCATCTCTCA CCCACTCTGT ACTTCTTGTC 2040

TTGAAACATG CAATATGTCT CTGAATGTTT TACTTGCTCT GTTTKGCCTC CTTCTAGCAA 2100

AAGAAAGCTC GTGCCGAATT CCTGCAGCCC GGGGGGATCC ACTAGTTCTA GAGCGGCCGC 2160

CACCGCGGTG GGAGCTCCAG CTTTTGGTTC CCTTTAGTGA GGGGTTAATT TCGAGCTTGG 2220

CGGTAATCAT GGGTCATAGC TTGTTTCCTG GTGTTGAAAT TGGNTATCCC GCTCACAAAT 2280

TCCACAACAA CAATACGAGC CGGAAGCATA ANGTN 2315

(2) INFORMATION FOR SEQ ID NO: 48:

(i) SEQUENCE CHARACTERISTICS:
(A) LENGTH: 3175 base pairs
(B) TYPE: nucleic acid
(C) STRANDEDNESS: double
(D) TOPOLOGY: linear

(xi) SEQUENCE DESCRIPTION: SEQ ID NO: 48:

TTTTTTTTGT CAATCACTTT AATAGATGTC CATAGGTAGT TCATATGAAT GCTTAAGTTA 60

CAAAATTAGC TGCCATGGTC CAAATGTATG GGACTTTAGG AAAGCTTTTC TTACTCAAAA 120

GATAACTAAG ACTATCAACT TTGATTTCTA AAATGTAATT TAAAGGTTTG TAAAACAAGG 180

CCACTACTAT AATTATATAA TATTAAAGTA ATTAAGTTTT ATGTTAGTTA TTTTAATAAC 240

AACTTCATGA ACTGTTAAAA ATATTACATT TGCATCTCTC AGTTTACATA TTTCTGTATT 300

AACTTGGAGA AAAACCCATG TGAAAAGTTT CCATGCAGTT ACAAAGGCAG CAGCACATGC 360

TGTTTTCACA GCAACTTGTT ATTGCCTCAG AACAGGCCTG CACTAAAGCA TCAACAAAAA 420

ATACCCACCA CCCCACTCCC ACCAGAAAAC CCAACCCTTA CCCATCCCCG GCAAAAATTA 480

CCTGGTACAA GCAATGACCT AAAAATGCTT TCTTGGTAAG AAGCATTTAT AAAATGCAGA 540

GATCTGAACA AGCTAAGTGC TCGTGCAGAT ACATGGGCCT CTCCTCCAAG AGTTGGTTCC 600

GCAAGAGGTG GAAAGAACTC TCAATAGTTT AGGAAAGCTC ATTTTCAAAA GTATACTTAC 660

ACATATTCAT GGCCATTTCT TTGAAAGAAC ATACCCAGCC TCAACTGTGG AAAAGATAAA 720

AGCAGAGGGA GAAGCAACGG CACACAGCCA TAATATAGAG AACAGAGCTT CTCCATGAAC 780

ATCCACCAGG CTGCAGCAAC CAAGAAGGAA AAAACATTTG TGATTTCACA CAGACCAATG 840

ATCTTACCTA GGTGAAGCAT TAATTTTTCA TGCATTTGTT ACTCAAGAAA ATAAACATAC 900

AACCACTTAA AATACAGCAT TCACGTTGTC ACTGGTTCGT GGTATCAGGT AAGGAAAAAA 960

TGATGCTCCT GTCCCTAGAA TTTTCCATGT ACATGTCAGT ATCCTAATGC CTACAGACTT 1020

CCTATTAATT TTGTTATCAG CATCTCCCAC CTAAAAACAT ACACTACATT ATGTTCTGGG 1080

TCCCTGAAAT AGAAAACATC AAGCAATGTT TATTGTGCAA TTCCAATCAT TATTTGCAGA 1140

ATCTTGGTTT AGAGTCAGTC TTTATAGCCA TTTCAACTGC TTGGTTTAAA CAAAAAGCAA 1200

CAATCTGGTT ATCTACCTAT AAATTTCAYG GTATTTCTTT AAACACTGAA GTACTAAAAG 1260

CACTGATGAT TTGTATTATA ATTTTTAAAA TATTTAAAAC CTACACAGAT TTCATAGATC 1320

ATTCCTTTTA TAAAATAATC AAAATAATTT GATTATCTGG AAAAAAAAAT TCTTGAAACA 1380

GAGCCCTTTC CAGGTATCTT CAATCTCTGT AAAACCCCAA ACCCCAAACA GAGTAGATGA 1440

TGAAATAAGG ATTTCTCAGT TGCCCAAGAC TGTCTGAAAT TTAAGGTTGA GAAATGGACT 1500

GGCGTTTTTC ATGTTTCCTG TGAATTCAGA GCTTACAGGT GGCATCAGAA CTCAAATCTC 1560

TGGGATGGCT TTACATGGCT TTCACTTTGA TTTGTTTCAT TTTCATTTGC TTCTTTTCCA 1620

ACTTCTTTTK CTCACGCCTC AATGCAGCCT CCTCCAGCCT GCGCTGTTTC TCAGGATCTT 1680

CCTCATTCAT GATTCGCTCC TTCTCTGCTC TTTTTTTCTC CTCCCGCCGA GACTGTGCTG 1740

CTTCCTGTCT TTGCACATGT GTCAGTTTCA AGAAGTTCTC TTCTACTCGG GCACGGTTCT 1800

TATCTGCTTT TTGTTTGCCT TCTCTGTTGA GTCGGAACTT TTTGGCTTTA TCAATAGAAT 1860

AAATCACCAT GTTCATCAGG GGTAGCAGTG CCTCCATATC CTTTGGGTAA GTGTTACCTG 1920

AGCCAGGCAC ATTAAATGTA AACAACAGTG TCCTCTTAGT GTCAGGTAGC TTTAAAGGCT 1980

GACCTTCCTC TTGCATAATT TTTGGACCAG AGAACTGGTC TGAAAAATGA ACAGATTCAA 2040

TCTTGTCAGC ATAGTGTGTA AGAAAGTGAA CCATCTTTGT ATCCATCATT CCGTCTGTGA 2100

CTTCTCCCAT CTCTGACAGG ATGGCCAAAG AGTCCGGCAG TCCATACTTT GCTCCAGACT 2160

TAGGTTTATC ACTACAAAAC TCACTCAAAT CCTGCATCTC TTTCTGTAGT CGCACCAAGG 2220

CTTTCCGTGT GCCAACAGCA AATACGTAGG TATCCATGTC TTCATCATTC ATGGTTACTT 2280

TTATTTGCAC TTGATCACTC ACTGGCCTCA TCATCCGGGC CAGGACATTC AGTAAGTCTT 2340

GTCTCTTGAG GAACCTCAGC TGGATAAGCA TGCCCTCACA GCACACTCGA CCAGAACACC 2400

ACAGGTTATA GATGTGCTCA TTCTCCTGGT TCAACTTTCC TGTGCTTGTG GCTTCTTTGT 2460

TAGTTCCATC ATCCCCCACT AAAGTAAAGT TGCTCTCCAA AAGCTCCCTA TGAGTGTTAA 2520

ACCAGGCCTG TGCAAGGCGA CTGTTTTTAT TCTTCCCAAT GATGTAATTC ATGATATAAG 2580

CAAGCAGACC AGTCACCATC AAAATTTCTA GATAATAACT CTCCCAGCTG TTCTGGAGGT 2640

GTGCAGGAAC ATCAACAATC GTTATTGGGT CTTTATTTTT GCTAGAAGAA GTATCTGGTT 2700

TGTCTTCATA ACCTTCAAAT TCTTCATCAT CATATGGTTC ACTCTCAGTA TCTCCCTCCT 2760

GGGTATCTGC ATCTTCAAAA TCTCCTTCTT GGTTTTCATC CTGCCCTTCC AACTCCACAG 2820

TGGTCTCATC TTCATCATCT TCAGTGATTA TGACCCGTTG AGGAGATTCA GTAACAGAGT 2880

CTTCCATGAC ATCCTCAAAT TCAGCGAAGT CATTATCATC ATACTCTACT ATGTCCTCCT 2940

CATCCTCAAA ATCATCAAAC TTGGCTTCAG AGACACTCCC AAACACCAGA AGGACAACAC 3000

AGAAAGTGTG GAAGGCTTTC ATTGCACCTT GAGAAAAAAA GCTGTGGCCG AAGCCGAAAC 3060

CCGGCCCAGC GCCCTGCGTC CGACACCCCT GCCCGGCCTG CTCTCGGCCT GGCCGCCGCC 3120

TCCGCGATCG CAGCGGTTTT ACTGCCCCGG ATGCCTCTAG GACGCAGCCA GAACC 3175

(2) INFORMATION FOR SEQ ID NO: 49:

(i) SEQUENCE CHARACTERISTICS:
(A) LENGTH: 783 base pairs
(B) TYPE: nucleic acid
(C) STRANDEDNESS: double
(D) TOPOLOGY: linear

(xi) SEQUENCE DESCRIPTION: SEQ ID NO: 49:

GGCACGCGGA AAGGCTGGCC TCTCTTCAMC ATGGGMTCTT CTGGACTTTT GAGCCTCCTG 60

GTGCTATTCG TCCTCTTAGC GAATGTCCAG GGACCTGGTC TGACTGATTG GTTATTTCCC 120

AGGAGATGTC CCAAAATCAG AGAAGAATGT GAATTCCAAG AAAGGGATGT GTGTACAAAG 180

GACAGACAAT GCCAGGACAA CAAGAAGTGT TGTGTCTTCA GCTGCGGAAA AAAATGTTTA 240

GATCTCAAAC AAGATGTATG CGAAATGCCA AAAGAAACTG GCCCCTGCCT GGCTTATTTT 300

CTTCATTGGT GGTATGACAA GAAAGATAAT ACTTGCTCCA TGTTTGTCTA TGGTGGCTGC 360

CAGGGGAAAC AATAACAACT TCCAATCCAA AGCCAACTGC CTGAACACCT GCAAGAATAA 420

ACGCTTTCCC TGATTGGATA AGGATGCACT GGAAGAACTG CCAGAATGTG GCTCATGCTC 480

TGAGTACTGT TCCTGTACCT GACTGATGCT CCAGACTGGC TTCCAGTTTC ACTCTCAGCA 540

TTCCAAGATC TTAGCCCTTC CCAGAACAGA ACGCTTGCAT CTACCTCCTC TTCCTCCATC 600

TTTGGCTCTT TTGATGCACA ATATCCATCC GTTTTGATTT CATCTTTATG TCCCCTTTAT 660

CTCCAACTTC TAGAACTCCC AGTTTATACC TGTGTCACTC TCAATTTTTT CCAGTAAAGT 720

ACTTGATGTW GAAAAAAAAA AAAAAAAAAA AAAACCGGCA CGAGGGGGGG CCCGGTACCC 780

AAT 783

(2) INFORMATION FOR SEQ ID NO: 50:

(i) SEQUENCE CHARACTERISTICS:
(A) LENGTH: 3030 base pairs
(B) TYPE: nucleic acid
(C) STRANDEDNESS: double
(D) TOPOLOGY: linear

(xi) SEQUENCE DESCRIPTION: SEQ ID NO: 50:

CTCTAAGAAC CTAGTGGATC CCCCCGGCCT GCAGGAATTC GGGCACGGAG GGGAGACTTN 60

CTGTGGCTAA GGGAGGGCGG GAAGGGCCCT CTGTGGGGCT GCCATTTTGG CTGGGACCTA 120

AATGCAGTAA AGGAGCAGCT ACGGGAATAT AGAGAGTGGG GCTTCCAGGC AGAGAAGCCT 180

GCAGTGCAAA GGTCTGCAGA CAACGACCTG GGCGTCTTCA AGGGACACAA GGAATCATAT 240

TGCCAGAACA CATTGTACAG GTAGCCAGGT GTCGGTCTCC AGCCTGAGAA CTCTGGCTGT 300

TGTTCCTTGT GTCGTCCCAT ATTCCTGCCT GGCCTGCGAT GGACATCAGC AAGGGCCTCC 360

CAGGCATGCA GGGAGGCCTC CACATATGGA TCTCTGAGAA CCGGAAGATG GTGCCGGTAC 420

CCGAGGGGGC TTACGGGAAC TTTTTCGAGG AACACTGCTA TGTCATCCTC CACGTCCCCC 480

AGAGCCCGAA GGYCACGCAG GGGGCGTCCA GCGACCTGCA CTACTGGGTC GGGAAGCAGG 540

CGGGTGCGGA AGCGCAGGGC GCTGCGGAGG CCTTCCAGCA GCGCCTACAG GACGAGCTGG 600

GGGGCCAGAC CGTGCTGCAC CGCGAGGCGC AGGGCCACGA GTCCGACTGC TTCTGCAGCT 660

ACTTCCGCCC GGGAATCATC TACAGGAAGG GAGGCCTAGC ATCTGACCTC AAGCATGTGG 720

AGACCAACTT GTTCAACATC CAGCGACTGC TGCACATCAA AGGGAGGAAG CACGTGTCTG 780

CCACTGAGGT GGAGCTCTCC TGGAACAGCT TTAATAAGGG TGACATCTTC CTGCTGGACC 840

TAGGCAAGAT GATGATTCAG TGGAATGGGC CCAAGACCAG CATTTCTGAG AAGGCTCGGG 900

GGCTGGYCTT GACCTACAGC CTCCGGGACA GGGAACGTGG TGGTGGTCGT GCACAGATTG 960

GTGTGGTGGA TGATGAGGCC AAAGCCCCGG ACCTCATGCA GATCATGGAG GCTGTGCTGG 1020

GCCGCAGGGT GGGCAGMCTG CGTGYCGCCA CGCCCAGCAA GGATATCAAC CAGCTGCAGA 1080

AGGCCAATGT TCGCCTGTAC CATGTCTATG AGAAGGGCAA AGACCTGGTG GTCCTGGAGT 1140

TGGCGACCCC CCCACTGACC CAGGACCTGC TGCAGGAGGA GGACTTCTAC ATCCTGGACC 1200

AGGGTGGCTT CAAGATCTAT GTGTGGCAGG GACGCATGTC TAGCCTCCAG GAGAGAAAGG 1260

CTGCCTTCAG CCGGGCTGTG GGCTTCATCC AGGCCAAGGG CTACCCGACC TACACCAACG 1320

TGGAGGTGGT GAACGACGGC GCCGAGTCGG CCGCGTTCAA GCAGCTCTTC CGGACTTGGT 1380

CTGAGAAGCG GCGCAGGAAC CAGAAGMTCG GCGGGAGGGA TAAATCGATT CATGTAAAGC 1440

TGGACGTGGG CAAGCTGCAC ACCCAGCCTA AGTTAGCGGC CCAGCTCAGG ATGGTGGACG 1500

ACGGCTCTGG GAAGGTGGAG GTGTGGTGCA TCCAGGACTT ACACAGGCAG CCCGTGGACC 1560

CCAAGCGTCA TGGACAGCTG TGTGCAGGCA ACTGCTACCT TGTGCTCTAC ACATACCAGA 1620

GGCTGGGCCG TGTCCAGTAC ATCCTGTACC TATGGCAGGG CCACCAGGCC ACTGCGGATG 1680

AGATTGAGGC CCTGAACAGC AACGCTGAGG AACTAGATGT CATGTATGGT GGCGTCCTAG 1740

TACAGGAGCA TGTGACCATG GGCAGCGAGC CCCCCCACTT CCTCGCCATC TTCCAGGGCC 1800

AGCTGGTGAT CTTCCAGGAG AGAGCTGGGC ACCACGGAAA GGGGCAGTCA GCATCCACCA 1860

CAAGGCTTTT CCAAGTGCAA GGCACTGACA GCCACAACAC CAGGACCATG GAGGTGCCAG 1920

CCCGTGCCTC ATCCCTCAAC TCCAGTGACA TCTTCTTGCT GGTCACAGCC AGCGTCTGCT 1980

ACCTCTGGTT TGGGAAAGGG CTGTAATGGT GATCAGCGTG AGATGGCACG GGTGGTGGTC 2040

ACTGTCATTT CCAGGAAGAA TGAGGAAACG GTGCTGGAGG GTCAGGAGCC TCCCCACTTC 2100

TGGGAGGCCC TGGGAGGCCG GGGCCCCCTA CCCCAGCAAC AAGAGGCTCC CTGAGGAGGT 2160

CCCCAGCTTC CAGCCACGAC TGTTTGAGTG CTCCAGCCAC ATGGGCTGCC TGGTCCTCGC 2220

AGAAGTGGGG TTCTTCAGCC AGGAGGACCT GGACAAGTAT GACATCATGT TACTGGACAC 2280

CTGGCAGGAG ATCTTCCTGT GGCTTGGGGA AGCTGCAAGT GAGTGGAAGG AGGCGGTGGC 2340

CTGGGGCCAG GAGTACCTGA AGACTCACCC AGCAGGGAGG AGCCCGGNCA CACCCATCGT 2400

GCTGGTCAAG CAGGGSCATG AGCCTCCCAC CTTCATTGGA TGGTTCTTCA CTTGGGACCC 2460

CTACAAGTGG ACTAGCCACC CATCCCACAA GGAAGTGGTG GATGGCAGCC CGGCAGCAGC 2520

ATCAACCATC TCTGAGATAA CAGCAGAAGT CAACAACTTC CGGCTATCCA GATGGCCGGG 2580

CAATGGCAGG GCAGGTGCCG TGGCCCTGCA GGCCCTCAAG GGCTCCCAGG ACAGCTCAGA 2640

GAATGATYTG GTGCGAAGCC CCAAGTCGGC TGGCAGCAGA ACCAGCAGCT CCGTCAGCAG 2700

CACCAGCGCC ACGATCAACG GGGGCCTGCG CCGGGAACAA CTGATGCACC AGGCTGTTGA 2760

GGACCTGCCA GAGGGCGTGG ACCCTGCCCG CAGGGAGTTC TATCTCTCAG ACTCTGACTT 2820

CCAAGATATC TTTGGGAAAT CCAAGGAGGA ATTCTACAGC ATGGCCACGT GGAGGCAGCG 2880

GCAGGAGAAA AAGCAGCTGG GCTTCTTCTG AACCCAAGCC CTCTCGACTG CCCCTATCCC 2940

CTGGACCCCA ACATACCTAC AATGCTGGGG AGGCCCTGCT TCCACTCCCC TCAGAGGCTT 3000

TTGGTCATCC TCTGCGTGTC AGTAAAAGCA 3030

(2) INFORMATION FOR SEQ ID NO: 51:

(i) SEQUENCE CHARACTERISTICS:
(A) LENGTH: 61 amino acids
(B) TYPE: amino acid
(D) TOPOLOGY: linear

(xi) SEQUENCE DESCRIPTION: SEQ ID NO: 51:

Met Glu His Ala Ala Gly Leu Pro Val Thr Arg His Pro Leu Ala Leu
1               5                   10                  15

Leu Leu Ala Leu Cys Pro Gly Pro Phe Pro Ala Leu Leu Leu Pro Leu
            20                  25                  30

Leu Pro Trp Gly Tyr Pro Leu Ala Pro Pro Gly Leu Cys Lys Leu Pro
        35                  40                  45

Gln Gly Ala Pro Leu Pro Cys Ser Ser Xaa Leu Thr Ser
    50                  55                  60

(2) INFORMATION FOR SEQ ID NO: 52:

(i) SEQUENCE CHARACTERISTICS:
(A) LENGTH: 243 amino acids
(B) TYPE: amino acid
(D) TOPOLOGY: linear
(xi) SEQUENCE DESCRIPTION: SEQ ID NO: 52:

Met Asp Gln Tyr Cys Ile Leu Gly Arg Ile Gly Glu Gly Ala Xaa Gly
1               5                   10                  15

Ile Val Phe Lys Ala Lys His Val Glu Thr Gly Glu Ile Val Ala Leu
            20                  25                  30

Lys Lys Val Ala Leu Arg Arg Leu Glu Asp Gly Phe Pro Asn Gln Ala
        35                  40                  45

Leu Arg Glu Ile Lys Ala Leu Gln Glu Met Glu Asp Asn Gln Tyr Val
    50                  55                  60

Val Gln Leu Lys Ala Val Phe Pro His Gly Gly Gly Phe Val Leu Ala
65                  70                  75                  80

Phe Glu Phe Met Leu Ser Asp Leu Ala Glu Val Val Arg His Ala Gln
                85                  90                  95

Arg Pro Leu Ala Gln Ala Gln Val Lys Ser Tyr Leu Gln Met Leu Leu
            100                 105                 110

Lys Gly Val Ala Phe Cys His Ala Asn Asn Ile Val His Arg Asp Leu
        115                 120                 125

Lys Pro Ala Asn Leu Leu Ile Ser Ala Ser Gly Gln Leu Lys Ile Ala
    130                 135                 140

Asp Phe Gly Leu Ala Arg Val Phe Ser Pro Asp Gly Ser Arg Leu Tyr
145                 150                 155                 160

Thr His Gln Val Ala Thr Arg Ser Ser Leu Ser Cys Arg Thr Thr Thr
                165                 170                 175

Arg Ser Pro Leu Arg Ser Arg Cys Pro Cys Pro Trp Arg Xaa Cys Cys
            180                 185                 190

Leu Thr Ser Leu Pro Arg His Trp Ile Cys Trp Val Asn Ser Phe Ser
        195                 200                 205

Thr Leu Leu Thr Ser Ala Ser Gln Leu Pro Arg Leu Ser Ser Ile Ser
    210                 215                 220

Thr Ser Ser Gln Leu Pro Cys Leu Pro Ile His Leu Ser Cys Arg Phe
225                 230                 235                 240

Leu Ser Val

(2) INFORMATION FOR SEQ ID NO: 53:

        (i) SEQUENCE CHARACTERISTICS:
                (A) LENGTH: 39 amino acids
                (B) TYPE: amino acid
                (D) TOPOLOGY: linear
        (xi) SEQUENCE DESCRIPTION: SEQ ID NO: 53:

Met Glu Ala Lys Phe Gly Leu Leu Cys Phe Leu Val Ser Thr Pro Trp
  1               5                  10                  15

Ala Glu Leu Leu Ser Leu Leu Leu His Leu Thr Gln Val Pro Phe Pro
             20                  25                  30

Gly Ser Gln Gly Pro Gly Phe
        35

(2) INFORMATION FOR SEQ ID NO: 54:

        (i) SEQUENCE CHARACTERISTICS:
                (A) LENGTH: 37 amino acids
                (B) TYPE: amino acid
                (D) TOPOLOGY: linear
        (xi) SEQUENCE DESCRIPTION: SEQ ID NO: 54:

Met Leu Ala Arg Lys Ala Glu Arg Gly Ser Met Gly Thr Ala Arg Asp
  1               5                  10                  15

Ser His Ile Leu Leu Val Cys Ser Val Val His Pro Ala Ser Ala Gln
             20                  25                  30

Pro Val Tyr Thr Val
        35

(2) INFORMATION FOR SEQ ID NO: 55:

        (i) SEQUENCE CHARACTERISTICS:
                (A) LENGTH: 317 amino acids
                (B) TYPE: amino acid
                (D) TOPOLOGY: linear
        (xi) SEQUENCE DESCRIPTION: SEQ ID NO: 55:

(2) INFORMATION FOR SEQ ID NO: 56:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 56:

Met Ser Trp Gly Ile Trp Lys Gly Leu Asp Leu Phe Pro Leu Ile Lys
1               5                   10                  15

Gly Asn Ser Ser Leu Cys Leu Phe Leu Leu Val Val Pro Lys Gly Tyr
            20                  25                  30

Ser Ser Ser Glu Ile Thr Arg Ala Leu
        35                  40

(2) INFORMATION FOR SEQ ID NO: 57:

(i) SEQUENCE CHARACTERISTICS:
(A) LENGTH: 57 amino acids
(B) TYPE: amino acid
(D) TOPOLOGY: linear
(xi) SEQUENCE DESCRIPTION: SEQ ID NO: 57:

Met Ser Leu Pro Cys His Leu Leu Pro Gly Leu Leu Gln Gln Leu Leu
1               5                   10                  15

Thr Ser Leu Pro Ala Phe Gln Phe Ser Ala Pro Leu Gln Val Phe Ser
            20                  25                  30

Leu Asp Gly Leu Ser Leu Pro Ala Pro Lys Leu Leu Thr Ala Ser Leu
        35                  40                  45

Cys Leu Gln Asp Glu Val Arg Ala Val
    50                  55

(2) INFORMATION FOR SEQ ID NO: 58:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 58:

Asn Leu Ile Phe Phe Ser Ile Thr Ile Tyr Ser Tyr Lys Lys Gly Ala
1               5                   10                  15

Ser Glu Lys Gly His Cys Arg Leu Cys Pro Leu Ala Ser Phe Val Ile
            20                  25                  30

(2) INFORMATION FOR SEQ ID NO: 59:

(i) SEQUENCE CHARACTERISTICS:
(A) LENGTH: 296 amino acids

(B) TYPE: amino acid
(D) TOPOLOGY: linear
(xi) SEQUENCE DESCRIPTION: SEQ ID NO: 59:

Met Ala Val Leu Ala Pro Leu Ile Ala Leu Val Tyr Ser Val Pro Arg
1               5                   10                  15

Leu Ser Arg Trp Leu Ala Gln Pro Tyr Tyr Leu Leu Ser Ala Leu Leu
            20                  25                  30

Ser Ala Ala Phe Leu Leu Val Arg Lys Leu Pro Pro Leu Cys His Gly
        35                  40                  45

Leu Pro Thr Gln Arg Glu Asp Gly Asn Pro Cys Asp Phe Asp Trp Arg
    50                  55                  60

Glu Val Glu Ile Leu Met Phe Leu Ser Ala Ile Val Met Met Lys Asn
65                  70                  75                  80

Arg Arg Ser Ile Thr Val Glu Gln His Ile Gly Asn Ile Phe Met Phe
                85                  90                  95

Ser Lys Val Ala Asn Thr Ile Leu Phe Phe Arg Leu Asp Ile Arg Met
            100                 105                 110

Gly Leu Leu Tyr Ile Thr Leu Cys Ile Val Phe Leu Met Thr Cys Lys
        115                 120                 125

Pro Pro Leu Tyr Met Gly Pro Glu Tyr Ile Lys Tyr Phe Asn Asp Lys
    130                 135                 140

Thr Ile Asp Glu Glu Leu Glu Arg Asp Lys Arg Val Thr Trp Ile Val
145                 150                 155                 160

Glu Phe Phe Ala Asn Trp Ser Asn Asp Cys Gln Ser Phe Ala Pro Ile
                165                 170                 175

Tyr Ala Asp Leu Ser Leu Lys Tyr Asn Cys Thr Gly Leu Asn Phe Gly
            180                 185                 190

Lys Val Asp Val Gly Arg Tyr Thr Asp Val Ser Thr Arg Tyr Lys Val
        195                 200                 205

Ser Thr Ser Pro Leu Thr Lys Gln Leu Pro Thr Leu Ile Leu Phe Gln
    210                 215                 220

Gly Gly Lys Glu Ala Met Arg Arg Pro Gln Ile Asp Lys Lys Gly Arg
225                 230                 235                 240

Ala Val Ser Trp Thr Phe Ser Glu Glu Asn Val Ile Arg Glu Phe Asn
                245                 250                 255

Leu Asn Glu Leu Tyr Gln Arg Ala Lys Lys Leu Ser Lys Ala Gly Asp
            260                 265                 270

Asn Ile Pro Glu Glu Gln Pro Val Xaa Ser Thr Pro Thr Thr Val Ser
        275                 280                 285

Asp Gly Glu Asn Lys Lys Asp Lys
    290                 295

(2) INFORMATION FOR SEQ ID NO: 60:

(i) SEQUENCE CHARACTERISTICS:
(A) LENGTH: 100 amino acids
(B) TYPE: amino acid
(D) TOPOLOGY: linear
(xi) SEQUENCE DESCRIPTION: SEQ ID NO: 60:

(2) INFORMATION FOR SEQ ID NO: 61:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 61:

Met Ile Gln Leu Ile Leu Gln Phe Trp Tyr Leu Phe Ser Met Leu Leu
1               5                   10                  15

Lys Pro Val Gln Gln Cys Gln His Cys Ser Gln Ile Thr Pro Ser Gly
            20                  25                  30

Thr Met Pro Thr Ser Glu Thr Val Phe Leu Ile Leu Phe Leu Pro
        35                  40                  45

(2) INFORMATION FOR SEQ ID NO: 62:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 62:

Met Ser Ala Pro Ala Pro Ser Cys Ser Ala Ser Gly Ile
1               5                   10

(2) INFORMATION FOR SEQ ID NO: 63:

(i) SEQUENCE CHARACTERISTICS:
(A) LENGTH: 335 amino acids
(B) TYPE: amino acid
(D) TOPOLOGY: linear
(xi) SEQUENCE DESCRIPTION: SEQ ID NO: 63:

Met Arg Gly Leu Gly Leu Trp Leu Leu Gly Ala Met Met Leu Pro Ala
1               5                   10                  15

Ile Ala Pro Ser Arg Pro Trp Ala Leu Met Glu Gln Tyr Glu Val Val
            20                  25                  30

Leu Pro Xaa Arg Leu Pro Gly Pro Arg Val Arg Arg Ala Leu Pro Ser
        35                  40                  45

His Leu Gly Leu His Pro Glu Arg Val Ser Tyr Val Leu Gly Ala Thr
    50                  55                  60

Gly His Asn Phe Thr Leu His Leu Arg Lys Asn Arg Asp Leu Leu Gly
65                  70                  75                  80

Ser Gly Tyr Thr Glu Thr Tyr Thr Ala Ala Asn Gly Ser Glu Val Thr
                85                  90                  95

Glu Gln Pro Arg Gly Gln Asp His Cys Phe Tyr Gln Gly His Val Glu
            100                 105                 110

Gly Tyr Pro Asp Ser Ala Ala Ser Leu Ser Thr Cys Ala Gly Leu Arg
        115                 120                 125

Gly Phe Phe Gln Val Gly Ser Asp Leu His Leu Ile Glu Pro Leu Asp
    130                 135                 140

Glu Gly Gly Glu Gly Gly Arg His Ala Val Tyr Gln Ala Glu His Leu
145                 150                 155                 160

Leu Gln Thr Ala Gly Thr Cys Gly Val Ser Asp Asp Ser Leu Gly Ser
                165                 170                 175

Leu Leu Gly Pro Arg Thr Ala Ala Val Phe Arg Pro Arg Pro Gly Asp
            180                 185                 190

Ser Leu Pro Ser Arg Glu Thr Arg Tyr Val Glu Leu Tyr Val Val Val
        195                 200                 205

Asp Asn Ala Glu Phe Gln Met Leu Gly Ser Glu Ala Ala Val Arg His
    210                 215                 220

Arg Val Leu Glu Val Val Asn His Val Asp Lys Leu Tyr Gln Lys Leu
225                 230                 235                 240

Asn Phe Arg Val Val Leu Val Gly Leu Glu Ile Trp Asn Ser Gln Asp

245                 250                 255

Arg Phe His Val Ser Pro Asp Pro Ser Val Thr Leu Glu Asn Leu Leu
            260                 265                 270

Thr Trp Gln Ala Arg Gln Arg Thr Arg Arg His Leu His Asp Asn Val
        275                 280                 285

Gln Leu Ile Thr Gly Val Asp Phe Xaa Gly Thr Thr Val Gly Phe Ala
    290                 295                 300

Arg Val Ser Thr Met Cys Ser His Ser Ser Gly Ala Val Asn Gln Asp
305                 310                 315                 320

His Ser Lys Asn Pro Val Gly Val Ala Cys Thr Met Ala His Glu
                325                 330                 335

(2) INFORMATION FOR SEQ ID NO: 64:

        (i) SEQUENCE CHARACTERISTICS:
                (A) LENGTH: 18 amino acids
                (B) TYPE: amino acid
                (D) TOPOLOGY: linear
        (xi) SEQUENCE DESCRIPTION: SEQ ID NO: 64:

Met Pro Gln Lys Lys Arg Phe Leu Met Leu Phe Gly Leu Leu Met Ala
  1               5                  10                  15

Cys Leu

(2) INFORMATION FOR SEQ ID NO: 65:

        (i) SEQUENCE CHARACTERISTICS:
                (A) LENGTH: 125 amino acids
                (B) TYPE: amino acid
                (D) TOPOLOGY: linear
        (xi) SEQUENCE DESCRIPTION: SEQ ID NO: 65:

Met Leu Ser Gln Pro Leu Val Gly Ala Gln Arg Arg Arg Arg Ala Val
  1               5                  10                  15

Gly Leu Ala Val Val Thr Leu Leu Asn Phe Leu Val Cys Phe Gly Pro
             20                  25                  30

Tyr Asn Val Ser His Leu Val Gly Tyr His Gln Arg Lys Ser Pro Trp
         35                  40                  45

Trp Arg Ser Ile Ala Val Xaa Phe Ser Ser Leu Asn Ala Ser Leu Asp
     50                  55                  60

Pro Leu Leu Phe Tyr Phe Ser Ser Ser Val Val Arg Arg Ala Phe Gly
 65                  70                  75                  80

Arg Gly Leu Gln Val Leu Arg Asn Gln Gly Ser Ser Leu Leu Gly Arg
                 85                  90                  95

Arg Gly Lys Asp Thr Ala Glu Gly Thr Asn Glu Asp Arg Gly Val Gly
            100                 105                 110

Gln Gly Glu Gly Met Pro Ser Ser Asp Phe Thr Thr Glu
        115                 120                 125

(2) INFORMATION FOR SEQ ID NO: 66:

        (i) SEQUENCE CHARACTERISTICS:
                (A) LENGTH: 77 amino acids
                (B) TYPE: amino acid
                (D) TOPOLOGY: linear
        (xi) SEQUENCE DESCRIPTION: SEQ ID NO: 66:

Met Arg Leu Val Phe Phe Phe Gly Val Ser Ile Ile Leu Val Leu Gly
  1               5                  10                  15

Ser Thr Phe Val Ala Tyr Leu Pro Asp Tyr Arg Cys Thr Gly Cys Pro
             20                  25                  30

Arg Ala Trp Asp Gly Met Lys Glu Trp Ser Arg Arg Glu Ala Glu Arg
         35                  40                  45

Leu Val Lys Tyr Arg Glu Ala Asn Gly Leu Pro Ile Met Glu Ser Asn
     50                  55                  60

Cys Phe Asp Pro Ser Lys Ile Gln Leu Pro Glu Asp Glu
 65                  70                  75

(2) INFORMATION FOR SEQ ID NO: 67:

        (i) SEQUENCE CHARACTERISTICS:
                (A) LENGTH: 121 amino acids
                (B) TYPE: amino acid
                (D) TOPOLOGY: linear
        (xi) SEQUENCE DESCRIPTION: SEQ ID NO: 67:

(2) INFORMATION FOR SEQ ID NO: 68:

        (i) SEQUENCE CHARACTERISTICS:
                (A) LENGTH: 26 amino acids
                (B) TYPE: amino acid
                (D) TOPOLOGY: linear
        (xi) SEQUENCE DESCRIPTION: SEQ ID NO: 68:

Met Val Val Met Glu Val Leu Met Thr Met Val Ala Ile Ile Ile Thr
  1               5                  10                  15

Ala Met Gly Met Met Ala Leu Met Thr Glu
             20                  25

(2) INFORMATION FOR SEQ ID NO: 69:

        (i) SEQUENCE CHARACTERISTICS:
                (A) LENGTH: 235 amino acids
                (B) TYPE: amino acid
                (D) TOPOLOGY: linear
        (xi) SEQUENCE DESCRIPTION: SEQ ID NO: 69:

Met Pro Trp Val Leu Leu Leu Leu Thr Leu Leu Thr His Ser Ala Val
  1               5                  10                  15

Ser Val Val Gln Ala Gly Leu Thr Gln Pro Pro Ser Val Ser Lys Asp
             20                  25                  30

Leu Arg Gln Thr Ala Thr Leu Thr Cys Thr Gly Asn Asn Asn Asn Val
         35                  40                  45

Gly Asp Gln Gly Ala Ala Trp Leu Gln Gln His Gln Gly His Pro Pro
     50                  55                  60

Lys Leu Leu Ser Tyr Arg Asn Asn Asn Arg Pro Ser Gly Ile Ser Glu
 65                  70                  75                  80

Arg Leu Ser Ala Ser Arg Ser Gly Ala Thr Ser Ser Leu Thr Ile Thr
                 85                  90                  95

Gly Leu Gln Pro Glu Asp Glu Ala Asp Tyr Tyr Cys Ala Ala Tyr Asp
            100                 105                 110

Ser Ser Leu Ala Val Trp Met Phe Gly Gly Gly Thr Lys Leu Thr Val
        115                 120                 125

Leu Gly Gln Pro Lys Ala Ala Pro Ser Val Thr Leu Phe Pro Pro Ser
    130                 135                 140

Ser Glu Glu Leu Gln Ala Asn Lys Ala Thr Leu Val Cys Leu Ile Ser

145                 150                 155                 160

Asp Phe Tyr Pro Gly Ala Val Thr Val Ala Trp Lys Ala Asp Ser Ser
                165                 170                 175

Pro Val Lys Ala Gly Val Glu Thr Thr Thr Pro Ser Lys Gln Ser Asn
            180                 185                 190

Asn Lys Tyr Ala Ala Ser Ser Tyr Leu Ser Leu Thr Pro Glu Gln Trp
        195                 200                 205

Lys Ser His Arg Ser Tyr Ser Cys Gln Val Thr His Glu Gly Ser Thr
    210                 215                 220

Val Glu Lys Thr Val Ala Pro Thr Glu Cys Ser
225                 230                 235

(2) INFORMATION FOR SEQ ID NO: 70:

(i) SEQUENCE CHARACTERISTICS:
(A) LENGTH: 217 amino acids
(B) TYPE: amino acid
(D) TOPOLOGY: linear
(xi) SEQUENCE DESCRIPTION: SEQ ID NO: 70:

Met Asp Ser Gln Gln Ala Ser Gly Thr Ile Val Gln Ile Val Ile Asn
  1               5                  10                  15

Asn Lys His Lys His Gly Gln Val Cys Val Ser Asn Gly Lys Thr Tyr
            20                  25                  30

Ser His Gly Glu Ser Trp His Pro Asn Leu Arg Ala Phe Gly Ile Val
        35                  40                  45

Glu Cys Val Leu Cys Thr Cys Asn Val Thr Lys Gln Glu Cys Lys Lys
    50                  55                  60

Ile His Cys Pro Asn Arg Tyr Pro Cys Lys Tyr Pro Gln Lys Ile Asp
65                  70                  75                  80

Gly Lys Cys Cys Lys Val Cys Pro Glu Glu Leu Pro Gly Gln Ser Phe
                85                  90                  95

Asp Asn Lys Gly Tyr Phe Cys Gly Glu Glu Thr Met Pro Val Tyr Glu
            100                 105                 110

Ser Val Phe Met Glu Asp Gly Glu Thr Thr Arg Lys Ile Ala Leu Glu
        115                 120                 125

Thr Glu Arg Pro Pro Gln Val Glu Val His Val Trp Thr Ile Arg Lys
    130                 135                 140

Gly Ile Leu Gln His Phe His Ile Glu Lys Ile Ser Lys Arg Met Phe
145                 150                 155                 160

Glu Glu Leu Pro His Phe Lys Leu Val Thr Arg Thr Thr Leu Ser Gln
                165                 170                 175

Trp Lys Ile Phe Thr Glu Gly Glu Ala Gln Ile Ser Gln Met Cys Ser
            180                 185                 190

Ser Arg Val Cys Arg Thr Glu Leu Glu Asp Leu Val Lys Val Leu Tyr
        195                 200                 205

Leu Glu Arg Ser Glu Lys Gly His Cys
    210                 215

(2) INFORMATION FOR SEQ ID NO: 71:

       (i) SEQUENCE CHARACTERISTICS:
              (A) LENGTH: 492 amino acids
              (B) TYPE: amino acid
              (D) TOPOLOGY: linear
       (xi) SEQUENCE DESCRIPTION: SEQ ID NO: 71:

Met Lys Ala Phe His Thr Phe Cys Val Val Leu Leu Val Phe Gly Ser
  1               5                  10                  15

Val Ser Glu Ala Lys Phe Asp Asp Phe Glu Asp Glu Glu Asp Ile Val
            20                  25                  30

Glu Tyr Asp Asp Asn Asp Phe Ala Glu Phe Glu Asp Val Met Glu Asp
        35                  40                  45

Ser Val Thr Glu Ser Pro Gln Arg Val Ile Ile Thr Glu Asp Asp Glu
    50                  55                  60

Asp Glu Thr Thr Val Glu Leu Glu Gly Gln Asp Glu Asn Gln Glu Gly
 65                  70                  75                  80

Asp Phe Glu Asp Ala Asp Thr Gln Glu Gly Asp Thr Glu Ser Glu Pro
                 85                  90                  95

Tyr Asp Asp Glu Glu Phe Glu Gly Tyr Glu Asp Lys Pro Asp Thr Ser
            100                 105                 110

Ser Ser Lys Asn Lys Asp Pro Ile Thr Ile Val Asp Val Pro Ala His
        115                 120                 125

Leu Gln Asn Ser Trp Glu Ser Tyr Tyr Leu Glu Ile Leu Met Val Thr
    130                 135                 140

Gly Leu Leu Ala Tyr Ile Met Asn Tyr Ile Ile Gly Lys Asn Lys Asn
145                 150                 155                 160

Ser Arg Leu Ala Gln Ala Trp Phe Asn Thr His Arg Glu Leu Leu Glu
                165                 170                 175

Ser Asn Phe Thr Leu Val Gly Asp Asp Gly Thr Asn Lys Glu Ala Thr
            180                 185                 190

Ser Thr Gly Lys Leu Asn Gln Glu Asn Glu His Ile Tyr Asn Leu Trp
        195                 200                 205

Cys Ser Gly Arg Val Cys Cys Glu Gly Met Leu Ile Gln Leu Arg Phe
    210                 215                 220

Leu Lys Arg Gln Asp Leu Leu Asn Val Leu Ala Arg Met Met Arg Pro
225                 230                 235                 240

Val Ser Asp Gln Val Gln Ile Lys Val Thr Met Asn Asp Glu Asp Met
                245                 250                 255

Asp Thr Tyr Val Phe Ala Val Gly Thr Arg Lys Ala Leu Val Arg Leu
            260                 265                 270

Gln Lys Glu Met Gln Asp Leu Ser Glu Phe Cys Ser Asp Lys Pro Lys
        275                 280                 285

Ser Gly Ala Lys Tyr Gly Leu Pro Asp Ser Leu Ala Ile Leu Ser Glu
    290                 295                 300

Met Gly Glu Val Thr Asp Gly Met Met Asp Thr Lys Met Val His Phe
305                 310                 315                 320

Leu Thr His Tyr Ala Asp Lys Ile Glu Ser Val His Phe Ser Asp Gln
                325                 330                 335

Phe Ser Gly Pro Lys Ile Met Gln Glu Glu Gly Gln Pro Leu Lys Leu
            340                 345                 350

Pro Asp Thr Lys Arg Thr Leu Leu Phe Thr Phe Asn Val Pro Gly Ser
        355                 360                 365

Gly Asn Thr Tyr Pro Lys Asp Met Glu Ala Leu Leu Pro Leu Met Asn
    370                 375                 380

Met Val Ile Tyr Ser Ile Asp Lys Ala Lys Lys Phe Arg Leu Asn Arg
385                 390                 395                 400

Glu Gly Lys Gln Lys Ala Asp Lys Asn Arg Ala Arg Val Glu Glu Asn
                405                 410                 415

Phe Leu Lys Leu Thr His Val Gln Arg Gln Glu Ala Ala Gln Ser Arg
            420                 425                 430

Arg Glu Glu Lys Lys Arg Ala Glu Lys Glu Arg Ile Met Asn Glu Glu
        435                 440                 445

Asp Pro Glu Lys Gln Arg Arg Leu Glu Glu Ala Ala Leu Arg Arg Glu
    450                 455                 460

Gln Lys Lys Leu Glu Lys Lys Gln Met Lys Met Lys Gln Ile Lys Val
465                 470                 475                 480

Lys Ala His Val Lys Pro Ser Gln Arg Phe Glu Phe
                485                 490

(2) INFORMATION FOR SEQ ID NO: 72:

    (i) SEQUENCE CHARACTERISTICS:
        (A) LENGTH: 36 amino acids
        (B) TYPE: amino acid
        (D) TOPOLOGY: linear

(xi) SEQUENCE DESCRIPTION: SEQ ID NO: 72:

Met Leu Phe Leu Cys Leu Leu Pro Ser Leu Phe Pro Pro Gly Leu Pro
1               5                   10                  15

Thr Thr His Tyr Ile Thr Ser Ile Cys Asn Gln Ser Cys Tyr His His
            20                  25                  30

Cys Ala Arg Ala
        35

(2) INFORMATION FOR SEQ ID NO: 73:

(i) SEQUENCE CHARACTERISTICS:
(A) LENGTH: 74 amino acids
(B) TYPE: amino acid
(D) TOPOLOGY: linear
(xi) SEQUENCE DESCRIPTION: SEQ ID NO: 73:

Met Ala Glu Leu Leu Leu Xaa Val Leu Ser Val Gln Ser Ala Val His
1               5                   10                  15

Glu Val Glu Ala Asn Glu Gly Gly Lys Gln Ser His Thr Pro Ala His
            20                  25                  30

Arg Gly Trp Asn Arg Arg Ala Ala Glu Val Arg Lys Ala Arg Leu Pro
        35                  40                  45

Leu Gly Val Thr Val Gly Pro Arg Cys Arg His Ala Val His Pro Ser
    50                  55                  60

Lys Gly Gly Ile Ser Ala Xaa Ala Val Leu
65                  70

(2) INFORMATION FOR SEQ ID NO: 74:

(i) SEQUENCE CHARACTERISTICS:
(A) LENGTH: 133 amino acids
(B) TYPE: amino acid
(D) TOPOLOGY: linear
(xi) SEQUENCE DESCRIPTION: SEQ ID NO: 74:

Met Gly Ser Ser Gly Leu Leu Ser Leu Leu Val Leu Phe Val Leu Leu
1               5                   10                  15

Ala Asn Val Gln Gly Pro Gly Leu Thr Asp Trp Leu Phe Pro Arg Arg
            20                  25                  30

Cys Pro Lys Ile Arg Glu Glu Cys Glu Phe Gln Glu Arg Asp Val Cys
        35                  40                  45

Thr Lys Asp Arg Gln Cys Gln Asp Asn Lys Lys Cys Cys Val Phe Ser
    50                  55                  60

Cys Gly Lys Lys Cys Leu Asp Leu Lys Gln Asp Val Cys Glu Met Pro
65                  70                  75                  80

Lys Glu Thr Gly Pro Cys Leu Ala Tyr Phe Leu His Trp Trp Tyr Asp
                85                  90                  95

Lys Lys Asp Asn Thr Cys Ser Met Phe Val Tyr Gly Gly Cys Gln Gly
            100                 105                 110

Asn Asn Asn Asn Phe Gln Ser Lys Ala Asn Cys Leu Asn Thr Cys Lys
        115                 120                 125

Asn Lys Arg Phe Pro
    130

(2) INFORMATION FOR SEQ ID NO: 75:

        (i) SEQUENCE CHARACTERISTICS:
                (A) LENGTH: 298 amino acids
                (B) TYPE: amino acid
                (D) TOPOLOGY: linear
        (xi) SEQUENCE DESCRIPTION: SEQ ID NO: 75:

Met Ala Arg Arg Ser Arg His Arg Leu Leu Leu Leu Leu Leu Arg Tyr
  1               5                  10                  15

Leu Val Val Ala Leu Gly Tyr His Lys Ala Tyr Gly Phe Ser Ala Pro
            20                  25                  30

Lys Asp Gln Gln Val Val Thr Ala Val Xaa Tyr Gln Glu Ala Ile Leu
        35                  40                  45

Ala Cys Lys Thr Pro Lys Lys Thr Val Xaa Ser Arg Leu Glu Trp Lys
    50                  55                  60

Lys Leu Gly Arg Ser Val Ser Phe Val Tyr Tyr Gln Gln Thr Leu Gln
 65                  70                  75                  80

Gly Asp Phe Lys Asn Arg Ala Glu Met Ile Asp Phe Asn Ile Arg Ile
                85                  90                  95

Lys Asn Val Thr Arg Ser Asp Ala Gly Lys Tyr Arg Cys Glu Val Ser
            100                 105                 110

Ala Pro Ser Glu Gln Gly Gln Asn Leu Glu Glu Asp Thr Val Thr Leu
        115                 120                 125

Glu Val Leu Val Ala Pro Ala Val Pro Ser Cys Glu Val Pro Ser Ser
    130                 135                 140

Ala Leu Ser Gly Thr Val Val Glu Leu Arg Cys Gln Asp Lys Glu Gly
145                 150                 155                 160

Asn Pro Ala Pro Glu Tyr Thr Trp Phe Lys Asp Gly Ile Arg Leu Leu
                165                 170                 175

Glu Asn Pro Arg Leu Gly Ser Gln Ser Thr Asn Ser Ser Tyr Thr Met
            180                 185                 190

Asn Thr Lys Thr Gly Thr Leu Gln Phe Asn Thr Val Ser Lys Leu Asp

195                 200                 205

Thr Gly Glu Tyr Ser Cys Glu Ala Arg Asn Ser Val Gly Tyr Arg Arg
    210                 215                 220

Cys Pro Gly Lys Arg Met Gln Val Asp Asp Leu Asn Ile Ser Gly Ile
225                 230                 235                 240

Ile Ala Ala Val Val Val Val Ala Leu Val Ile Ser Val Cys Gly Leu
                245                 250                 255

Gly Val Cys Tyr Ala Gln Arg Lys Gly Tyr Phe Ser Lys Glu Thr Ser
            260                 265                 270

Phe Gln Lys Ser Asn Ser Ser Ser Lys Ala Thr Thr Met Ser Glu Asn
        275                 280                 285

Asp Phe Lys His Thr Lys Ser Phe Ile Ile
    290                 295

(2) INFORMATION FOR SEQ ID NO: 76:

(i) SEQUENCE CHARACTERISTICS:
(A) LENGTH: 856 amino acids
(B) TYPE: amino acid
(D) TOPOLOGY: linear
(xi) SEQUENCE DESCRIPTION: SEQ ID NO: 76:

Met Asp Ile Ser Lys Gly Leu Pro Gly Met Gln Gly Gly Leu His Ile
1               5                   10                  15

Trp Ile Ser Glu Asn Arg Lys Met Val Pro Val Pro Glu Gly Ala Tyr
            20                  25                  30

Gly Asn Phe Phe Glu Glu His Cys Tyr Val Ile Leu His Val Pro Gln
        35                  40                  45

Ser Pro Lys Xaa Thr Gln Gly Ala Ser Ser Asp Leu His Tyr Trp Val
    50                  55                  60

Gly Lys Gln Ala Gly Ala Glu Ala Gln Gly Ala Ala Glu Ala Phe Gln
65                  70                  75                  80

Gln Arg Leu Gln Asp Glu Leu Gly Gly Gln Thr Val Leu His Arg Glu
                85                  90                  95

Ala Gln Gly His Glu Ser Asp Cys Phe Cys Ser Tyr Phe Arg Pro Gly
            100                 105                 110

Ile Ile Tyr Arg Lys Gly Gly Leu Ala Ser Asp Leu Lys His Val Glu
        115                 120                 125

Thr Asn Leu Phe Asn Ile Gln Arg Leu Leu His Ile Lys Gly Arg Lys
    130                 135                 140

His Val Ser Ala Thr Glu Val Glu Leu Ser Trp Asn Ser Phe Asn Lys
145                 150                 155                 160

Gly Asp Ile Phe Leu Leu Asp Leu Gly Lys Met Met Ile Gln Trp Asn
                165                 170                 175

Gly Pro Lys Thr Ser Ile Ser Glu Lys Ala Arg Gly Leu Xaa Leu Thr
            180                 185                 190

Tyr Ser Leu Arg Asp Arg Glu Arg Gly Gly Gly Arg Ala Gln Ile Gly
        195                 200                 205

Val Val Asp Asp Glu Ala Lys Ala Pro Asp Leu Met Gln Ile Met Glu
    210                 215                 220

Ala Val Leu Gly Arg Arg Val Gly Xaa Leu Arg Ala Ala Thr Pro Ser
225                 230                 235                 240

Lys Asp Ile Asn Gln Leu Gln Lys Ala Asn Val Arg Leu Tyr His Val
                245                 250                 255

Tyr Glu Lys Gly Lys Asp Leu Val Val Leu Glu Leu Ala Thr Pro Pro
            260                 265                 270

Leu Thr Gln Asp Leu Leu Gln Glu Glu Asp Phe Tyr Ile Leu Asp Gln
        275                 280                 285

Gly Gly Phe Lys Ile Tyr Val Trp Gln Gly Arg Met Ser Ser Leu Gln
    290                 295                 300

Glu Arg Lys Ala Ala Phe Ser Arg Ala Val Gly Phe Ile Gln Ala Lys
305                 310                 315                 320

Gly Tyr Pro Thr Tyr Thr Asn Val Glu Val Val Asn Asp Gly Ala Glu
                325                 330                 335

Ser Ala Ala Phe Lys Gln Leu Phe Arg Thr Trp Ser Glu Lys Arg Arg
            340                 345                 350

Arg Asn Gln Lys Leu Gly Gly Arg Asp Lys Ser Ile His Val Lys Leu
        355                 360                 365

Asp Val Gly Lys Leu His Thr Gln Pro Lys Leu Ala Ala Gln Leu Arg
    370                 375                 380

Met Val Asp Asp Gly Ser Gly Lys Val Glu Val Trp Cys Ile Gln Asp
385                 390                 395                 400

Leu His Arg Gln Pro Val Asp Pro Lys Arg His Gly Gln Leu Cys Ala
                405                 410                 415

Gly Asn Cys Tyr Leu Val Leu Tyr Thr Tyr Gln Arg Leu Gly Arg Val
            420                 425                 430

Gln Tyr Ile Leu Tyr Leu Trp Gln Gly His Gln Ala Thr Ala Asp Glu
        435                 440                 445

Ile Glu Ala Leu Asn Ser Asn Ala Glu Glu Leu Asp Val Met Tyr Gly
    450                 455                 460

Gly Val Leu Val Gln Glu His Val Thr Met Gly Ser Glu Pro Pro His
465                 470                 475                 480

Phe Leu Ala Ile Phe Gln Gly Gln Leu Val Ile Phe Gln Glu Arg Ala
                485                 490                 495

Gly His His Gly Lys Gly Gln Ser Ala Ser Thr Thr Arg Leu Phe Gln
            500                 505                 510

Val Gln Gly Thr Asp Ser His Asn Thr Arg Thr Met Glu Val Pro Ala
        515                 520                 525

Arg Ala Ser Ser Leu Asn Ser Ser Asp Ile Phe Leu Leu Val Thr Ala
    530                 535                 540

Ser Val Cys Tyr Leu Trp Phe Gly Lys Gly Cys Asn Gly Asp Gln Arg
545                 550                 555                 560

Glu Met Ala Arg Val Val Val Thr Val Ile Ser Arg Lys Asn Glu Glu
                565                 570                 575

Thr Val Leu Glu Gly Gln Glu Pro Pro His Phe Trp Glu Ala Leu Gly
            580                 585                 590

Gly Arg Xaa Pro Tyr Pro Ser Asn Lys Arg Leu Pro Glu Glu Val Pro
        595                 600                 605

Ser Phe Gln Pro Arg Leu Phe Glu Cys Ser Ser His Met Gly Cys Leu
    610                 615                 620

Val Leu Ala Glu Val Gly Phe Phe Ser Gln Glu Asp Leu Asp Lys Tyr
625                 630                 635                 640

Asp Ile Met Leu Leu Asp Thr Trp Gln Glu Ile Phe Leu Trp Leu Gly
                645                 650                 655

Glu Ala Ala Ser Glu Trp Lys Glu Ala Val Ala Trp Gly Gln Glu Tyr
            660                 665                 670

Leu Lys Thr His Pro Ala Gly Arg Ser Pro Xaa Thr Pro Ile Val Leu
        675                 680                 685

Val Lys Gln Gly His Glu Pro Pro Thr Phe Ile Gly Trp Phe Phe Thr
    690                 695                 700

Trp Asp Pro Tyr Lys Trp Thr Ser His Pro Ser His Lys Glu Val Val
705                 710                 715                 720

Asp Gly Ser Pro Ala Ala Ala Ser Thr Ile Ser Glu Ile Thr Ala Glu
                725                 730                 735

Val Asn Asn Phe Arg Leu Ser Arg Trp Pro Gly Asn Gly Arg Ala Gly
            740                 745                 750

Ala Val Ala Leu Gln Ala Leu Lys Gly Ser Gln Asp Ser Ser Glu Asn
        755                 760                 765

Asp Leu Val Arg Ser Pro Lys Ser Ala Gly Ser Arg Thr Ser Ser Ser
    770                 775                 780

Val Ser Ser Thr Ser Ala Thr Ile Asn Gly Gly Leu Arg Arg Glu Gln
785                 790                 795                 800

Leu Met His Gln Ala Val Glu Asp Leu Pro Glu Gly Val Asp Pro Ala
                805                     810                     815

Arg Arg Glu Phe Tyr Leu Ser Asp Ser Asp Phe Gln Asp Ile Phe Gly
            820                     825                     830

Lys Ser Lys Glu Glu Phe Tyr Ser Met Ala Thr Trp Arg Gln Arg Gln
        835                     840                     845

Glu Lys Lys Gln Leu Gly Phe Phe
    850                     855

(2) INFORMATION FOR SEQ ID NO: 77:

        (i) SEQUENCE CHARACTERISTICS:
                (A) LENGTH: 39 amino acids
                (B) TYPE: amino acid
                (D) TOPOLOGY: linear
        (xi) SEQUENCE DESCRIPTION: SEQ ID NO: 77:

Met Pro Cys Val Phe Cys Tyr Leu Leu Leu Leu Val Gln Phe Thr Tyr
  1               5                  10                  15

Thr Phe Thr Leu Ser Asn Pro Asn Ser Ser Ser Arg Pro Asp Ser Asp
             20                  25                  30

Phe Asn Phe Leu Lys Ala Ile
         35

(2) INFORMATION FOR SEQ ID NO: 78:

        (i) SEQUENCE CHARACTERISTICS:
                (A) LENGTH: 30 amino acids
                (B) TYPE: amino acid
                (D) TOPOLOGY: linear
        (xi) SEQUENCE DESCRIPTION: SEQ ID NO: 78:

Met Ala Leu Ser Val Leu Val Leu Leu Leu Leu Ala Val Leu Tyr Glu
  1               5                  10                  15

Gly Ile Lys Val Gly Lys Ala Ser Cys Ser Thr Arg Tyr Trp
             20                  25                  30

(2) INFORMATION FOR SEQ ID NO: 79:

        (i) SEQUENCE CHARACTERISTICS:
                (A) LENGTH: 45 amino acids
                (B) TYPE: amino acid
                (D) TOPOLOGY: linear
        (xi) SEQUENCE DESCRIPTION: SEQ ID NO: 79:

Met Pro Ala Leu Val Leu Leu Pro Arg Val Leu Pro Pro Gly Gln Gly
  1               5                  10                  15

Glu Val Gln Arg Val Arg Cys Pro Tyr Val Gly Asn Ser Ser Gly Arg
            20                  25                  30

Lys Ile Trp Phe Gly Phe Ile Leu Arg Ala Ile Lys His
        35                  40                  45

(2) INFORMATION FOR SEQ ID NO: 80:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 80:

Met Leu Ser Phe Lys Leu Leu Leu Leu Ala Val Ala Leu Gly Phe Phe
1               5                   10                  15

Glu Gly Asp Ala Lys Phe Gly Glu Arg Asn Glu Gly Ser Gly Gln Gly
            20                  25                  30

Gly Glu Gly Ala
        35

(2) INFORMATION FOR SEQ ID NO: 81:

(i) SEQUENCE CHARACTERISTICS:
(A) LENGTH: 293 amino acids
(B) TYPE: amino acid
(D) TOPOLOGY: linear
(xi) SEQUENCE DESCRIPTION: SEQ ID NO: 81:

Leu Ala Pro Leu Ile Ala Leu Val Tyr Ser Val Pro Arg Leu Ser Arg
1               5                   10                  15

Trp Leu Ala Gln Pro Tyr Tyr Leu Leu Ser Ala Leu Leu Ser Ala Ala
            20                  25                  30

Phe Leu Leu Val Arg Lys Leu Pro Pro Leu Cys His Gly Leu Pro Thr
        35                  40                  45

Gln Arg Glu Asp Gly Asn Pro Cys Asp Phe Asp Trp Arg Glu Val Glu
    50                  55                  60

Ile Leu Met Phe Leu Ser Ala Ile Val Met Met Lys Asn Arg Arg Ser
65                  70                  75                  80

Ile Thr Val Glu Gln His Ile Gly Asn Ile Phe Met Phe Ser Lys Val
                85                  90                  95

Ala Asn Thr Ile Leu Phe Phe Arg Leu Asp Ile Arg Met Gly Leu Leu
            100                 105                 110

Tyr Ile Thr Leu Cys Ile Val Phe Leu Met Thr Cys Lys Pro Pro Leu
        115                 120                 125

Tyr Met Gly Pro Glu Tyr Ile Lys Tyr Phe Asn Asp Lys Thr Ile Asp

130                 135                 140

Glu Glu Leu Glu Arg Asp Lys Arg Val Thr Trp Ile Val Glu Phe Phe
145                 150                 155                 160

Ala Asn Trp Ser Asn Asp Cys Gln Ser Phe Ala Pro Ile Tyr Ala Asp
                165                 170                 175

Leu Ser Leu Lys Tyr Asn Cys Thr Gly Leu Asn Phe Gly Lys Val Asp
            180                 185                 190

Val Gly Arg Tyr Thr Asp Val Ser Thr Arg Tyr Lys Val Ser Thr Ser
        195                 200                 205

Pro Leu Thr Lys Gln Leu Pro Thr Leu Ile Leu Phe Gln Gly Gly Lys
    210                 215                 220

Glu Ala Met Arg Arg Pro Gln Ile Asp Lys Lys Gly Arg Ala Val Ser
225                 230                 235                 240

Trp Thr Phe Ser Glu Glu Asn Val Ile Arg Glu Phe Asn Leu Asn Glu
                245                 250                 255

Leu Tyr Gln Arg Ala Lys Lys Leu Ser Lys Ala Gly Asp Asn Ile Pro
            260                 265                 270

Glu Glu Gln Pro Val Ala Ser Thr Pro Thr Thr Val Ser Asp Gly Glu
        275                 280                 285

Asn Lys Lys Asp Lys
    290

(2) INFORMATION FOR SEQ ID NO: 82:

(i) SEQUENCE CHARACTERISTICS:
(A) LENGTH: 143 amino acids
(B) TYPE: amino acid
(D) TOPOLOGY: linear
(xi) SEQUENCE DESCRIPTION: SEQ ID NO: 82:

Met Arg Gly Leu Gly Leu Trp Leu Leu Gly Ala Met Met Leu Pro Ala
1               5                   10                  15

Ile Ala Pro Ser Arg Pro Trp Ala Leu Met Glu Gln Tyr Glu Val Val
            20                  25                  30

Leu Pro Trp Arg Leu Pro Gly Pro Arg Val Arg Arg Ala Leu Pro Ser
        35                  40                  45

His Leu Gly Leu His Pro Glu Arg Val Ser Tyr Val Leu Gly Ala Thr
    50                  55                  60

Gly His Asn Phe Thr Leu His Leu Arg Lys Asn Arg Asp Leu Leu Gly
65                  70                  75                  80

Ser Gly Tyr Thr Glu Thr Tyr Thr Ala Ala Asn Gly Ser Glu Val Thr
                85                  90                  95

Glu Gln Pro Arg Gly Gln Asp His Cys Phe Tyr Gln Gly His Leu Glu
            100                 105                 110

Gly Thr Gly Leu Ser Arg Gln Pro Gln His Leu Cys Arg Pro Gln Gly
        115                 120                 125

Phe Leu Pro Gly Gly Val Arg Pro Ala Pro Asp Arg Ala Pro Gly
    130                 135                 140

(2) INFORMATION FOR SEQ ID NO: 83:

(i) SEQUENCE CHARACTERISTICS:
(A) LENGTH: 121 amino acids
(B) TYPE: amino acid
(D) TOPOLOGY: linear
(xi) SEQUENCE DESCRIPTION: SEQ ID NO: 83:

Met Arg Ile Met Leu Leu Phe Thr Ala Ile Leu Ala Phe Ser Leu Ala
1               5                   10                  15

Gln Ser Phe Gly Ala Val Cys Lys Glu Pro Gln Glu Glu Val Val Pro
            20                  25                  30

Gly Gly Gly Arg Ser Lys Arg Asp Pro Asp Leu Tyr Gln Leu Leu Gln
        35                  40                  45

Arg Leu Phe Lys Ser His Ser Ser Leu Glu Gly Leu Leu Lys Ala Leu
    50                  55                  60

Ser Gln Xaa Ser Thr Asp Pro Lys Glu Ser Thr Ser Pro Glu Lys Arg
65                  70                  75                  80

Asp Met His Asp Phe Phe Val Gly Xaa Met Gly Lys Arg Ser Val Gln
                85                  90                  95

Pro Asp Ser Pro Thr Asp Val Asn Gln Glu Asn Val Pro Ser Phe Gly
            100                 105                 110

Ile Leu Lys Tyr Pro Pro Arg Ala Glu
1[?]5 120

(2) INFORMATION FOR SEQ ID NO: 84:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 84:

Met Val Leu Leu Met Val Trp Val Val Met Ala Val Val Val Glu Ala
1               5                   10                  15

Val Glu Val Thr Met Gly Lys Ala Ala
            20                  25

(2) INFORMATION FOR SEQ ID NO: 85:

(i) SEQUENCE CHARACTERISTICS:
(A) LENGTH: 4 amino acids
(B) TYPE: amino acid
(D) TOPOLOGY: linear
(xi) SEQUENCE DESCRIPTION: SEQ ID NO: 85:

Ser Leu His Ala
1

(2) INFORMATION FOR SEQ ID NO: 86:

(i) SEQUENCE CHARACTERISTICS:
(A) LENGTH: 235 amino acids
(B) TYPE: amino acid
(D) TOPOLOGY: linear
(xi) SEQUENCE DESCRIPTION: SEQ ID NO: 86:

Met Pro Trp Val Leu Leu Leu Leu Thr Leu Leu Thr His Ser Ala Val
1               5                   10                  15

Ser Val Val Gln Ala Gly Leu Thr Gln Pro Pro Ser Val Ser Lys Asp
            20                  25                  30

Leu Arg Gln Thr Ala Thr Leu Thr Cys Thr Gly Asn Asn Asn Asn Val
        35                  40                  45

Gly Asp Gln Gly Ala Ala Trp Leu Gln Gln His Gln Gly His Pro Pro
    50                  55                  60

Lys Leu Leu Ser Tyr Arg Asn Asn Asn Arg Pro Ser Gly Ile Ser Glu
65                  70                  75                  80

Arg Leu Ser Ala Ser Arg Ser Gly Ala Thr Ser Ser Leu Thr Ile Thr
                85                  90                  95

Gly Leu Gln Pro Glu Asp Glu Ala Asp Tyr Tyr Cys Ala Ala Tyr Asp
            100                 105                 110

Ser Ser Leu Ala Val Trp Met Phe Gly Gly Gly Thr Lys Leu Thr Val
        115                 120                 125

Leu Gly Gln Pro Lys Ala Ala Pro Ser Val Thr Leu Phe Pro Pro Ser
    130                 135                 140

Ser Glu Glu Leu Gln Ala Asn Lys Ala Thr Leu Val Cys Leu Ile Ser
145                 150                 155                 160

Asp Phe Tyr Pro Gly Ala Val Thr Val Ala Trp Lys Ala Asp Ser Ser
                165                 170                 175

Pro Val Lys Ala Gly Val Glu Thr Thr Thr Pro Ser Lys Gln Ser Asn
            180                 185                 190

Asn Lys Tyr Ala Ala Ser Ser Tyr Leu Ser Leu Thr Pro Glu Gln Trp
        195                 200                 205

Lys Ser His Lys Ser Tyr Ser Cys Gln Val Thr His Glu Gly Ser Thr
    210                 215                 220

Val Glu Lys Thr Val Ala Pro Thr Glu Cys Ser
225                 230                 235

(2) INFORMATION FOR SEQ ID NO: 87:

        (i) SEQUENCE CHARACTERISTICS:
                (A) LENGTH: 87 amino acids
                (B) TYPE: amino acid
                (D) TOPOLOGY: linear
        (xi) SEQUENCE DESCRIPTION: SEQ ID NO: 87:

Met Ser Leu Asn Val Leu Leu Ala Leu Phe Xaa Leu Leu Leu Ala Lys
  1               5                  10                  15

Glu Ser Ser Cys Arg Ile Pro Ala Ala Arg Gly Asp Pro Leu Val Leu
             20                  25                  30

Glu Arg Pro Pro Pro Arg Trp Glu Leu Gln Leu Leu Val Pro Phe Ser
         35                  40                  45

Glu Gly Leu Ile Ser Ser Leu Ala Val Ile Met Gly His Ser Leu Phe
     50                  55                  60

Pro Gly Val Glu Ile Gly Tyr Pro Ala His Lys Phe His Asn Asn Asn
 65                  70                  75                  80

Thr Ser Arg Lys His Xaa Val
                 85

(2) INFORMATION FOR SEQ ID NO: 88:

        (i) SEQUENCE CHARACTERISTICS:
                (A) LENGTH: 106 amino acids
                (B) TYPE: amino acid
                (D) TOPOLOGY: linear
        (xi) SEQUENCE DESCRIPTION: SEQ ID NO: 88:

Met Ala Leu His Gly Phe His Phe Asp Leu Phe His Phe His Leu Leu
  1               5                  10                  15

Leu Phe Gln Leu Leu Xaa Leu Thr Pro Gln Cys Ser Leu Leu Gln Pro
             20                  25                  30

Ala Leu Phe Leu Arg Ile Phe Leu Ile His Asp Ser Leu Leu Leu Cys
         35                  40                  45

Ser Phe Phe Leu Leu Pro Pro Arg Leu Cys Cys Phe Leu Ser Leu His
     50                  55                  60

Met Cys Gln Phe Gln Glu Val Leu Phe Tyr Ser Gly Thr Val Leu Ile
 65                  70                  75                  80

Cys Phe Leu Phe Ala Phe Ser Val Glu Ser Glu Leu Phe Gly Phe Ile
                85                  90                  95

Asn Arg Ile Asn His His Val His Gln Gly
            100                 105

(2) INFORMATION FOR SEQ ID NO: 89:

        (i) SEQUENCE CHARACTERISTICS:
                (A) LENGTH: 59 amino acids
                (B) TYPE: amino acid
                (D) TOPOLOGY: linear
        (xi) SEQUENCE DESCRIPTION: SEQ ID NO: 89:

Met Tyr Ala Lys Cys Gln Lys Lys Leu Ala Pro Ala Trp Leu Ile Phe
  1               5                  10                  15

Phe Ile Gly Gly Met Thr Arg Lys Ile Ile Leu Ala Pro Cys Leu Ser
            20                  25                  30

Met Val Ala Ala Arg Gly Asn Asn Asn Asn Phe Gln Ser Lys Ala Asn
        35                  40                  45

Cys Leu Asn Thr Cys Lys Asn Lys Arg Phe Pro
    50                  55

(2) INFORMATION FOR SEQ ID NO: 90:

        (i) SEQUENCE CHARACTERISTICS:
                (A) LENGTH: 32 amino acids
                (B) TYPE: amino acid
                (D) TOPOLOGY: linear
        (xi) SEQUENCE DESCRIPTION: SEQ ID NO: 90:

Met Glu Val Pro Ala Arg Ala Ser Ser Leu Asn Ser Ser Asp Ile Phe
  1               5                  10                  15

Leu Leu Val Thr Ala Ser Val Cys Tyr Leu Trp Phe Gly Lys Gly Leu
            20                  25                  30

(2) INFORMATION FOR SEQ ID NO: 91:

        (i) SEQUENCE CHARACTERISTICS:
                (A) LENGTH: 178 amino acids
                (B) TYPE: amino acid
                (D) TOPOLOGY: linear
        (xi) SEQUENCE DESCRIPTION: SEQ ID NO: 91:

Phe Ser Val Thr Asn Asn Thr Glu Cys Gly Lys Leu Leu Glu Glu Ile
  1               5                  10                  15

Lys Cys Ala Leu Cys Ser Pro His Ser Gln Ser Leu Phe His Ser Pro
            20                  25                  30

Glu Arg Glu Val Leu Glu Arg Asp Leu Val Leu Pro Leu Leu Cys Lys
        35                  40                  45

Asp Tyr Cys Lys Glu Phe Phe Tyr Thr Cys Arg Gly His Ile Pro Gly
    50                  55                  60

Phe Leu Gln Thr Thr Ala Asp Glu Phe Cys Phe Tyr Tyr Ala Arg Lys
65                  70                  75                  80

Asp Gly Gly Leu Cys Phe Pro Asp Phe Pro Arg Lys Gln Val Arg Gly
                85                  90                  95

Pro Ala Ser Asn Tyr Leu Asp Gln Met Glu Glu Tyr Asp Lys Val Glu
            100                 105                 110

Glu Ile Ser Arg Lys His Lys His Asn Cys Phe Cys Ile Gln Glu Val
        115                 120                 125

Val Ser Gly Leu Arg Gln Pro Val Gly Ala Leu His Ser Gly Asp Gly
    130                 135                 140

Ser Gln Arg Leu Phe Ile Leu Glu Lys Glu Gly Tyr Val Lys Ile Leu
145                 150                 155                 160

Thr Pro Glu Gly Glu Ile Phe Lys Glu Pro Tyr Leu Asp Ile His Lys
                165                 170                 175

Leu Val

(2) INFORMATION FOR SEQ ID NO: 92:

     (i) SEQUENCE CHARACTERISTICS:
          (A) LENGTH: 216 amino acids
          (B) TYPE: amino acid
          (D) TOPOLOGY: linear
     (xi) SEQUENCE DESCRIPTION: SEQ ID NO: 92:

Asp Gly Asn Pro Cys Asp Phe Asp Trp Arg Glu Val Glu Ile Leu Met
  1               5                  10                  15

Phe Leu Ser Ala Ile Val Met Met Lys Asn Arg Arg Ser Ile Thr Val
            20                  25                  30

Glu Gln His Ile Gly Asn Ile Phe Met Phe Ser Lys Val Ala Asn Thr
        35                  40                  45

Ile Leu Phe Phe Arg Leu Asp Ile Arg Met Gly Leu Leu Tyr Ile Thr
    50                  55                  60

Leu Cys Ile Val Phe Leu Met Thr Cys Lys Pro Pro Leu Tyr Met Gly
65                  70                  75                  80

Pro Glu Tyr Ile Lys Tyr Phe Asn Asp Lys Thr Ile Asp Glu Glu Leu
                85                  90                  95

Glu Arg Asp Lys Arg Val Thr Trp Ile Val Glu Phe Phe Ala Asn Trp

100                 105                 110

Ser Asn Asp Cys Gln Ser Phe Ala Pro Ile Tyr Ala Asp Leu Ser Leu
        115                 120                 125

Lys Tyr Asn Cys Thr Gly Leu Asn Phe Gly Lys Val Asp Val Gly Arg
    130                 135                 140

Tyr Thr Asp Val Ser Thr Arg Tyr Lys Val Ser Thr Ser Pro Leu Thr
145                 150                 155                 160

Lys Gln Leu Pro Thr Leu Ile Leu Phe Gln Gly Gly Lys Glu Ala Met
                165                 170                 175

Arg Arg Pro Gln Ile Asp Lys Lys Gly Arg Ala Val Ser Trp Thr Phe
            180                 185                 190

Ser Glu Glu Asn Val Ile Arg Glu Phe Asn Leu Asn Glu Leu Tyr Gln
        195                 200                 205

Arg Ala Lys Lys Leu Ser Lys Ala
    210                 215

(2) INFORMATION FOR SEQ ID NO: 93:

(i) SEQUENCE CHARACTERISTICS:
(A) LENGTH: 196 amino acids
(B) TYPE: amino acid
(D) TOPOLOGY: linear
(xi) SEQUENCE DESCRIPTION: SEQ ID NO: 93:

Gln Leu Ile Val Thr Ala Arg Thr Thr Arg Gly Leu Asp Pro Leu Phe
  1               5                  10                  15

Gly Met Cys Glu Lys Phe Leu Gln Glu Val Asp Phe Phe Gln Arg Tyr
             20                  25                  30

Phe Ile Ala Asp Leu Pro His Leu Gln Asp Ser Phe Val Asp Lys Leu
         35                  40                  45

Leu Asp Leu Met Pro Arg Leu Met Thr Ser Lys Pro Ala Glu Val Val
     50                  55                  60

Lys Ile Leu Gln Thr Met Leu Arg Gln Ser Ala Phe Leu His Leu Pro
 65                  70                  75                  80

Leu Pro Glu Gln Ile His Lys Ala Ser Ala Thr Ile Ile Glu Pro Ala
                 85                  90                  95

Gly Glu Phe Arg Gln Pro Phe Ala Val Tyr Leu Trp Val Gly Gly Cys
            100                 105                 110

Pro Gly Met Leu Met Gln Pro Trp Ser Met Cys Arg Ile Leu Arg Thr
        115                 120                 125

Leu Leu Arg Ser Arg Val Leu Tyr Pro Asp Gly Gln Xaa Ser Asp Asp
    130                 135                 140

Ser Pro Gln Ala Cys Arg Leu Pro Glu Ser Trp Pro Arg Ala Ala Pro
145                 150                 155                 160

Ala His His Ser Gly Leu Ser Leu Pro His Arg Leu Asp Arg Gly Met
                165                 170                 175

Pro Gly Gly Ser Glu Ala Ala Ala Gly Leu Gln Leu Gln Cys Ser His
            180                 185                 190

Ser Lys Met Pro
        195

(2) INFORMATION FOR SEQ ID NO: 94:

        (i) SEQUENCE CHARACTERISTICS:
                (A) LENGTH: 255 amino acids
                (B) TYPE: amino acid
                (D) TOPOLOGY: linear
        (xi) SEQUENCE DESCRIPTION: SEQ ID NO: 94:

Ile His Leu Ala Leu Val Glu Leu Leu Lys Asn Leu Thr Lys Tyr Pro
  1               5                  10                  15

Thr Asp Arg Asp Ser Ile Trp Lys Cys Leu Lys Phe Leu Gly Ser Arg
             20                  25                  30

His Pro Thr Leu Val Leu Pro Leu Val Pro Glu Leu Leu Ser Thr His
         35                  40                  45

Pro Phe Phe Asp Thr Ala Glu Pro Asp Met Asp Asp Pro Ala Tyr Ile
     50                  55                  60

Ala Val Leu Val Leu Ile Phe Asn Ala Ala Lys Thr Cys Pro Thr Met
 65                  70                  75                  80

Pro Ala Leu Phe Ser Asp His Th[?] Phe Arg His Tyr Ala Tyr Leu Arg
                 85                  90                  95

Asp Ser Leu Ser His Leu Val Pro Ala Leu Arg Leu Pro Gly Arg Lys
            100                 105                 110

Leu Val Ser Ser Ala Val Ser Pro Ser Ile Ile Pro Gln Glu Asp Pro
        115                 120                 125

Ser Gln Gln Phe Leu Gln Gln Ser Leu Glu Arg Val Tyr Ser Leu Gln
    130                 135                 140

His Leu Asp Pro Gln Gly Ala Gln Glu Leu Leu Glu Phe Thr Ile Arg
145                 150                 155                 160

Asp Leu Gln Arg Leu Gly Glu Leu Gln Ser Glu Leu Ala Gly Val Ala
                165                 170                 175

Asp Phe Ser Ala Thr Tyr Leu Arg Cys Gln Leu Leu Leu Ile Lys Ala
            180                 185                 190

Leu Gln Glu Lys Leu Trp Asn Val Ala Ala Pro Leu Tyr Leu Lys Gln
        195                 200                 205

Ser Asp Leu Ala Ser Ala Ala Ala Lys Gln Ile Met Glu Glu Thr Tyr
    210                 215                 220

Lys Met Glu Phe Met Tyr Ser Gly Val Glu Asn Lys Gln Val Val Ile
225                 230                 235                 240

Ile His His Met Arg Leu Gln Ala Lys Ala Leu Gln Leu Ile Val
                245                 250                 255

(2) INFORMATION FOR SEQ ID NO: 95:

        (i) SEQUENCE CHARACTERISTICS:
                (A) LENGTH: 137 amino acids
                (B) TYPE: amino acid
                (D) TOPOLOGY: linear
        (xi) SEQUENCE DESCRIPTION: SEQ ID NO: 95:

Arg Phe Tyr Ser Asn Ser Cys Cys Leu Cys Cys His Val Arg Thr Gly
  1               5                  10                  15

Thr Ile Leu Leu Gly Val Trp Tyr Leu Ile Ile Asn Ala Val Val Leu
             20                  25                  30

Leu Ile Leu Leu Ser Ala Leu Ala Asp Pro Asp Gln Tyr Asn Phe Ser
         35                  40                  45

Ser Ser Glu Leu Gly Gly Asp Phe Glu Phe Met Asp Asp Ala Asn Met
     50                  55                  60

Cys Ile Ala Ile Ala Ile Ser Leu Leu Met Ile Leu Ile Cys Ala Met
 65                  70                  75                  80

Ala Thr Tyr Gly Ala Tyr Lys Gln Arg Ala Ala Gly Ile Ile Pro Phe
                 85                  90                  95

Phe Cys Tyr Gln Ile Phe Asp Phe Ala Leu Asn Met Leu Val Ala Ile
            100                 105                 110

Thr Val Leu Ile Tyr Pro Asn Ser Ile Gln Glu Tyr Ile Arg Gln Leu
        115                 120                 125

Pro Pro Asn Phe Pro Tyr Arg Asp Asp
    130                 135

(2) INFORMATION FOR SEQ ID NO: 96:

        (i) SEQUENCE CHARACTERISTICS:
                (A) LENGTH: 87 amino acids
                (B) TYPE: amino acid
                (D) TOPOLOGY: linear
        (xi) SEQUENCE DESCRIPTION: SEQ ID NO: 96:

Phe Pro Thr Glu Met Met Ser Cys Ala Val Asn Pro Thr Cys Leu Val
  1               5                  10                  15

Leu Ile Ile Leu Leu Phe Ile Ser Ile Ile Leu Thr Phe Lys Gly Tyr
            20                  25                  30

Leu Ile Ser Cys Val Trp Asn Cys Tyr Arg Tyr Ile Asn Gly Arg Asn
        35                  40                  45

Ser Ser Asp Val Leu Val Tyr Val Thr Ser Asn Asp Thr Thr Val Leu
    50                  55                  60

Leu Pro Pro Tyr Asp Asp Ala Thr Val Asn Gly Ala Ala Lys Glu Pro
65                  70                  75                  80

Pro Pro Pro Tyr Val Ser Ala
                85

(2) INFORMATION FOR SEQ ID NO: 97:

(i) SEQUENCE CHARACTERISTICS:
(A) LENGTH: 97 amino acids
(B) TYPE: amino acid
(D) TOPOLOGY: linear
(xi) SEQUENCE DESCRIPTION: SEQ ID NO: 97:

Ile Ala Pro Ser Arg Pro Trp Ala Leu Met Glu Gln Tyr Glu Val Val
1               5                   10                  15

Leu Pro Trp Arg Leu Pro Gly Pro Arg Val Arg Arg Ala Leu Pro Ser
            20                  25                  30

His Leu Gly Leu His Pro Glu Arg Val Ser Tyr Val Leu Gly Ala Thr
        35                  40                  45

Gly His Asn Phe Thr Leu His Leu Arg Lys Asn Arg Asp Leu Leu Gly
    50                  55                  60

Ser Gly Tyr Thr Glu Thr Tyr Thr Ala Ala Asn Gly Ser Glu Val Thr
65                  70                  75                  80

Glu Gln Pro Arg Gly Gln Asp His Cys Phe Tyr Gln Gly His Leu Glu
                85                  90                  95

Gly

(2) INFORMATION FOR SEQ ID NO: 98:

(i) SEQUENCE CHARACTERISTICS:
(A) LENGTH: 240 amino acids
(B) TYPE: amino acid
(D) TOPOLOGY: linear
(xi) SEQUENCE DESCRIPTION: SEQ ID NO: 98:

Pro Asp Ser Ala Ala Ser Leu Ser Thr Cys Ala Gly Leu Arg Gly Phe
1               5                   10                  15

Phe Gln Val Gly Ser Asp Leu His Leu Ile Glu Pro Leu Asp Glu Gly
            20                  25                  30

Gly Glu Gly Gly Arg His Ala Val Tyr Gln Ala Glu His Leu Leu Gln
        35                  40                  45

Thr Ala Gly Thr Cys Gly Val Ser Asp Asp Ser Leu Gly Ser Leu Leu
    50                  55                  60

Gly Pro Arg Thr Ala Ala Val Phe Arg Pro Arg Pro Gly Asp Ser Leu
65                  70                  75                  80

Pro Ser Arg Glu Thr Arg Tyr Val Glu Leu Tyr Val Val Val Asp Asn
                85                  90                  95

Ala Glu Phe Gln Met Leu Gly Ser Glu Ala Ala Val Arg His Arg Val
            100                 105                 110

Leu Glu Val Val Asn His Val Asp Lys Leu Tyr Gln Lys Leu Asn Phe
        115                 120                 125

Arg Val Val Leu Val Gly Leu Glu Ile Trp Asn Ser Gln Asp Arg Phe
    130                 135                 140

His Val Ser Pro Asp Pro Ser Val Thr Leu Glu Asn Leu Leu Thr Trp
145                 150                 155                 160

Gln Ala Arg Gln Arg Thr Arg Arg His Leu His Asp Asn Val Gln Leu
                165                 170                 175

Ile Thr Gly Val Asp Phe Thr Gly Thr Thr Val Gly Phe Ala Arg Val
            180                 185                 190

Ser Ala Met Cys Ser His Ser Ser Gly Ala Val Asn Gln Asp His Ser
        195                 200                 205

Lys Asn Pro Val Gly Val Ala Cys Thr Met Ala His Glu Met Gly His
    210                 215                 220

Asn Leu Gly Met Asp His Asp Glu Asn Val Gln Gly Cys Arg Cys Gln
225                 230                 235                 240

(2) INFORMATION FOR SEQ ID NO: 99:

(i) SEQUENCE CHARACTERISTICS:
(A) LENGTH: 118 amino acids
(B) TYPE: amino acid
(D) TOPOLOGY: linear
(xi) SEQUENCE DESCRIPTION: SEQ ID NO: 99:

Phe Glu Ala Gly Arg Cys Ile Met Ala Arg Pro Ala Leu Ala Pro Ser
1               5                   10                  15

Phe Pro Arg Met Phe Ser Asp Cys Ser Gln Ala Tyr Leu Glu Ser Phe
            20                  25                  30

Leu Glu Arg Pro Gln Ser Val Cys Leu Ala Asn Ala Pro Asp Leu Ser
        35                  40                  45

His Leu Val Gly Gly Pro Val Cys Gly Asn Leu Phe Val Glu Arg Gly
    50                  55                  60

Glu Gln Cys Asp Cys Gly Pro Pro Glu Asp Cys Arg Asn Arg Cys Cys
65                  70                  75                  80

Asn Ser Thr Thr Cys Gln Leu Ala Glu Gly Ala Gln Cys Ala His Gly
                85                  90                  95

Thr Cys Cys Gln Glu Cys Lys Val Lys Pro Ala Gly Glu Leu Cys Arg
            100                 105                 110

Pro Lys Lys Asp Met Cys
        115

(2) INFORMATION FOR SEQ ID NO: 100:

        (i) SEQUENCE CHARACTERISTICS:
                (A) LENGTH: 330 amino acids
                (B) TYPE: amino acid
                (D) TOPOLOGY: linear
        (xi) SEQUENCE DESCRIPTION: SEQ ID NO: 100:

Met Leu Pro Asp Trp Lys Xaa Ser Leu Ile Leu Met Ala Tyr Ile Ile
  1               5                  10                  15

Ile Phe Leu Thr Gly Leu Pro Ala Asn Leu Leu Ala Leu Arg Ala Phe
            20                  25                  30

Val Gly Arg Ile Arg Gln Pro Gln Pro Ala Pro Val His Ile Leu Leu
        35                  40                  45

Leu Ser Leu Thr Leu Ala Asp Leu Leu Leu Leu Leu Leu Leu Pro Phe
    50                  55                  60

Lys Ile Ile Glu Ala Ala Ser Asn Phe Arg Trp Tyr Leu Pro Lys Val
65                  70                  75                  80

Val Cys Ala Leu Thr Ser Phe Gly Phe Tyr Ser Ser Ile Tyr Cys Ser
                85                  90                  95

Thr Trp Leu Leu Ala Gly Ile Ser Ile Glu Arg Tyr Leu Gly Val Ala
            100                 105                 110

Phe Pro Val Gln Tyr Lys Leu Ser Arg Arg Pro Leu Tyr Gly Val Ile
        115                 120                 125

Ala Ala Leu Val Ala Trp Val Met Ser Phe Gly His Cys Thr Ile Val
    130                 135                 140

Ile Ile Xaa Gln Tyr Leu Asn Thr Thr Glu Gln Val Arg Ser Gly Asn
145                 150                 155                 160

Glu Ile Thr Cys Tyr Glu Asn Phe Thr Asp Asn Gln Leu Asp Val Val
                165                 170                 175

Leu Pro Val Arg Xaa Glu Leu Cys Leu Val Leu Phe Phe Xaa Pro Met
            180                 185                 190

Ala Val Thr Ile Phe Cys Tyr Trp Arg Phe Val Trp Ile Met Leu Ser
        195                 200                 205

Gln Pro Leu Val Gly Ala Gln Arg Arg Arg Arg Ala Val Gly Leu Ala
    210                 215                 220

Val Val Thr Leu Leu Asn Phe Leu Val Cys Phe Gly Pro Tyr Asn Val
225                 230                 235                 240

Ser His Leu Val Gly Tyr His Gln Arg Lys Ser Pro Trp Trp Arg Ser
                245                 250                 255

Ile Ala Val Xaa Phe Ser Ser Leu Asn Ala Ser Leu Asp Pro Leu Leu
            260                 265                 270

Phe Tyr Phe Ser Ser Ser Val Val Arg Arg Ala Phe Gly Arg Gly Leu
        275                 280                 285

Gln Val Leu Arg Asn Gln Gly Ser Ser Leu Leu Gly Arg Arg Gly Lys
    290                 295                 300

Asp Thr Ala Glu Gly Thr Asn Glu Asp Arg Gly Val Gly Gln Gly Glu
305                 310                 315                 320

Gly Met Pro Ser Ser Asp Phe Thr Thr Glu
                325                 330

(2) INFORMATION FOR SEQ ID NO: 101:

        (i) SEQUENCE CHARACTERISTICS:
                (A) LENGTH: 17 amino acids
                (B) TYPE: amino acid
                (D) TOPOLOGY: linear
        (xi) SEQUENCE DESCRIPTION: SEQ ID NO: 101:

Cys Ser Thr Trp Leu Leu Ala Gly Ile Ser Ile Glu Arg Tyr Leu Gly
  1               5                  10                  15

Val

(2) INFORMATION FOR SEQ ID NO: 102:

        (i) SEQUENCE CHARACTERISTICS:
                (A) LENGTH: 94 amino acids
                (B) TYPE: amino acid
                (D) TOPOLOGY: linear
        (xi) SEQUENCE DESCRIPTION: SEQ ID NO: 102:

Cys Thr Ile Val Ile Ile Xaa Gln Tyr Leu Asn Thr Thr Glu Gln Val
  1               5                  10                  15

Arg Ser Gly Asn Glu Ile Thr Cys Tyr Glu Asn Phe Thr Asp Asn Gln
             20                  25                  30

Leu Asp Val Val Leu Pro Val Arg Xaa Glu Leu Cys Leu Val Leu Phe
        35                  40                  45

Phe Xaa Pro Met Ala Val Thr Ile Phe Cys Tyr Trp Arg Phe Val Trp
    50                  55                  60

Ile Met Leu Ser Gln Pro Leu Val Gly Ala Gln Arg Arg Arg Arg Ala
65                  70                  75                  80

Val Gly Leu Ala Val Val Thr Leu Leu Asn Phe Leu Val Cys
                85                  90

(2) INFORMATION FOR SEQ ID NO: 103:

(i) SEQUENCE CHARACTERISTICS:
(A) LENGTH: 143 amino acids
(B) TYPE: amino acid
(D) TOPOLOGY: linear
(xi) SEQUENCE DESCRIPTION: SEQ ID NO: 103:

Gly Leu Pro Ala Ala Arg Val Arg Trp Glu Ser Ser Phe Ser Arg Thr
1               5                   10                  15

Val Val Ala Pro Ser Ala Val Ala Xaa Lys Arg Pro Pro Glu Pro Thr
            20                  25                  30

Thr Pro Trp Gln Glu Asp Pro Glu Pro Glu Asp Glu Asn Leu Tyr Glu
        35                  40                  45

Lys Asn Pro Asp Ser His Gly Tyr Asp Lys Asp Pro Val Leu Asp Val
    50                  55                  60

Trp Asn Met Arg Leu Val Phe Phe Phe Gly Val Ser Ile Ile Leu Val
65                  70                  75                  80

Leu Gly Ser Thr Phe Val Ala Tyr Leu Pro Asp Tyr Arg Cys Thr Gly
                85                  90                  95

Cys Pro Arg Ala Trp Asp Gly Met Lys Glu Trp Ser Arg Arg Glu Ala
            100                 105                 110

Glu Arg Leu Val Lys Tyr Arg Glu Ala Asn Gly Leu Pro Ile Met Glu
        115                 120                 125

Ser Asn Cys Phe Asp Pro Ser Lys Ile Gln Leu Pro Glu Asp Glu
    130                 135                 140

(2) INFORMATION FOR SEQ ID NO: 104:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 104:

Pro Glu Lys Arg Asp Met His Asp Phe Phe Val Gly Leu Met Gly Lys

1                   5                   10                  15

Arg Ser Val Gln Pro Asp Ser Pro Thr Asp Val Asn Gln Glu Asn Val
            20                  25                  30

Pro Ser Phe Gly
        35

(2) INFORMATION FOR SEQ ID NO: 105:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 105:

Lys Arg Asp Met His Asp Phe Phe Val Gly Leu Met Gly Lys Arg
1                   5                   10                  15

(2) INFORMATION FOR SEQ ID NO: 106:

(i) SEQUENCE CHARACTERISTICS:
(A) LENGTH: 10 amino acids
(B) TYPE: amino acid
(D) TOPOLOGY: linear
(xi) SEQUENCE DESCRIPTION: SEQ ID NO: 106:

Asp Met His Asp Phe Phe Val Gly Leu Met
1                   5                   10

(2) INFORMATION FOR SEQ ID NO: 107:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 107:

Glu Trp Glu Ala Thr Glu Glu Met Glu Trp Ile Ile Arg Glu Ala Met
1                   5                   10                  15

(2) INFORMATION FOR SEQ ID NO: 108:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 108:

Trp Glu Trp Gly Thr Ile Thr Val Glu Asp Met Val Leu Leu Met Val
1                   5                   10                  15

Trp Val Val Met Ala Val Val Val Glu Ala Val Glu Val Thr Met Gly
            20                  25                  30

Lys Ala Ala
        35

(2) INFORMATION FOR SEQ ID NO: 109:

        (i) SEQUENCE CHARACTERISTICS:
                (A) LENGTH: 18 amino acids
                (B) TYPE: amino acid
                (D) TOPOLOGY: linear
        (xi) SEQUENCE DESCRIPTION: SEQ ID NO: 109:

Gly Met Gly Gly Tyr Gly Arg Asp Gly Met Asp Asn Gln Gly Gly Tyr
  1               5                  10                  15

Gly Ser

(2) INFORMATION FOR SEQ ID NO: 110:

        (i) SEQUENCE CHARACTERISTICS:
                (A) LENGTH: 43 amino acids
                (B) TYPE: amino acid
                (D) TOPOLOGY: linear
        (xi) SEQUENCE DESCRIPTION: SEQ ID NO: 110:

Gly Met Gly Asn Asn Tyr Ser Gly Gly Tyr Gly Thr Pro Asp Gly Leu
  1               5                  10                  15

Gly Gly Tyr Gly Arg Gly Gly Gly Gly Ser Gly Gly Tyr Tyr Gly Gln
            20                  25                  30

Gly Gly Met Ser Gly Gly Gly Trp Arg Gly Met
        35                  40

(2) INFORMATION FOR SEQ ID NO: 111:

        (i) SEQUENCE CHARACTERISTICS:
                (A) LENGTH: 26 amino acids
                (B) TYPE: amino acid
                (D) TOPOLOGY: linear
        (xi) SEQUENCE DESCRIPTION: SEQ ID NO: 111:

Phe Thr His Ser Phe Ile Leu Glu His Ala Phe Ser Leu Leu Ile Thr
  1               5                  10                  15

Leu Pro Val Ser Ser Trp Ala Ala Asn Asn
            20                  25

(2) INFORMATION FOR SEQ ID NO: 112:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 112:

Cys Glu Met Pro Lys Glu Thr Gly Pro Cys Leu Ala Tyr Phe Leu His
1               5                   10                  15

Trp Trp Tyr Asp Lys Lys Asp Asn Thr Cys Ser Met Phe Val Tyr Gly
            20                  25                  30

Gly Cys Gln Gly Asn Asn Asn Asn Phe Gln Ser Lys Ala Asn Cys Leu
        35                  40                  45

Asn Thr Cys
    50

(2) INFORMATION FOR SEQ ID NO: 113:

(i) SEQUENCE CHARACTERISTICS:
(A) LENGTH: 384 amino acids
(B) TYPE: amino acid
(D) TOPOLOGY: linear
(xi) SEQUENCE DESCRIPTION: SEQ ID NO: 113:

Met Met Ile Gln Trp Asn Gly Pro Lys Thr Ser Ile Ser Glu Lys Ala
1               5                   10                  15

Arg Gly Leu Xaa Leu Thr Tyr Ser Leu Arg Asp Arg Glu Arg Gly Gly
            20                  25                  30

Gly Arg Ala Gln Ile Gly Val Val Asp Asp Glu Ala Lys Ala Pro Asp
        35                  40                  45

Leu Met Gln Ile Met Glu Ala Val Leu Gly Arg Arg Val Gly Xaa Leu
    50                  55                  60

Arg Xaa Ala Thr Pro Ser Lys Asp Ile Asn Gln Leu Gln Lys Ala Asn
65                  70                  75                  80

Val Arg Leu Tyr His Val Tyr Glu Lys Gly Lys Asp Leu Val Val Leu
                85                  90                  95

Glu Leu Ala Thr Pro Pro Leu Thr Gln Asp Leu Leu Gln Glu Glu Asp
            100                 105                 110

Phe Tyr Ile Leu Asp Gln Gly Gly Phe Lys Ile Tyr Val Trp Gln Gly
        115                 120                 125

Arg Met Ser Ser Leu Gln Glu Arg Lys Ala Ala Phe Ser Arg Ala Val
    130                 135                 140

Gly Phe Ile Gln Ala Lys Gly Tyr Pro Thr Tyr Thr Asn Val Glu Val
145                 150                 155                 160

Val Asn Asp Gly Ala Glu Ser Ala Ala Phe Lys Gln Leu Phe Arg Thr
                165                 170                 175

Trp Ser Glu Lys Arg Arg Arg Asn Gln Lys Xaa Gly Gly Arg Asp Lys
            180                 185                 190

Ser Ile His Val Lys Leu Asp Val Gly Lys Leu His Thr Gln Pro Lys
        195                 200                 205

Leu Ala Ala Gln Leu Arg Met Val Asp Asp Gly Ser Gly Lys Val Glu
    210                 215                 220

Val Trp Cys Ile Gln Asp Leu His Arg Gln Pro Val Asp Pro Lys Arg
225                 230                 235                 240

His Gly Gln Leu Cys Ala Gly Asn Cys Tyr Leu Val Leu Tyr Thr Tyr
                245                 250                 255

Gln Arg Leu Gly Arg Val Gln Tyr Ile Leu Tyr Leu Trp Gln Gly His
            260                 265                 270

Gln Ala Thr Ala Asp Glu Ile Glu Ala Leu Asn Ser Asn Ala Glu Glu
        275                 280                 285

Leu Asp Val Met Tyr Gly Gly Val Leu Val Gln Glu His Val Thr Met
    290                 295                 300

Gly Ser Glu Pro Pro His Phe Leu Ala Ile Phe Gln Gly Gln Leu Val
305                 310                 315                 320

Ile Phe Gln Glu Arg Ala Gly His His Gly Lys Gly Gln Ser Ala Ser
                325                 330                 335

Thr Thr Arg Leu Phe Gln Val Gln Gly Thr Asp Ser His Asn Thr Arg
            340                 345                 350

Thr Met Glu Val Pro Ala Arg Ala Ser Ser Leu Asn Ser Ser Asp Ile
        355                 360                 365

Phe Leu Leu Val Thr Ala Ser Val Cys Tyr Leu Trp Phe Gly Lys Gly
    370                 375                 380

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein , a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 12301 Park Lawn Drive, Rockville, Maryland 20852, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.).

### Polynucleotides and Polypeptides of the Invention

### FEATURES OF PROTEIN ENCODED BY GENE NO: 1

It has been discovered that this gene is expressed primarily in pituitary and to a lesser extent in T cells and endometrial stromal cells.

Therefore, polynucleotides and polypeptides of the invention are useful as reagents for differential identification of the tissue(s) or cell type(s) present in a biological sample and for diagnosis of diseases and conditions which include, but are not limited to, endocrine disorders and inflammation particularly in CNS injury. Similarly, polypeptides and antibodies directed to these polypeptides are in providing immunological probes for differential identification of the tissue(s) or cell type(s). For a number of disorders of the above tissues or cells, particularly of the immune system and central nervous system, expression of this gene at significantly higher or lower levels may be routinely detected in certain tissues and cell types (e.g., pituitary, T-cells, and endometrium, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for treating diseases of the endocrine system or disease that result in inflammation in the CNS. This gene maps to chromosome 1 and, therefore, is useful in chromosome mapping.

### FEATURES OF PROTEIN ENCODED BY GENE NO: 2

The translation product of this gene shares sequence homology with CDC2 serine threonine kinase which is thought to be important in regulating progression through the cell cycle.

This gene is expressed primarily in adrenal gland tumors and to a lesser extent in brain, pineal gland and gall bladder.

Therefore, polynucleotides and polypeptides of the invention are useful as reagents for differential identification of the tissue(s) or cell type(s) present in a biological sample and for diagnosis of diseases and conditions which include, but are not limited to, cancers, particularly of the adrenal gland, and endocrine disorders. Similarly, polypeptides and antibodies directed to these polypeptides are in providing immunological probes for differential identification of the tissue(s) or cell type(s). For a number of disorders of the above tissues or cells, particularly of the adrenal gland, brain and gall bladder. expression of this gene at significantly higher or lower levels may be routinely detected in certain tissues (e.g., adrenal gland, brain and other tissue of the nervous system, pineal gland, and gall bladder, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to CDC2 kinase indicates that the protein product of this clone is useful for treating cancers.

### FEATURES OF PROTEIN ENCODED BY GENE NO: 3

The translation product of this gene shares sequence homology with egg specific protein of xenopus oocytes which may play a role in binding intracellular DNA. See, Genbank accession NO: gil214636 and Eur. J. Biochem. 1992 Jun 15; 206(3): 673-683. Based on the sequence similarity between the translation product of this gene and egg-specific protein, the translation product of this gene is expected to share certain biological activities with egg-specific protein.

This gene is expressed primarily in placenta and to a lesser extent in T-cells.

Therefore, polynucleotides and polypeptides of the invention are useful as reagents for differential identification of the tissue(s) or cell type(s) present in a biological sample and for diagnosis of diseases and conditions which include, but are not limited to, inflammation. Similarly, polypeptides and antibodies directed to these polypeptides are in providing immunological probes for differential identification of the tissue(s) or cell type(s). For a number of disorders of the above tissues or cells, particularly of the immune, expression of this gene at significantly higher or lower levels may be routinely detected in certain tissues and cell types (e.g., placenta, and T-cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:53 as residues: Pro-32 to Gly-38.

The tissue distribution and homology to egg specific protein of xenopus oocytes indicates that polynucleotides and polypeptides corresponding to the gene are useful for treating inflammation mediated by T-cells.

### FEATURES OF PROTEIN ENCODED BY GENE NO: 4

The translation product of this gene shares sequence homology with mouse FGD-1 which is thought to be important in regulating the signal transduction response to small G proteins. See, for example, Genbank accession NO: gil722343.

This gene is expressed primarily in breast lymph nodes, and to a lesser extent in thymus.

Therefore, polynucleotides and polypeptides of the invention are useful as reagents for differential identification of the tissue(s) or cell type(s) present in a biological sample and for diagnosis of diseases and conditions which include, but are not limited to, immune response particularly in breast cancer. Similarly, polypeptides and antibodies directed to these polypeptides are in providing immunological probes for differential identification of the tissue(s) or cell type(s). For a number of disorders of the above tissues or cells, particularly of the immune system, expression of this gene at significantly higher or lower levels may be routinely detected in certain tissues (e.g., mammary tissue, lymphoid tissue, and thymus, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to FGD1 indicates that polynucleotides and polypeptides corresponding to the gene are useful for regulating signalling and growth of breast tumors and in inflammatory responses in the immune system.

### FEATURES OF PROTEIN ENCODED BY GENE NO: 5

The translation product of this gene shares sequence homology with a gene up-regulated by thyroid hormone in tadpoles and is expressed specifically in the tail and only at metamorphosis. (See Genbank accession NO: 1234787, see also, Brown, D.D., et al., Proc. Natl. Acad. Sci. U.S.A. 93:1924-1929 (1996). This protein is thought to be important in the tail resorption program of *Xenopus laevis*. Preferred polypeptide fragments comprise the amino acid sequence: FSVTNNTECGKLLEEIKC ALCSPHSQSLFHSPEREVLERDLVLPLLCKDYCKEFFYTCRGHIPGFLQTTADEF CFYYARKDGGLCFPDFPRKQVRGPASNYLDQMEEYDKVEEISRKHKHNCFCIQ EVVSGLRQPVGALHSGDGSQRLFILEKEGYVKILTPEGEIFKEPYLDIHKLV (SEQ ID NO: 91).

Also preferred are polynucleotide fragments encoding these polypeptide fragments.

This gene is expressed primarily in umbilical vein endothelial cells and to a lesser extent primary dendritic cells.

Therefore, polynucleotides and polypeptides of the invention are useful as reagents for differential identification of the tissue(s) or cell type(s) present in a biological sample and for diagnosis of diseases and conditions which include, but are not limited to, vascular conditions where unwanted angiogenesis occurs such as retinopathy and in conditions such as restenosis and cancer. Similarly, polypeptides and antibodies directed to these polypeptides are in providing immunological probes for differential identification of the tissue(s) or cell type(s). For a number of disorders of the above tissues or cells, particularly of the vascular system, expression of this gene at significantly higher or lower levels may be routinely detected in certain tissues and cell types (e.g., vascular tissue, endothelial cells, and dendritic cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 55 as residues: Lys-21 to Met-52, Asp-179 to Ala-189, Asp-194 to Val-202, Ile-205 to Asn-212.

The tissue distribution and homology the *Xenopus laevis* gene indicates that polynucleotides and polypeptides corresponding to the gene are useful for treatment/diagnosis of problems involving the vascular system since expression is in endothelial cells.

### FEATURES OF PROTEIN ENCODED BY GENE NO: 6

This gene is expressed primarily in an endometrial tumor and to a lesser extent in skin tumor.

Therefore, polynucleotides and polypeptides of the invention are useful as reagents for differential identification of the tissue(s) or cell type(s) present in a biological sample and for diagnosis of diseases and conditions which include, but are not limited to, tumors, in particular, skin and endometrial tumors. Similarly, polypeptides and antibodies directed to these polypeptides are in providing immunological probes for differential identification of the tissue(s) or cell type(s). For a number of disorders of the above tissues or cells, particularly of the skin and reproductive system, expression of this gene at significantly higher or lower levels may be routinely detected in certain tissues (e.g., endometrium, and epidermis, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to the gene are useful for treatment/diagnosis of endometrial and/or skin tumors, based on levels of expression in these tissues.

### FEATURES OF PROTEIN ENCODED BY GENE NO: 7

This gene is expressed primarily in human neutrophils and to a lesser extent in fetal liver.

Therefore, polynucleotides and polypeptides of the invention are useful as reagents for differential identification of the tissue(s) or cell type(s) present in a biological sample and for diagnosis of diseases and conditions which include, but are not limited to, cancers of the immune system and/or liver. Similarly, polypeptides and antibodies directed to these polypeptides are in providing immunological probes for differential identification of the tissue(s) or cell type(s). For a number of disorders of the above tissues or cells, particularly of the immune or hepatic systems, expression of this gene at significantly higher or lower levels may be routinely detected in certain tissues and cell types (e.g., blood cells, and liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to the gene are useful for regulation of cell division or treatment of cancers, particularly of the immune and hepatic systems.

### FEATURES OF PROTEIN ENCODED BY GENE NO: 8

This gene is expressed primarily in various regions of the brain including corpus callosum and hippocampus and amygdala and to a lesser extent in multiple other tissues.

Therefore, polynucleotides and polypeptides of the invention are useful as reagents for differential identification of the tissue(s) or cell type(s) present in a biological sample and for diagnosis of diseases and conditions which include, but are not limited to, diseases of the central nervous system including ischemia, epilepsy, Parkinson's disease or any other disease where neuronal survival is decreased. Similarly, polypeptides and antibodies directed to these polypeptides are in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., brain and other tissue of the nervous system, and amygdala, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 58 as residues: Ser-11 to His-21.

The tissue distribution suggests that polynucleotides and polypeptides corresponding to the gene are useful for treatment/diagnosis of conditions or diseases relating to the central nervous system based on the expression in various tissues of the brain.

### FEATURES OF PROTEIN ENCODED BY GENE NO: 9

The translation product of this gene shares sequence homology with a thioredoxine homolog from *C. elegans* which possesses dithiol-disulfide oxidoreductase activity. Preferred polypeptide fragments comprise the amino acid sequence: DGNPCDFDWREVEILMFLSAIVMMKNRRSITVEQHIGNIFMFSKVAN TILFFRLDIRMGLLYITLCIVFLMTCKPPLYMGPEYIKYFNDKTIDEELERDKRVT WIVEFFANWSNDCQSFAPIYADLSLKYNCTGLNFGKVDVGRYTDVSTRYKVST SPLTKQLPTLILFQGGKEAMRRPQIDKKGRAVSWTFSEENVIREFNLNELYQRA KKLSKA (SEQ ID NO:92). Polynucleotides encoding these polypeptide fragments are also encompassed by the invention.

This gene is expressed primarily in fetal liver and to a lesser extent in other tissue.

Therefore, polynucleotides and polypeptides of the invention are useful as reagents for differential identification of the tissue(s) or cell type(s) present in a biological sample and for diagnosis of diseases and conditions which include, but are not limited to, developmental diseases including problem with early hematapoiesis. Similarly, polypeptides and antibodies directed to these polypeptides are in providing immunological probes for differential identification of the tissue(s) or cell type(s). For a number of disorders of the above tissues or cells, particularly of the hematapoietic system, expression of this gene at significantly higher or lower levels may be routinely detected in certain tissues (e.g., liver, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 59 as residues: Pro-50 to Phe-61, Glu-148 to Arg-155, Thr-200 to Ser-209, Arg-232 to Gly-239, Gln-262 to Ser-268, Ala-270 to Val-280.

The tissue distribution and homology to thioreductase suggests that polynucleotides and polypeptides corresponding to the gene are useful for treatment of disorders involving protein folding abnormalities and diagnosis/treatment of developmental or hematapoietic disorders.

### FEATURES OF PROTEIN ENCODED BY GENE NO: 10

This gene is expressed primarily in T-cells and to a lesser extent in smooth muscle. This gene maps to chromosome 14, and therefore can be used in linkage analysis as a marker for chromosome 14.

Therefore, polynucleotides and polypeptides of the invention are useful as reagents for differential identification of the tissue(s) or cell type(s) present in a biological sample and for diagnosis of diseases and conditions which include, but are not limited to, diseases of immune dysfunction such as inflammation and autoimmunity including rheumatoid arthritis and Lupus. Similarly, polypeptides and antibodies directed to these polypeptides are in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., T-cells, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 60 as residues: Ala-3 to Thr-9, Ser-40 to Asn-53, Ser-59 to Asp-85, Gly-89 to Thr-100.

The tissue distribution suggests that polynucleotides and polypeptides corresponding to the gene are useful for treatment/diagnosis of immune and inflammatory diseases.

### FEATURES OF PROTEIN ENCODED BY GENE NO: 11

The translation product of this gene shares sequence homology with a W04A4.5 protein found in the *Caenorhabditis elegans* genome (See Accession NO: 2414330). Preferred polypeptide fragments comprise the amino acid sequence: IHLALVELLKNL TKYPTDRDSIWKCLKFLGSRHPTLVLPLVPELLSTHPFFDTAEPDMDDPAYIAVL VLIFNAAKTCPTMPALFSDHTFRHYAYLRDSLSHLVPALRLPGRKLVSSAVSPSI IPQEDPSQQFLQQSLERVYSLQHLDPQGAQELLEFTIRDLQRLGELQSELAGVAD FSATYLRCQLLLIKALQEKLWNVAAPLYLKQSDLASAAAKQIMEETYKMEFMY SGVENKQVVIIHHMRLQAKALQLIV (SEQ ID NO:94); or QLIVTARTTRGLDPLF GMCEKFLQEVDFFQRYFIADLPHLQDSFVDKLLDLMPRLMTSKPAEVVKILQTM LRQSAFLHLPLPEQIHKASATIIEPAGEFRQPFAVYLWVGGCPGMLMQPWSMC RILRTLLRSRVLYPDGQXSDDSPQACRLPESWPRAAPAHHSGLSLPHRLDRGM PGGSEAAAGLQLQCSHSKMP (SEQ ID NO:93). Polynucleotides encoding this polypeptide are also encompassed by the invention. Based on the conserved homology between invertebrate and human, it is likely that this gene plays an essential role in the development or the functions of human and animal body. This gene maps to chromosome 11, and therefore can be used in linkage analysis as a marker for chromosome 11.

This gene is expressed primarily in hypothalamus and other brain tissues and to a lesser extent in human breast, colon carcinoma, and cells of T-cell origin including T-cell lymphoma.

Therefore, polynucleotides or polypeptides of the invention are useful as reagents for differential identification of the tissue(s) or cell type(s) present in a biological sample and for diagnosis of diseases and conditions which include, but are not limited to disorder, inflammatory and immune disorders, cancers involving cells of lymphoid origin, or other infected or neoplastic lesions with T-cell infiltration. Similarly, polypeptides and antibodies directed to these polypeptides are in providing immunological probes for differential identification of the tissue(s) or cell type(s). For a number of disorders of the above tissues or cells, particularly of the immune and central nervous system including autoimmune disorders, expression of this gene at significantly higher or lower levels may be routinely detected in certain tissues and cell types (e.g., brain and other tissue of the nervous system, mammary tissue, colon, T-cells, lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in the hypothalamus indicates that the protein product of this clone is an endocrine or an extracellular protein regulatory factor in nature. The abundant presence in the brain tissues may indicate its involvement in neural development, such as neuronal survival and maintenance, neuronal connection and axonal guidance, in neural physiology, such as neural impulses transmission, short term and long term potentiation, or signal quenching. Furthermore, the gene product may have functions outside the nerve tissues as it is often found in tissues with T-cell enrichment. For example, in the lesions of colon carcinoma, breast cancer, bone marrow cells, T-cell lymphoma, activated T-cells, and tissues or cells of immune importance, the gene expression levels are significant, which indicate the immunological involvement, likely cellular immunity in nature. Therefore polynucleotides and polypeptides corresponding to the gene are useful for treatment or diagnosis of disorders of the endocrine system, neural dysfunctions or neurodegeneration, immune or inflammatory diseases, or as a proliferative/differentiation agent for cells of lymphoid origin.

### FEATURES OF PROTEIN ENCODED BY GENE NO: 12

The translation product of this gene shares sequence homology with a 27-kDa protein (mouse transporter protein (MTP)) with four predicted transmembrane-spanning domains. which is thought to be important in the transport of nucleosides and/or nucleoside derivatives between the cytosol and the lumen of an intracellular membrane-bound compartment. Preferred polypeptide fragments comprise the amino acid sequence: RFYSNSCCLCCHVRTGTILLGVWYLIINAVVLLILLSALADPDQYNFS SSELGGDFEFMDDANMCIAIAISLLMILICAMATYGAYKQRAAGIIPFFCYQIFDF ALNMLVAITVLIYPNSIQEYIRQLPPNFPYRDD (SEQ ID NO:95); or FPTEMMSCA VNPTCLVLIILLFISIILTFKGYLISCVWNCYRYINGRNSSDVLVYVTSNDTTVLL PPYDDATVNGAAKEPPPPYVSA (SEQ ID NO: 96). Polynucleotides encoding these polypeptides are also encompassed by the invention. It is likely that a second signal sequence is located upstream from the predicted signal sequence. Moreover, it is likely that a frame shift exists, which can easily be clarified using known molecular biology techniques.

This gene is expressed primarily in an endometrial tumor and normal ovary and to a lesser extent in a stromal cell line, T-cells and other cancer tissues including skin, testes chondrosarcoma, and synovial sarcoma .

Therefore, polynucleotides and polypeptides of the invention are useful as reagents for differential identification of the tissue(s) or cell type(s) present in a biological sample and for diagnosis of diseases and conditions which include, but are not limited to, cancer, particularly of the female reproductive organs and inflammatory and immune disorders. Similarly, polypeptides and antibodies directed to these polypeptides are in providing immunological probes for differential identification of the tissue(s) or cell type(s). For a number of disorders of the above tissues or cells, particularly of the female reproductive and immune], expression of this gene at significantly higher or lower levels may be routinely detected in certain tissues and cell types (e.g., ovary and testes and other reproductive tissue, stromal cells, and T-cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the MTP transporter gene suggests that polynucleotides and polypeptides corresponding to the gene are useful for treatment/diagnosis of certain cancers by blocking the ability to utilize nucleotide and nucleoside derivatives, and may also be useful modulation of immune responses by regulating the transport of these molecules.

### FEATURES OF PROTEIN ENCODED BY GENE NO: 13

The translation product of this gene shares sequence homology with a mouse cysteine-rich glycoproteina/mouse monocyte surface antigen (MS2 precursor). (See Accession NO: 1709103.) Moreover, another group recently cloned this gene, calling it human MS2, a myelomonocytic cell surface protein. (See Accession NO: 1864005.) This transmembrane protein is a member of the hemorrhagic snake venom family. Thus, based on homology, it is likely that this gene have activity similar to monocyte or myelomonocyte surface antigen M2S. Preferred polypeptide fragments comprise the amino acid sequence: IAPSRPWALMEQYEVVLPWRLPGPRVRRALPSHLGLHPE RVSYVLGATGHNFTLHLRKNRDLLGSGYTETYTAANGSEVTEQPRGQDHCFY QGHLEG (SEQ ID NO:97); PDSAASLSTCAGLRGFFQVGSDLHLIEPLDEGGEGG RHAVYQAEHLLQTAGTCGVSDDSLGSLLGPRTAAVFRPRPGDSLPSRETRYVEL YVVVDNAEFQMLGSEAAVRHRVLEVVNHVDKLYQKLNFRVVLVGLEIWNSQD RFHVSPDPSVTLENLLTWQARQRTRRHLHDNVQLITGVDFTGTTVGFARVSAM CSHSSGAVNQDHSKNPVGVACTMAHE MGHNLGMDHDENVQGCRCQ (SEQ ID NO:98); and/or FEAGRCIMARPALAPSFPRMFSDCSQAYLESFLERPQSVCLA NAPDLSHLVGGPVCGNLFVERGEQCDCGPPEDCRNRCCNSTTCQLAEGAQCA HGTCCQECKVKPAGELCRPKKDMC (SEQ ID NO:99). Polynucleotides encoding these polypeptides are also encompassed by the invention.

This gene is expressed primarily in human eosinophils and human tonsils.

Therefore, polynucleotides or polypeptides of the invention are useful as reagents for differential identification of the tissue(s) or cell type(s) present in a biological sample and for diagnosis of diseases and conditions which include, but are not limited to, disorders relating to eosinophilic leukocyte, and tonsillitis. Similarly, polypeptides and antibodies directed to these polypeptides are in providing immunological probes for differential identification of the tissue(s) or cell type(s). For a number of disorders of the above tissues or cells, particularly of the hematopoietic system and lymphoid system, expression of this gene at significantly higher or lower levels may be routinely detected in certain tissues and cell types (e.g., blood cells, and tonsils, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product of this clone is useful for diagnosis and treatment of immune disorders.

### FEATURES OF PROTEIN ENCODED BY GENE NO: 14

A polypeptide sequence which overlaps with the translation product of this gene has recently been identified as g16 (see Genbank accession NO: gil2636658). These proteins are thought to be tumor suppressors.

This gene is expressed primarily in immune system cells, e.g., eosinophils, activated T-cells, activated monocytes, activated neutrophils, dendritic cells, Hodgkin's lymphoma, and in vascularized tissues such as umbilical vein, microvascular endothelial cells and trachea.

Therefore, polynucleotides and polypeptides of the invention are useful as reagents for differential identification of the tissue(s) or cell type(s) present in a biological sample and for diagnosis of diseases and conditions which include, but are not limited to, immune system disorders such as cancer. Similarly, polypeptides and antibodies directed to these polypeptides are in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues and cell types (e.g., blood cells, dendritic cells, vascular tissue, and lymphoid tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and similarity to g16 suggests that polynucleotides and polypeptides corresponding to the gene are useful for diagnosis and treatment of immune system disorders such as cancers. It is believed that tumor suppressor genes are often deleted in particular cancers.

### FEATURES OF PROTEIN ENCODED BY GENE NO: 15

The translation product of this gene shares sequence homology with a frog thrombin receptor [Xenopus laevis]. Moreover, another group recently cloned this same gene, also recognizing the homology to thrombing receptors. (See Accession NO: 2347084.) Preferred polypeptide fragments comprise the amino acid sequence: MLPD WKXSLILMAYIIIFLTGLPANLLALRAFVGRIRQPQPAPVHILLLSLTLADLLLLLL LPFKIIEAASNFRWYLPKVVCALTSFGFYSSIYCSTWLLAGISIERYLGVAFPVQ YKLSRRPLYGVIAALVAWVMSFGHCTIVIIXQYLNTTEQVRSGNEITCYENFTD NQLDVVLPVRXELCLVLFFXPMAVTIFCYWRFVWIMLSQPLVGAQRRRRAVGL AVVTLLNFLVCFGPYNVSHLVGYHQRKSPWWRSIAVXFSSLNASLDPLLFYFS SSVVRRAFGRGLQVLRNQGSSLLGRRGKDTAEGTNEDRGVGQGEGMPSSDFT TE (SEQ ID NO:100); CSTWLLAGISIERYLGV (SEQ ID NO:101); or CTIVIIXQYL NTTEQVRSGNEITCYENFTDNQLDVVLPVRXELCLVLFFXPMAVTIFCYWRFV WIMLSQPLVGAQRRRRAVGLAVVTLLNFLVC (SEQ ID NO:102). Polynucleotides encoding these polypeptides are also encompassed by the invention.

Also preferred are the polynucleotide fragments encoding these polypeptide fragments. This gene maps to chromosomal location 19q13.1, and therefore can be used as a marker in linkage analysis for chromosome 19.

This gene is expressed primarily in activated human neutrophil and IL5 induced eosinophil.

Therefore, polynucleotides and polypeptides of the invention are useful as reagents for differential identification of the tissue(s) or cell type(s) present in a biological sample and for diagnosis of diseases and conditions which include, but are not limited to, neutropenia, neutrophilia, and eosinophilic leukocyte related disorders. Similarly, polypeptides and antibodies directed to these polypeptides are in providing immunological probes for differential identification of the tissue(s) or cell type(s). For a number of disorders of the above tissues or cells, particularly of the hematopoietic system and hemopoietic system, expression of this gene at significantly higher or lower levels may be routinely detected in certain tissues and cell types (e.g., bone marrow, blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 65 as residues: Tyr-41 to Trp-48.

The tissue distribution a suggests that polynucleotides and polypeptides corresponding to the gene are useful for diagnosis and treatment of immune disorders.

### FEATURES OF PROTEIN ENCODED BY GENE NO: 16

This gene is homologous to the mouse NP15.6 gene, a novel neuronal protein whose expression is developmentally regulated. (See Accession NO: 1771306.) Therefore, based on homology, it is likely that this gene would have activity similar to NP15.6. Preferred polypeptide fragments comprise the amino acid sequence: GLPAARVRWESSFSRTVVAPSAVAXKRPPEPTTPWQEDPEPEDENLYEKNPDS HGYDKDPVLDVWNMRLVFFFGVSIILVLGSTFVAYLPDYRCTGCPRAWDGMK EWSRREAERLVKYREANGLPIMESNCFDPSKIQLPEDE (SEQ ID NO:103). Polynucleotides encoding these polypeptides are also encompassed by the invention.

This gene maps to chromosome X, and therefore polynucleotides of the present invention can be used in linkage analysis as a marker for chromosome X.

This gene is expressed primarily in hematopoietic cells.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., hematopoietic cells, and tissue of the nervous system, and cancerous and wounded tissues) or bodily fluids (e.g., serum, plasma, urine, synovial fluid or spinal fluid) or 0 another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 66 as residues: Pro-24 to Gly-30, Gly-37 to Ala-46.

The tissue distribution indicates that the protein product of this clone is useful for diagnosis and treatment of immune and endocrine disorders and neoplasias.

### FEATURES OF PROTEIN ENCODED BY GENE NO: 17

The translation product of this gene shares sequence homology with Preprotachykinin B which is thought to be important in the signal transduction and information processing in the nervous system. (See Accession NO:163590; see also Kotani,H., et al., Proc. Natl. Acad. Sci. U.S.A. 83:7074-7078 (1986).) The tachykinin group of neuropeptides exists in four different forms which are derived from one gene in the rat. Alternative splicing accounts for the alpha, beta, gamma, and delta forms. The most famous of these neuropeptides is substance P which appears to mediate the pain sensation and wheal formation in certain *in vivo* models. It thus may be a key player in the inflammatory response. The tachykinins also have smooth muscle contraction (i.e. bronchoconstriction) and vasodilator effects. Additionally, neovascularization and various cell-type specific proliferation effects have been seen. The fact that this clone was isolated from placenta RNA may make this an interesting gene to characterize. The known neurokinins are expressed in either the central nervous system or peripheral neurons. It may be that this new neurokinin modulates smooth muscle or vascularization associated with reproduction. Therefore, base on homology it is likely that the polypeptides of the invention are active in the signal transduction and information processing in the nervous system. Preferred polypeptide fragments comprise the amino acid sequence: PEKRDMHDFFVGLMGKRSVQPDSPTDVNQE NVPSFG (SEQ ID NO: 104); KRDMHDFFVGLMGKR (SEQ ID NO: 105); and/or DMHDFFVGLM (SEQ ID NO: 106). Polynucleotides encoding these polypeptides are also encompassed by the invention. This maps to chromosome 12 and therefore can be used in linkage analysis as a marker for chromosome 12.

This gene is expressed primarily in human placenta and to a lesser extent in soares placenta.

Therefore, polynucleotides and polypeptides of the invention are useful as reagents for differential identification of the tissue(s) or cell type(s) present in a biological sample and for diagnosis of diseases and conditions which include, but are not limited to, embryonic and reproductive disorders. Similarly, polypeptides and antibodies directed to these polypeptides are in providing immunological probes for differential identification of the tissue(s) or cell type(s). For a number of disorders of the above tissues or cells, particularly of the reproductive and embryonic systems, expression of this gene at significantly higher or lower levels may be routinely detected in certain tissues (e.g., placenta, and tissue of the nervous system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 67 as residues: Gly-34 to Asp-42, Ala-67 to Asp-81, Arg-93 to Asn-107.

The tissue distribution and homology to preprotachykinin B suggests that polynucleotides and polypeptides corresponding to the gene are useful for the diagnosis and treatment of reproductive and embryonic disorders, and cancer. These polypeptides and polynucleotides of the invention can also be used to treat Alzheimer's disease by inhibition of neurotoxicity due to the beta-amyloid peptide and long-lasting analgesic and anti-inflammatory activities by neurokinin B analogs.

### FEATURES OF PROTEIN ENCODED BY GENE NO: 18

The translation product of this gene shares sequence homology with ftp-3, an hnRNP protein which is thought to be important in RNA splicing and packaging. In preferred embodiments, the polypeptides of the invention comprise the sequence: EWEATEEMEWIIREAM (SEQ ID NO: 107); WEWGTITVEDMVLLMVWVVMAVV VEAVEVTMGKAA (SEQ ID NO:108); GMGGYGRDGMDNQGGYGS (SEQ ID NO:109); and/or GMGNNYSGGYGTPDGLGGYGRGGGGSGGYYGQGGMSGG GWRGM (SEQ ID NO:110). Polynucleotides encoding these polypeptides are also encompassed by the invention.

This gene is expressed primarily in frontal cortex and amygdala of human brain and to a lesser extent in human smooth muscle.

Therefore, polynucleotides and polypeptides of the invention are useful as reagents for differential identification of the tissue(s) or cell type(s) present in a biological sample and for diagnosis of diseases and conditions which include, but are not limited to, human brain diseases. Similarly, polypeptides and antibodies directed to these polypeptides are in providing immunological probes for differential identification of the tissue(s) or cell type(s). For a number of disorders of the above tissues or cells, particularly of the nervous system, expression of this gene at significantly higher or lower levels may be routinely detected in certain tissues (e.g., brain and other tissue of the nervous system, and smooth muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ftp-3 indicates that the protein product of this clone is useful for the diagnosis and treatment of human brain diseases and disorders involving improper RNA splicing such as thalessemia. Additionally, this gene maps to chromosome 10 and therefore polypeptides of the present invention can be used in linkage analysis as a marker for chromosome 10.

### FEATURES OF PROTEIN ENCODED BY GENE NO: 19

The translation product of this gene shares sequence homology with immunoglobulin lambda light chain which is thought to be important in immunal functions.

This gene is expressed primarily in human thymus and to a lesser extent in human colon, soares breast, bone marrow and breast lymph node.

Therefore, polynucleotides and polypeptides of the invention are useful as reagents for differential identification of the tissue(s) or cell type(s) present in a biological sample and for diagnosis of diseases and conditions which include, but are not limited to, immunal diseases. Similarly, polypeptides and antibodies directed to these polypeptides are in providing immunological probes for differential identification of the tissue(s) or cell type(s). For a number of disorders of the above tissues or cells, particularly of the immune system expression of this gene at significantly higher or lower levels may be routinely detected in certain tissues (e.g., thymus, colon, mammary tissue, bone marrow, and lymphoid tissue, and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 69 as residues: Gly-43 to Asp-50, Gln-57 to Lys-65, Arg-70 to Gly-77, Thr-185 to Tyr-195, Pro-205 to Ser-215.

The tissue distribution and homology with immunoglobulin lambda light chain indicates that polynucleotides and polypeptides corresponding to the gene are useful for the diagnosis and/or treatment of immunal diseases.

### FEATURES OF PROTEIN ENCODED BY GENE NO: 20

The translation product of this gene shares sequence homology with *Xenopus* chordin (Accession NO:L35764) which is thought to be important in dorsal-ventral patterning and is activated by organizer-specific homeobox genes. See, e.g., Sasai, Y., et al., Cell 79:779-790 (1994).) This gene has also been determined to be a powerful morphogen.

This gene is expressed primarily in early stage human tissues, prostate, and adipose tissues and to a lesser extent, in other tissues.

Therefore, polynucleotides and polypeptides of the invention are useful as reagents for differential identification of the tissue(s) or cell type(s) present in a biological sample and for diagnosis of diseases and conditions which include, but are not limited to, developmental disorders. Similarly, polypeptides and antibodies directed to these polypeptides are in providing immunological probes for differential identification of the tissue(s) or cell type(s). For a number of disorders of the above tissues or cells, particularly of the embryo and fetal tissues, expression of this gene at significantly higher or lower levels may be routinely detected in certain tissues (e.g., embryonic and fetal tissue, prostate, and adipose tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 70 as residues: Asn-28 to Trp-38, Val-57 to Lys-64, His-66 to Lys-82, Glu-90 to Gly-100, Glu-210 to Cys-217.

Chordin plays important role dorsal-ventral patterning in *Xenopus*. The tissue distribution and homology to chordin suggests that polynucleotides and polypeptides corresponding to the gene are useful for diagnosis and treatment of wounds and developmental disorders, such as cancer.

### FEATURES OF PROTEIN ENCODED BY GENE NO: 21

This gene is expressed primarily in immune tissues such as monocyte, fetal liver, fetal spleen, T-cell, thymus etc. and to a lesser extent in colon cancer, breast cancer, early stage human tissues and a few other tissues.

Therefore, polynucleotides and polypeptides of the invention are useful as reagents for differential identification of the tissue(s) or cell type(s) present in a biological sample and for diagnosis of diseases and conditions which include, but are not limited to, immune disorders such as immune deficiencies, autoimmune diseases, and inflammatory diseases. Similarly, polypeptides and antibodies directed to these polypeptides are in providing immunological probes for differential identification of the tissue(s) or cell type(s). For a number of disorders of the above tissues or cells, particularly of the immune systems, expression of this gene at significantly higher or lower levels may be routinely detected in certain tissues and cell types (e.g., blood cells, liver, spleen, thymus, colon, and mammary tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 71 as residues: Glu-61 to Thr-67, Glu-72 to Asp-81, Glu-83 to Asp-118, Gly-156 to Arg-162, Asp-184 to Tyr-205, Met-251 to Asp-257, Ser-284 to Tyr-293, Lys-351 to Arg-357, Gly-367 to Asp-375, Asn-399 to Glu-414, Gln-424 to Arg-443, Glu-447 to Glu-457, Arg-462 to Lys-476, Lys-485 to Phe-492.

The tissue distribution suggests that polynucleotides and polypeptides corresponding to the gene are useful for diagnosis and treatment of immune disorders such as immune deficiencies, autoimmune diseases, and inflammatory diseases.

### FEATURES OF PROTEIN ENCODED BY GENE NO: 22

In one embodiment of the invention, the polypeptides of the invention comprise the sequence FTHSFILEHAFSLLITLPVSSWAANN (SEQ ID NO: 111).

This gene is expressed primarily in chronic synovitis and to a lesser extent in other tissues.

Therefore, polynucleotides and polypeptides of the invention are useful as reagents for differential identification of the tissue(s) or cell type(s) present in a biological sample and for diagnosis of chronic synovitis. Similarly, polypeptides and antibodies directed to these polypeptides are in providing immunological probes for differential identification of the tissue(s) or cell type(s). For a number of disorders of the synovium, expression of this gene at significantly higher or lower levels may be routinely detected in certain tissues (e.g., synovial tissue and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution suggests that polynucleotides and polypeptides corresponding to the gene are useful for diagnosis and treatment of chronic synovitis.

### FEATURES OF PROTEIN ENCODED BY GENE NO: 23

This gene is expressed primarily in testes and to a lesser extent in other tissues.

Therefore, polynucleotides and polypeptides of the invention are useful as reagents for differential identification of the tissue(s) or cell type(s) present in a biological sample and for diagnosis of diseases and conditions which include, but are not limited to, testes related diseases such as infertility and endocrine disorders. Similarly, polypeptides and antibodies directed to these polypeptides are in providing immunological probes for differential identification of the tissue(s) or cell type(s). For a number of disorders of the above tissues or cells, particularly of the testes, expression of this gene at significantly higher or lower levels may be routinely detected in certain tissues (e.g., testes and other reproductive tissue, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution suggests that polynucleotides and polypeptides corresponding to the gene are useful for diagnosis and treatment of testes related diseases such as infertility and endocrine disorders.

### FEATURES OF PROTEIN ENCODED BY GENE NO: 24

The translation product of this gene shares sequence homology with the nucleotide sequence of a new HLA-DRB1(*)11 allele (DRB1(*)1124), which is thought to be important in organ transplantation and immune disorders. The translation product of this gene also shares homology with protease inhibitors such as aprotinin and others with Kunitz-type domains. Kunitz-type domains are known in the art to possess protease inhibiting activity. A Kunitz-type domain is contained within the translation product of this gene and has the amino acid sequence: CEMPKETGPCLAY FLHWWYDKKDNTCSMFVYGGCQGNNNNFQSKANCLNTC (SEQ ID NO:112). Thus, preferred polypeptides of the invention comprise the amino acid sequence of the Kunitz-type domain shown immediately above.

It has been discovered by analyzing hundreds of thousands of ESTs that this gene is expressed primarily in the testes and epididymus. Northern blot analysis has confirmed expression primarily in the testes.

Therefore, polynucleotides and polypeptides of the invention are useful as reagents for differential identification of the tissue(s) or cell type(s) present in a biological sample and for diagnosis of diseases and conditions which include, but are not limited to, diseases related to the testes and epididymus. Similarly, polypeptides and antibodies directed to these polypeptides are in providing immunological probes for differential identification of the tissue(s) or cell type(s). For a number of disorders of the above tissues or cells, particularly of the diseases related to the testes and epididymus, and organ transplantation. expression of this gene at significantly higher or lower levels may be routinely detected in certain tissues (e.g., testes and other reproductive tissue, and tissue and cells of the immune system, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in (SEQ ID NO:74) as residues: Pro-30 to Arg-37, Val-47 to Lys-59, Trp-94 to Thr-101, Cys-110 to Cys-123, Thr-126 to Pro-133.

The tissue distribution and homology to protease inhibitors indicates that polynucleotides and polypeptides corresponding to the gene are useful for diagnosis and treatment of diseases related to the testes, epididymus, and organ transplantation. More specifically, these polypeptides are particularly useful in the treatment of hyperfilbronolytic hemorrhage and traumatic hemorrhagic shock as well as in diseases connected with excessive release of pancreatic elastase (pancreatitis), serum elastase (artherosclerosis), leukocyte elastase in acute and chronic inflammation with damage to connective tissue, in damage to vessel walls, in necrotic diseases, and degeneration of lung tissue.

### FEATURES OF PROTEIN ENCODED BY GENE NO: 25

The translation product of this gene shares sequence homology with CpG islands genes which are short stretches of DNA containing a high density of non-methylated CpG dinucleotides, predominantly associated with coding regions. As CpG islands overlap with approximately 60% of human genes, the CpG island library can be used to isolate full-length cDNAs and to place genes on genomic maps.

This gene is expressed primarily in the testes and to a lesser extent in the lung, tonsils, placenta, and rhabdomyosarcoma.

Therefore, polynucleotides and polypeptides of the invention are useful as reagents for differential identification of the tissue(s) or cell type(s) present in a biological sample and for diagnosis of diseases and conditions which include, but are not limited to, diseases related to the testes, lung, tonsils, placenta, and tumors. Similarly, polypeptides and antibodies directed to these polypeptides are in providing immunological probes for differential identification of the tissue(s) or cell type(s). For a number of disorders of the above tissues or cells, particularly of the diseases related to the testes, lung, tonsils, placenta, and tumors, expression of this gene at significantly higher or lower levels may be routinely detected in certain tissues (e.g., testes and other reproductive tissue, lung, tonsils, placenta, and striated muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:75 as residues: Met-1 to His-7.

The tissue distribution indicates that polynucleotides and polypeptides corresponding to the gene are useful for diagnosis and treatment of diseases related to the testes, lung, tonsils, placenta, and tumors.

### FEATURES OF PROTEIN ENCODED BY GENE NO: 26

The translation product of this gene shares sequence homology with the sequence of human villin: a large duplicted domain homologue with other actin severing proteins and a unique small carboxy terminal domain related to villin specificity which is thought to be important in actin capping and processing. This gene has now been published. See DNA Res. (1997) 28:4(1):35-43. It has been shown that this gene is homozygously deleted in a lung carcinoma cell line suggesting a possible role for the translation product of this gene in suppressing tumors. In any case, a suppressor gene is likely located close to this gene and accordingly, this gene can be used as a cancer marker. Preferred polypeptides of this invention comprise the following amino acid sequence: MMIQWNGPKTSISEKARGLXLTYSLRDRERGGGRAQIGVVDDEAKA PDLMQIMEVLGRRVGXLRXATPSKDINQLQKANVRLYHVYEKGKDLVVLELA TPPLTQDLLQEEDFYILDQGGFKIYVWQGRMSSLQERKAAFSRAVGFIQAKGYP TYTNVEVVNDGAESAAFKQLFRTWSEKRRRNQKXGGRDKSIHVKLDVGKLH TQPKLAAQLRMVDDGSGKVEVWCIQDLHRQPVDPKRHGQLCAGNCYLVLYTY QRLGRVQYILYLWQGHQATADEIEALNSNAEELDVMYGGVLVQEHVTMGSEPP HFLAIFQGQLVIFQERAGHHGKGQSASTTRLFQVQGTDSHNTRTMEVPARASS LNSSDIFLLVTASVCYLWFGKG (SEQ ID NO:113).

It has been discovered by analyzing EST sequences that this gene is expressed primarily in a healing wound 7.5 hours after incision, pancreas tumor, CD34+ cell, human osteoclastoma, stromal cells, human thymus and to a lesser extent in pancreas tumor, spleen, and apoptotic T cell. Northern blots were carried out and showed that this gene was expressed in all tissues tested: spleen, thymus, prostate, testis, ovary, small intestine, colon, and peripheral blood leukocytes. The most intense band (expression) was seen in the colon, with the least intense band seen in peripheral blood leukocytes.

Therefore, polynucleotides and polypeptides of the invention are useful as reagents for differential identification of the tissue(s) or cell type(s) present in a biological sample and for diagnosis of diseases and conditions which include, but are not limited to, cancers including, lung carcinoma, osteoclastoma, pancreas tumor, immune disorders, and infectious diseases. Similarly, polypeptides and antibodies directed to these polypeptides are in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., bone, stromal cells, thymus, pancreas, lung, spleen, and blood cells, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to the Villin family of actin severing proteins suggests that polynucleotides and polypeptides corresponding to the gene are useful for diagnosis and treatment of cancers, particularly osteoclastoma, pancreas tumor, lung carcinoma, other immune disorders, and infectious diseases. It has recently been shown that sputum samples from cystic fibrosis patients contains actin filaments and that plasma gelsolin can reduce the viscosity of these samples. Accordingly, the translation product of this gene is useful in the treatment of cystic fibrosis. This gene has been mapped to 3p22-p21.3.

### FEATURES OF PROTEIN ENCODED BY GENE NO: 27

This gene is expressed primarily in a human HCC cell line, mouse liver metastasis and muscle tissue from a human patient with multiple sclerosis.

Therefore, polynucleotides and polypeptides of the invention are useful as reagents for differential identification of the tissue(s) or cell type(s) present in a biological sample and for diagnosis of diseases and conditions which include, but are not limited to, tumor metastasis and multiple sclerosis. Similarly, polypeptides and antibodies directed to these polypeptides are in providing immunological probes for differential identification of the tissue(s) or cell type(s). For a number of disorders of the above tissues or cells, particularly of the immune system expression of this gene at significantly higher or lower levels may be routinely detected in certain tissues (e.g., liver, and muscle, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:77 as residues: Ser-21 to Asp-32.

The tissue distribution indicates that polynucleotides and polypeptides corresponding to the gene are useful for diagnosis and treatment of multiple sclerosis and tumor metastasis. The nucleotide sequence 3' of the poly A tail, as shown in the sequence listing is vector sequence as would be readily appreciated by those of skill in the art. Polypeptides of the invention preferrably do not contain such vector sequences or sequences which hybridize to such vector sequences.

### FEATURES OF PROTEIN ENCODED BY GENE NO: 28

The translation product of this gene shares sequence homology with the sequence CEESL52F (Genbank accession NO: U80441); coded for by *C. elegans* cDNA yk5 which is thought to be important in embryonic development. The translation product of this gene has recently been described elsewhere (See Proc. Natl. Acad. Sci. U S A (1997) 8:94(14):7481-7486, incorporated herein by reference in its entirety), as hCTR2: a human gene for copper uptake.

This gene is expressed primarily in placenta and human amygdala, and to a lesser extent in adult brain, primary dendritic cells, keratinocytes, activated monocytes, human cerebellum, and activated T-cells.

Therefore, polynucleotides and polypeptides of the invention are useful as reagents for differential identification of the tissue(s) or cell type(s) present in a biological sample and for diagnosis of diseases and conditions which include, but are not limited to, embryonic development, neuronal cell differentiation, disorders associated with copper metabolism and immune responses. Similarly, polypeptides and antibodies directed to these polypeptides are in providing immunological probes for differential identification of the tissue(s) or cell type(s). For a number of disorders of the above tissues or cells, particularly of the abnormal embryonic development, neuronal cell disorders, disorders involving abnormal copper metabolism and immune system disorders, expression of this gene at significantly higher or lower levels may be routinely detected in certain tissues (e.g., placenta, amygdala, brain and other tissue of the nervous system, dendritic cells, blood cells, keratinocytes, and cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 78 as residues: Ser-24 to Trp-30.

The tissue distribution and similarity to hCTR1 and hCTR2 indicates that polynucleotides and polypeptides corresponding to the gene are useful for diagnosis and treatment of abnormal embryonic development, neuronal cell disorders, disorders involving copper metabolism and immune system disorders. This gene has been mapped to 9q31-q32.

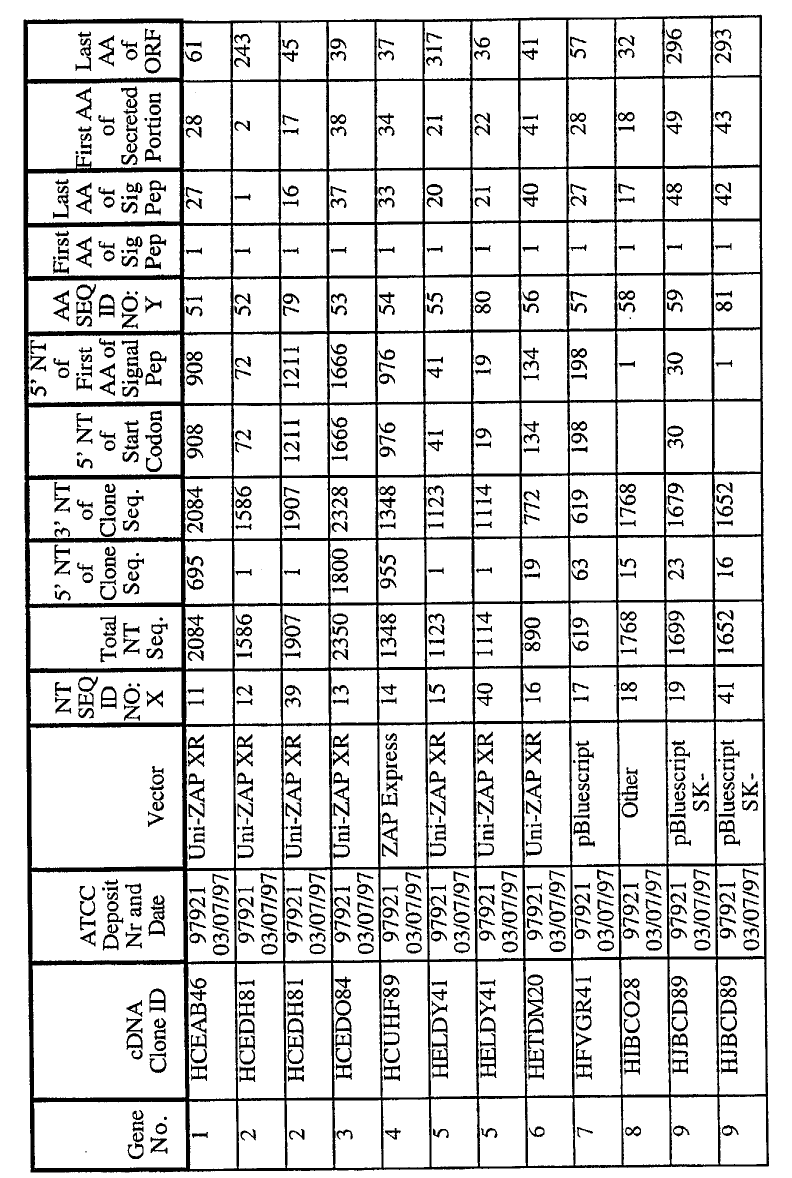

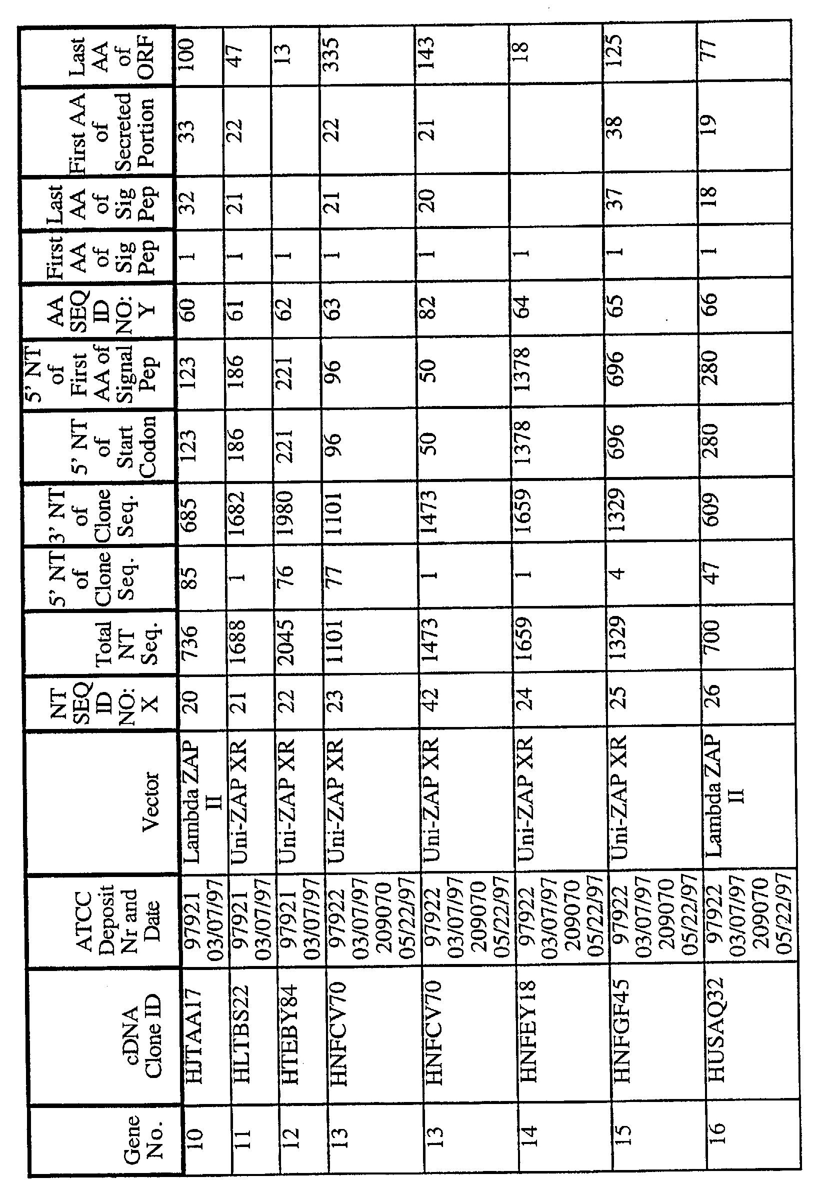

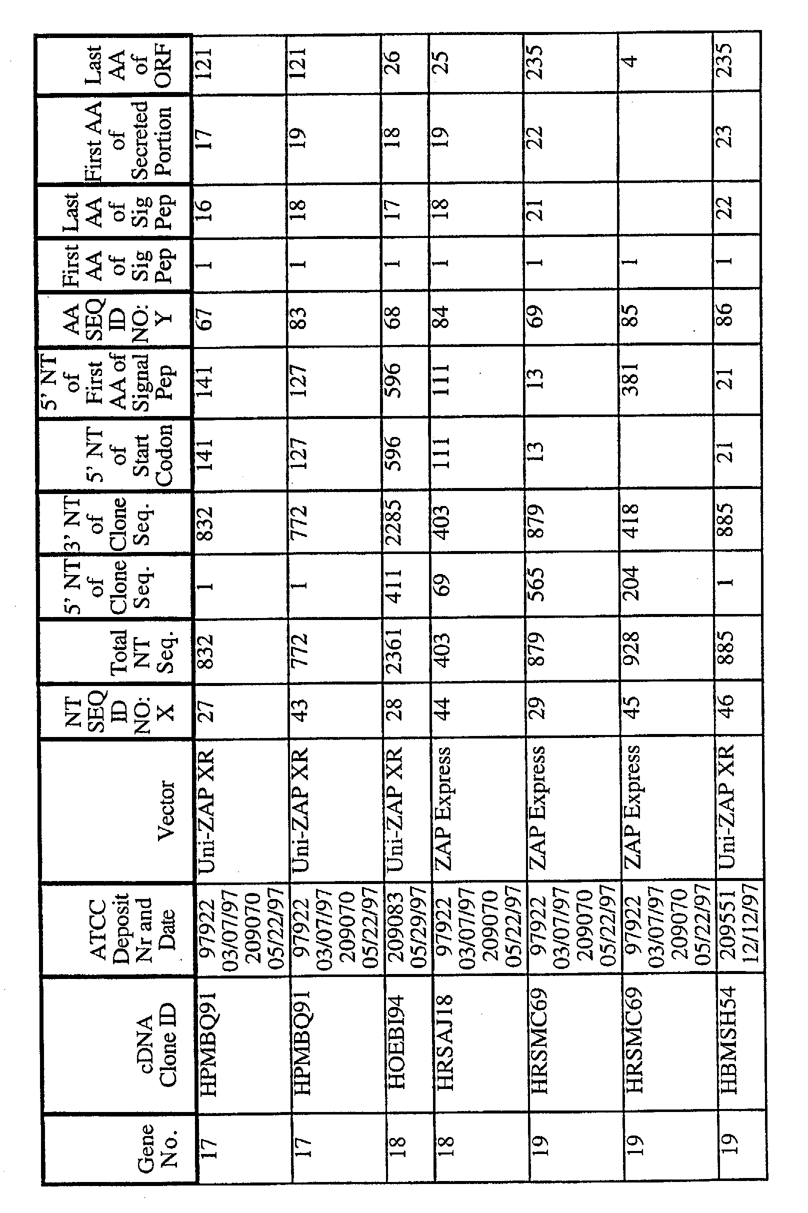

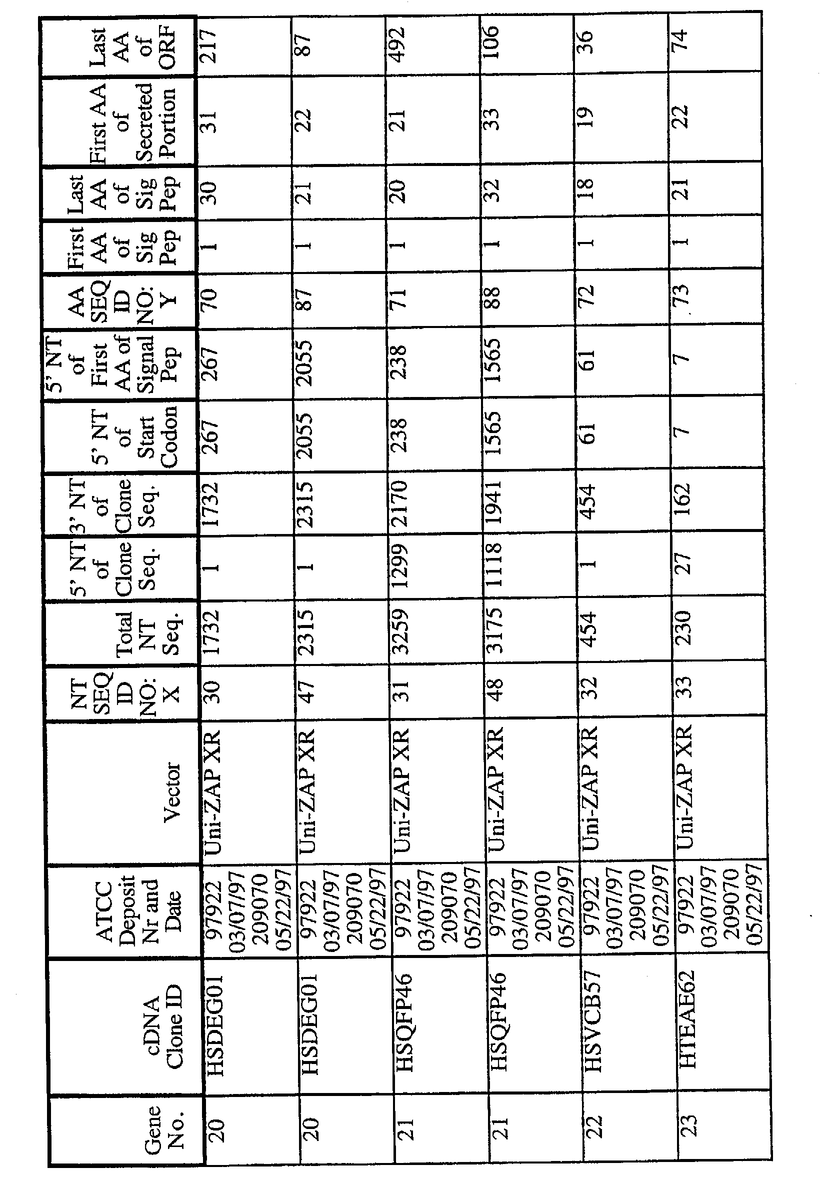

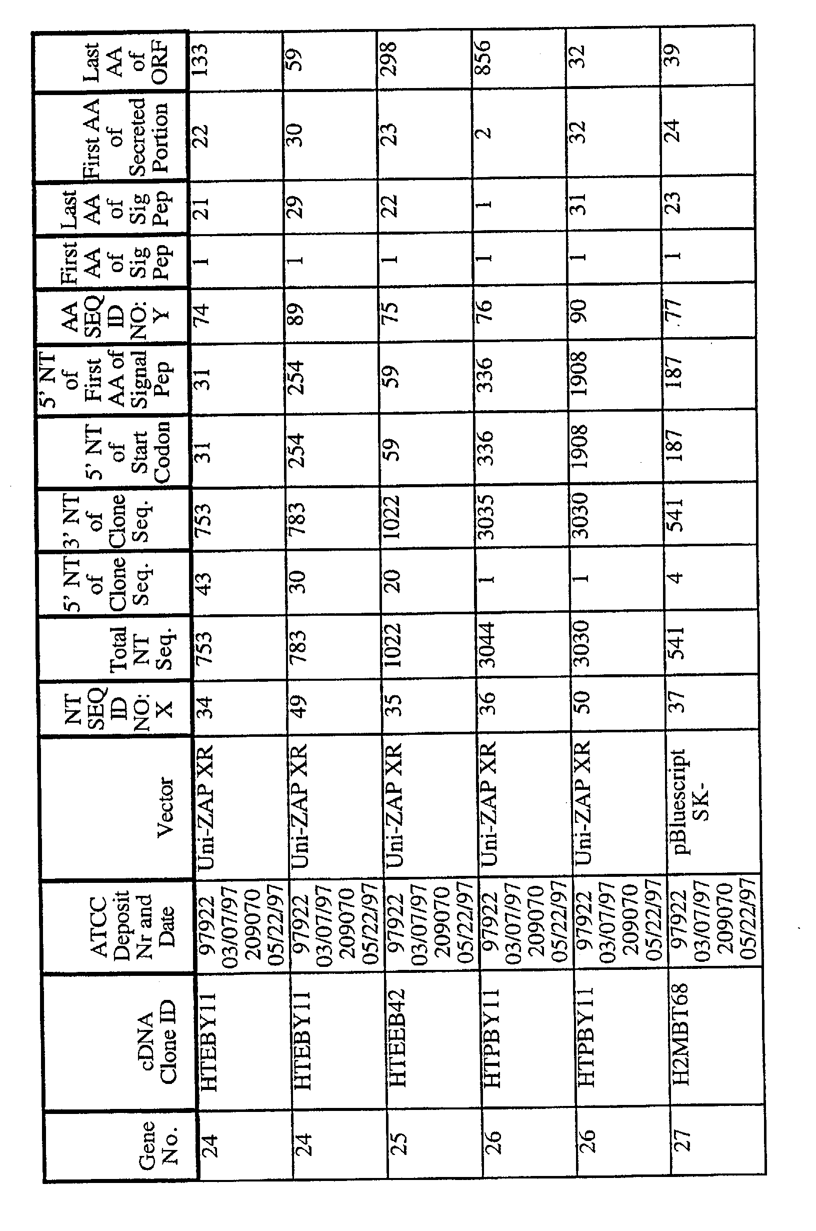

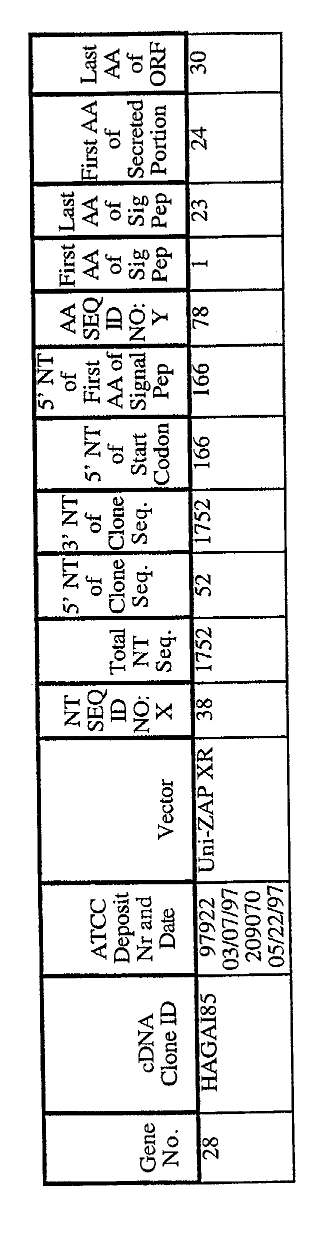

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

"Identity" per se has an art-recognized meaning and can be calculated using published techniques. (See, e.g.: (COMPUTATIONAL MOLECULAR BIOLOGY, Lesk, A.M., ed., Oxford University Press, New York, (1988); BIOCOMPUTING: INFORMATICS AND GENOME PROJECTS, Smith, D.W., ed., Academic Press, New York, (1993); COMPUTER ANALYSIS OF SEQUENCE DATA, PART I, Griffin, A.M., and Griffin, H.G., eds., Humana Press, New Jersey, (1994); SEQUENCE ANALYSIS IN MOLECULAR BIOLOGY, von Heinje, G., Academic Press, (1987); and SEQUENCE ANALYSIS PRIMER, Gribskov, M. and Devereux, J., eds., M Stockton Press, New York, (1991).) While there exists a number of methods to measure identity between two polynucleotide or polypeptide sequences, the term "identity" is well known to skilled artisans. (Carillo, H., and Lipton, D., SIAM J Applied Math 48:1073 (1988).) Methods commonly employed to determine identity or similarity between two sequences include, but are not limited to, those disclosed in "Guide to Huge Computers," Martin J. Bishop, ed., Academic Press, San Diego, (1994), and Carillo, H., and Lipton, D., SIAM J Applied Math 48:1073 (1988). Methods for aligning polynucleotides or polypeptides are codified in computer programs, including the GCG program package (Devereux, J., et al., Nucleic Acids Research (1984) 12(1):387 (1984)), BLASTP, BLASTN, FASTA (Atschul, S.F. et al., J. Molec. Biol. 215:403 (1990), Bestfit program (Wisconsin Sequence Analysis Package, Version 8 for Unix, Genetics Computer Group, University Research Park, 575 Science Drive, Madison, WI 53711 (using the local homology algorithm of Smith and Waterman, Advances in Applied Mathematics 2:482-489 (1981).)

When using any of the sequence alignment programs to determine whether a particular sequence is, for instance, 95% identical to a reference sequence, the parameters are set so that the percentage of identity is calculated over the full length of the reference polynucleotide and that gaps in identity of up to 5% of the total number of nucleotides in the reference polynucleotide are allowed.

A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245 (1990).) The term "sequence" includes nucleotide and amino acid sequences. In a sequence alignment the query and subject sequences are either both nucleotide sequences or both amino acid sequences. The result of said global sequence alignment is in percent identity. Preferred parameters used in a FASTDB search of a DNA sequence to calculate percent identiy are: Matrix=Unitary, k-tuple=4, Mismatch Penalty=1, Joining Penalty=30, Randomization Group Length=0, and Cutoff Score=1, Gap Penalty=5, Gap Size Penalty 0.05, and Window Size=500 or query sequence length in nucleotide bases, whichever is shorter. Preferred parameters employed to calculate percent identity and similarity of an amino acid alignment are: Matrix=PAM 150, k-tuple=2, Mismatch Penalty=1, Joining Penalty=20, Randomization Group Length=0, Cutoff Score=1, Gap Penalty=5, Gap Size Penalty=0.05, and Window Size=500 or query sequence length in amino acid residues, whichever is shorter.

As an illustration, a polynucleotide having a nucleotide sequence of at least 95% "identity" to a sequence contained in SEQ ID NO:X or the cDNA contained in the deposited clone, means that the polynucleotide is identical to a sequence contained in SEQ ID NO:X or the cDNA except that the polynucleotide sequence may include up to five point mutations per each 100 nucleotides of the total length (not just within a given 100 nucleotide stretch). In other words, to obtain a polynucleotide having a nucleotide sequence at least 95% identical to SEQ ID NO:X or the deposited clone, up to 5% of the nucleotides in the sequence contained in SEQ ID NO:X or the cDNA can be deleted, inserted, or substituted with other nucleotides. These changes may occur anywhere throughout the polynucleotide.

Further embodiments of the present invention include polynucleotides having at least 85% identity, more preferably at least 90% identity, and most preferably at least 95%, 96%, 97%, 98% or 99% identity to a sequence contained in SEQ ID NO:X or the cDNA contained in the deposited clone. Of course, due to the degeneracy of the genetic code, one of ordinary skill in the art will immediately recognize that a large number of the polynucleotides having at least 85%, 90%, 95%, 96%, 97%, 98%, or 99% identity will encode a polypeptide identical to an amino acid sequence contained in SEQ ID NO:Y or the expressed protein produced by the deposited clone.

Similarly, by a polypeptide having an amino acid sequence having at least, for example, 95% "identity" to a reference polypeptide, is intended that the amino acid sequence of the polypeptide is identical to the reference polypeptide except that the polypeptide sequence may include up to five amino acid alterations per each 100 amino acids of the total length of the reference polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

Further embodiments of the present invention include polypeptides having at least 80% identity, more preferably at least 85% identity, more preferably at least 90% identity, and most preferably at least 95%, 96%, 97%, 98% or 99% identity to an amino acid sequence contained in SEQ ID NO:Y or the expressed protein produced by the deposited clone. Preferably, the above polypeptides should exhibit at least one biological activity of the protein.

In a preferred embodiment, polypeptides of the present invention include polypeptides having at least 90% similarity, more preferably at least 95% similarity, and still more preferably at least 96%, 97%, 98%, or 99% similarity to an amino acid sequence contained in SEQ ID NO:Y or the expressed protein produced by the deposited clone.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, or 701 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998-4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell . Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or E. *coli*. Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the fl origin of replication ("ori"), such that in one orientation, single stranded rescue initiated from the fl ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for I min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and Xbal and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra).*

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4°C or frozen at -80° C. In addition to the above expression vector, the present invention further includes an expression vector comprising phage operator and promoter elements operatively linked to a polynucleotide of the present invention, called pHE4a. (ATCC Accession Number XXXXXX.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, Xhol, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCI. The CM-20 column is then eluted using a 10 column volume linear gradient ranging from 0.2 M NaCI, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E*. *coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells. The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra).* The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

### Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC

ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-lml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (116.6 mg/L of CaCl2 (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂0; 71.02 mg/L of Na₂HPO₄; 4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂0; 6.65 mg/ml of L-Aspartic Acid; 29.56 mg/ml of L-Cystine-2HCL-H₂0; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂0; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂0; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | | **JAKs** | | | **STATS** | **GAS(elements) or ISRE Ligand** |
|---|---|---|---|---|---|---|
| | **tyk2** | **Jak1** | **Jak2** | **Jak3** | | |
| IFN family | | | | | | |
| IFN-a/B | + | + | - | - | 1,2,3 | ISRE |
| IFN-g | | + | + | - | 1 | GAS (IRF1>Lys6>IFP) |
| II-10 | + | ? | ? | - | 1,3 | |

| gp130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) | - | + | - | + | 6 | GAS (IRF1 = IFP |
| >>Ly6)(IgH) | | | | | | |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using Sail and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, II-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H1 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well),

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mis of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGACTTTCCGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes Sail and NotI, and inserted into a vector containing neomycin resistance. Particularly, the NF-κB/SV40/SEAP cassette was inserted into pGFP-1 (Clontech), replacing the GFP gene, after restricting pGFP-1 with Sail and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well is used in place of A431 extract. Plates are then treated with a commercial polyclonal (rabbit) antibody (lug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well arid incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

## Claims

1. A nucleic acid molecule comprising a polynucleotide having a nucleotide sequence selected from the group consisting of:
(a) a polynucleotide having the entire nucleotide sequence of SEQ ID NO: 27
(b) a polynucleotide having the entire cDNA sequence included in ATCC Deposit No. 97922, which is hybridizable to SEQ ID NO: 27;
(c) a polynucleotide fragment of SEQ ID NO: 27;
(d) a polynucleotide fragment of the cDNA sequence included in ATCC Deposit No. 97922, which is hybridizable to SEQ ID NO: 27;
(e) a polynucleotide encoding a polypeptide fragment of SEQ ID NO: 67;
(f) a polynucleotide encoding a polypeptide fragment encoded by the cDNA sequence included in ATCC Deposit No. 97922, which is hybridizable to SEQ ID NO: 27;
(g) a polynucleotide encoding a polypeptide fragment of SEQ ID NO: 67 or a polypeptide fragment encoded by the cDNA sequence included in ATCC Deposit No. 97922, having biological activity;
(h) a polynucleotide encoding a polypeptide fragment of SEQ ID NO: 67 or a polypeptide fragment encoded by the cDNA sequence included in ATCC Deposit No. 97922, which is secreted;
(i) a polynucleotide encoding a full length polypeptide of SEQ ID NO: 67 or being encoded by the cDNA sequence included in ATCC Deposit No. 97922;
(j) a polynucleotide encoding a polypeptide domain of SEQ ID NO: 67;
(k) a polynucleotide encoding a polypeptide domain encoded by the cDNA sequence included in ATCC Deposit No. 97922, which is hybridizable to SEQ ID NO: 27;
(l) a polynucleotide encoding a polypeptide epitope of SEQ ID NO: 67;
(m) a polynucleotide encoding a polypeptide epitope encoded by the cDNA sequence included in ATCC Deposit No. 97922, which is hybridizable to SEQ ID NO: 27;
(n) a polynucleotide which is a variant of SEQ ID NO: 27;
(o) a polynucleotide encoding a variant of SEQ ID NO: 67;
(p) a polynucleotide which is an allelic variant of SEQ ID NO: 27;
(q) a polynucleotide encoding an allelic variant of SEQ ID NO: 67;
(r) a polynucleotide which encodes a species homologue of the SEQ ID NO: 67;
(s) a polynucleotide which is at least 90% identical to a polynucleotide as defined in any one of (a) to (r);
(t) a polynucleotide encoding a polypeptide having an amino acid sequence at least 90% identical to the amino acid sequence of a polypeptide encoded by a polynucleotide of any one of (a) to (s); and
(u) a polynucleotide capable of hybridizing under stringent conditions to any one of the polynucleotides specified in (a) to (t), wherein said polynucleotide does not hybridize under stringent conditions to a nucleic acid molecule having a nucleotide sequence of only A residues or of only T residues;
or the complementary strand of polynucleotide (a) to (u).

2. The nucleic acid molecule of claim 1, wherein the nucleotide sequence comprises sequential nucleotide deletions from either the 5 prime terminus or the 3 prime terminus.

3. The nucleic acid molecule of claim 1 or 2 which is DNA or RNA.

4. The gene corresponding to the cDNA sequence of SEQ ID NO: 27 or encoding the polypeptide having the amino acid sequence of SEQ ID NO: 67.

5. A vector comprising the nucleic acid molecule of any one of claims 1 to 3.

6. A method of making a recombinant host cell comprising introducing the nucleic acid molecule of any one of claims 1 to 3, or the vector of claim 5.

7. A recombinant host cell containing the nucleic acid molecule of claims 1 to 3 or the vector of claim 5 or produced by the method of claim 6.

8. The recombinant host cell of claim 7 which expresses a polypeptide encoded by the nucleic acid molecule of any one of claims 1 to 3.

9. A method of making a polypeptide encoded by the nucleic acid molecule of any one of claims 1 to 3 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3 or obtainable by the method of claim 9.

11. A polypeptide comprising a polypeptide selected from the group consisting of:
(a) a polypeptide fragment of SEQ ID NO: 67;
(b) a polypeptide fragment of SEQ ID NO: 67 having biological activity;
(c) a polypeptide fragment of SEQ ID NO: 67, which is secreted;
(d) a full length polypeptide of SEQ ID NO: 67;
(e) a polypeptide domain of SEQ ID NO: 67;
(f) a polypeptide epitope of SEQ ID NO: 67;
(g) a polypeptide variant of SEQ ID NO: 67;
(h) an allelic variant of SEQ ID NO: 67;
(i) a species homologue of SEQ ID NO: 67; and
(j) a polypeptide which is at least 90% identical to a polypeptide as defined in any one of (a) to (i).

12. An antibody that binds specifically to the polypeptide of claim 10 or 11.

13. An antagonist of the polypeptide of claim 10 or 11 comprising a molecule closely related to the natural ligand of said polypeptide or a polypeptide related to the natural receptor of the polypeptide of claim 10 or 11 or a fragment thereof.

14. An agonist of the polypeptide of claim 10 or 11 comprising a molecule closely related to the natural ligand of said polypeptide or a polypeptide related to the natural receptor of the polypeptide of claim 10 or 11 or a fragment thereof.

15. A method for identifying antagonists or agonists of the polypeptide of claim 10 or 11 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

16. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

17. An in vitro method of diagnosing a pathological condition or a susceptibility to a pathological condition in a subject comprising:
(a) determining the presence or amount of expression of the polypeptide of claim 10 or 11 in a biological sample; and
(b) diagnosing a pathological condition or a susceptibility to a pathological condition based on the presence , or amount of expression of the polypeptide.

18. A method for identifying a binding partner to the polypeptide of claim 10 or 11 comprising:
(a) contacting the polypeptide of claim 10 or 11 with a binding partner; and
(b) determining whether the binding partner effects an activity of the polypeptide.

19. A method of identifying an activity in a biological assay, wherein the method comprises:
(a) expressing the nucleic acid molecule of any one of claims 1 to 3 in a cell;
(b) isolating the supernatant;
(c) detecting an activity in a biological assay; and
(d) identifying the protein in the supernatant having the activity.

20. The product produced by the method of claim 19 or identified by the method of claim 15 or 18.

21. A pharmaceutical composition comprising the nucleic acid molecule of any one of claims 1 to 3, the polypeptide of claim 10 or 11, a DNA encoding and capable of expressing said polypeptide in vivo, the antibody of claim 12, the antagonist of claim 13 or the agonist of claim 14, or the product of claim 20 and optionally a pharmaceutically acceptable carrier.

22. Use of the nucleic acid molecule of any one of claims 1 to 3, the polypeptide of claim 10 or 11, the antibody of claim 12, the antagonist of claim 13 or the agonist of claim 14, or the product of claim 20 or a DNA encoding and capable of expressing said polypeptide in vivo for the preparation of a pharmaceutical composition for preventing, treating, or ameliorating a medical condition.

23. A diagnostic composition comprising the nucleic acid molecule of any one of claims 1 to 3 or the antibody of claim 12.

24. A method for the production of a pharmaceutical composition comprising the steps of the method of claim 15, 18 or 19 and the further step of formulating the compound identified in a pharmaceutically acceptable form.

25. The polypeptide of claim 11, wherein the polypeptide is post-translationally modified, fused to a heterologous polypeptide, or coupled to polyethylene glycol.
